(19) Europäisches Patentamt
European Patent Office
Office européen des brevets

(11) EP 4 478 250 A1

(12) **EUROPEAN PATENT APPLICATION**

(43) Date of publication:
**18.12.2024 Bulletin 2024/51**

(21) Application number: **23386048.5**

(22) Date of filing: **13.06.2023**

(51) International Patent Classification (IPC):
***G06N 3/0442*** (2023.01) ***G06N 3/08*** (2023.01)
***G06N 20/00*** (2019.01) ***G16B 15/30*** (2019.01)

(52) Cooperative Patent Classification (CPC):
**G06N 20/00; G06N 3/0442; G06N 3/08; G16B 15/30; G16B 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **NEC OncoImmunity AS**
**0379 Oslo (NO)**

(72) Inventors:
- **Vardaxis, Alexandros Ioannis**
  **0379 Oslo (NO)**
- **Simovski, Boris**
  **0379 Oslo (NO)**
- **Clancy, Trevor**
  **0379 Oslo (NO)**
- **Stratford, Richard**
  **0379 Oslo (NO)**

(74) Representative: **Gill Jennings & Every LLP**
**The Broadgate Tower**
**20 Primrose Street**
**London EC2A 2ES (GB)**

(54) **METHOD OF TRAINING A MACHINE LEARNING MODEL**

(57) A method of training a machine learning model is disclosed, comprising: generating (204) first pseudo-random data (116); performing (206) a first pass of machine learning training, using positive training data (114) and using the first pseudo-random data as negative training data, to complete a first epoch; generating (208) second pseudo-random data; and performing (210) a second pass of machine learning training, using the second pseudo-random data as negative training data and using the positive training data, to complete a second epoch.

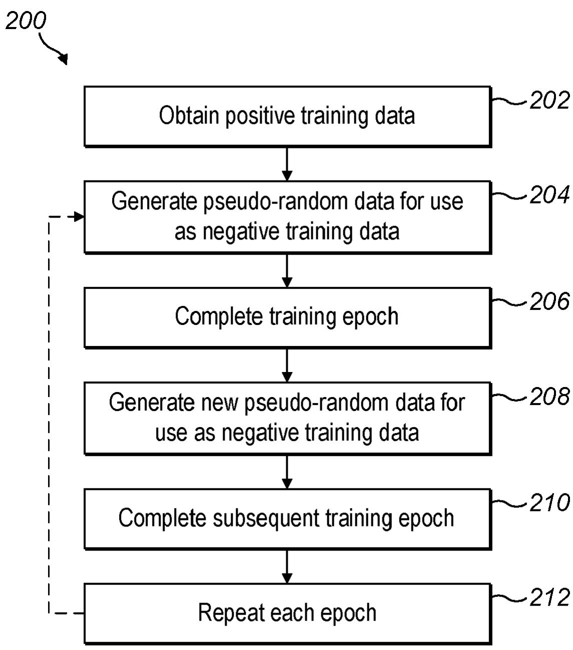


*FIG. 2*

EP 4 478 250 A1

## Description

### FIELD OF THE INVENTION

The invention relates to a method of training a machine learning model. In particular, the method relates to training a machine learning model in the absence of negative data that is confirmed not to have a particular characteristic.

### BACKGROUND

Machine learning models are often trained using positive training data, containing examples known to exhibit a particular characteristic or property, and negative training data, containing examples known not to have the characteristic. Machine learning models use this training to recognise features in test data shared by the positive or negative training data. In turn, this allows the model to assess the likelihood that a given example in the test data shares the characteristic in the positive training data.

However, in some scenarios, confirmed or verified negative examples may be limited in number, or may not be available at all. For various reasons, it may not be practical to confirm whether a given sample exhibits the characteristic the model is to be trained to recognise.

One specific example where negative training data is difficult to obtain at scale relates to B-Cell epitopes (BCEs). The accurate identification or prediction of BCEs on antigens is of importance for vaccine design, molecular diagnostics, immune-monitoring and immunotherapy. Conformational BCEs (CBCEs) can be discovered using X-ray crystallography on antigen-antibody structure complexes. However, it may not be possible, or may be highly impractical, to use empirical methods to confirm that a given amino-acid sequence for an antigen is not associated with a CBCE, at least at the scale required for machine learning training.

The issue of a shortfall or absence of confirmed negative examples can arise in a wide range of fields where the use of machine learning models would be desirable. There is therefore a need to provide a method of training a machine learning model in a robust manner in such cases.

### SUMMARY OF INVENTION

According to a first aspect of the present invention, there is provided a method of training a machine learning model, comprising: generating first pseudo-random data; performing a first pass of machine learning training, using positive training data and using the first pseudo-random data as negative training data, to complete a first epoch; generating second pseudo-random data; and performing a second pass of machine learning training, using the second pseudo-random data as negative training data and using the positive training data, to complete a second epoch.

In this way, machine learning training can be performed even in the absence of verified negative data. The pseudo-random nature of the negative training data means that the negative training data can be presumed not to have a characteristic or property, which is common to samples in the positive training data, with a high degree of accuracy. Generating different negative training data in the first epoch and the second epoch allows the machine learning model to be exposed to different examples in each epoch, fundamentally creating a more robust machine learning model.

The use of pseudo-random negative data in the first and second epochs can also reduce the presence of false negatives compared to unlabelled negative training data that has not be pseudo-randomly generated. Additionally, generating different pseudo-random data for each of the first and second epochs avoids performing several training iterations on the same false negatives over the first and second epochs. This, in turn, avoids unfavourably skewing the machine learning model.

The skilled person would appreciate that the term "epoch" refers to a full training iteration where the machine learning model is trained using all of the available positive and negative training data. In the context of neural networks and machine learning, an epoch refers to a complete pass or iteration through the entire training dataset. During the training phase of a neural network, a dataset is divided into smaller batches or subsets, and the network is trained on each batch in succession. One epoch is completed when the network has iterated through all the batches. Therefore, "performing a first pass of machine learning training to complete a first epoch" refers to the completion of a single training epoch, which may include several batches. Any number of epochs can be performed in the manner above until the machine learning model is completely trained or converges. Alternatively, some epochs may be completed based on the same negative training data while other epochs may be completed in the manner described above, i.e., based on different pseudo-random data.

The machine learning training based on the positive and negative data in each pass can be performed using any suitable known technique. In one example, weights of the machine learning model can be adjusted to minimise a loss function. The skilled person would appreciate that different types of machine learning model may require different implementations, as known in the art.

Preferably, the positive training data contains examples verified as having a first characteristic, and training the machine learning model is performed using only the positive training data and pseudo-random data assumed not to have the first characteristic as negative training data. In this way, the machine learning model is trained without using any training examples verified by other means as not having the first characteristic. In such cases, the use of pseudo-random data may be particularly advantageous due to a total unavailability of verified negative data.

The first characteristic can be any suitable characteristic, such as a property of a physical object or system. In one example, the first characteristic is whether a protein comprises a B-Cell epitope. In further examples, the first characteristic can relate to the contents of a video, audio, image, or text file. In these latter cases, using pseudo-random data as negative data can enable machine learning model to be exposed to much larger number of training examples compared to a finite unlabelled dataset retrieved from a public source. In yet further examples, the first characteristic can be an underlying property of a physical object, such as a density or density range.

Preferably, the positive training data and or the first and second pseudo-random data comprises sets of data elements arranged in an array, vector, matrix or sequence.

Preferably, the machine learning model is configured to predict whether a protein comprises a B-Cell epitope that is likely to instigate a binding event with an antibody (e.g., instigating an immunogenic response and/or instigated by an immunogenic response), based on an input comprising an amino acid sequence of the protein. The B-Cell epitopes can be linear or conformational B-Cell epitopes. Searching for B-Cell epitopes in amino acid sequences can be particularly suited to using pseudo-random data as negative data. This may be because it is particularly unlikely that a pseudo-randomly generated amino acid sequence contains a B-Cell epitope. Thus, machine learning models trained in this way and for this purpose may be particularly effective.

In general, the protein may be a protein, protein-domain, or a protein sub-unit. The term "protein" may include any protein subsequence that may have a viable 3D or functional structure. Typically, the protein comprises an antigen. The protein may comprise a neoantigen. The amino acid sequence of the protein may be obtained using techniques known to the skilled person in the art, for example by one of: oligonucleotide hybridisation methods, nucleic acid amplification based methods (including but not limited to polymerase chain reaction based methods), automated prediction based on DNA or RNA sequencing, de novo peptide sequencing, Edman sequencing or any peptide related sequencing including and not limited to mass spectrometry. The amino acid sequence may be downloaded from a bioinformatic depository such as UniProt (www.uniprot.org).

Preferably, the positive training data comprises encodings of one or more verified B-cell epitopes. The encodings can be represented as one-hot encodings with associated permutation vectors that define the order and number of each amino acid in a particular sequence. Any other suitable data structure can be used to represent samples forming the positive training data in other examples. Preferably, each encoding represents a respective verified B-cell epitope as a set of data elements, each data element (or at least a plurality of data elements in the set) corresponding to an amino acid of the amino acid sequence of a protein.

Preferably, the first pseudo-random data and the second pseudo-random data comprise encodings, each encoding including a plurality of data elements, each data element corresponding to an amino acid of an amino acid sequence of a protein that is assumed not to comprise a B-cell epitope. In this way, the positive training data and the negative training data can be represented with similar data structures.

Each of the encodings can be in the form of a binary vector. In such embodiments, the binary vector will typically be in the form of a sequence of "1"s and "0"s, with the "1"s each corresponding to an amino acid of the protein being a part of the candidate epitope (e.g. a probable Ag-Ab contact point), and the "0"s each corresponding to an amino acid of the protein not forming part of the candidate epitope (e.g. not a probable Ag-Ab contact point). In some embodiments, different encoding protocols may be used (e.g. one-hot encoding) to represent the candidate epitopes.

In some embodiments, the method further comprises subsequently using the machine learning model in the design of a vaccine or immuno-therapeutics. For example, the machine learning model trained according to the first aspect of the invention can be used to identify a B-Cell epitope that is incorporated into a vaccine.

Preferably, a set of data elements in the first pseudo-random data or the second pseudo-random data has a second characteristic that is shared with one or more sets of data elements in the positive training data. More preferably, the first and second pseudo-random data include a plurality of sets of data elements that have the second characteristic. In this way, the second characteristic is "fixed" in at least one set of data elements in the negative training data. This allows the first pseudo-random data and/or the second pseudo-random data to provide more realistic examples. In other words, the negative training data can more closely represent the positive training data, thereby providing more useful negative training data. Fixing the second characteristic in this manner avoids generating negative training data that is closer to random noise than realistic examples of the subject of the training data.

The second characteristic can be any suitable property of a set of data elements in the positive training data. In one example, the second characteristic can be the frequency with which a particular value arises, such as a particular colour in an image or a particular amino acid in an amino acid sequence. In another example, the second characteristic can be a length of the set of data elements.

Preferably, generating the first pseudo-random data and/or generating the second pseudo-random data comprises pseudo-randomly modifying one or more sets of data elements in the positive training data. In this way, the negative training data can more closely represent the positive training data, thereby providing more useful negative training data.

Preferably, each set of data elements in the positive training data may be pseudo-randomly modified several times to create several associated pseudo-random sets of data elements. For each set of data elements in the positive training data (i.e., for each positive example), N sets of data elements may be pseudo-randomly generated to create N sets of data elements associated with each set of data elements in the positive training data. N can take values of 10, 20 or 30 in some specific examples. This enables a variety of negative samples to be included in the negative training data that are similar, but nevertheless pseudo-randomly vary from, samples in the positive training data, to create a more robust training method.

Preferably, a set of data elements in the first pseudo-random data and/or the second pseudo-random data includes a subset of values that are present in a set of the positive training data. In this way, the first or second pseudo-random data can include a particular aspect of examples in the positive training data to provide more realistic negative training data. This can have the effect that the subset is present in examples in the negative training data but is shifted to other locations in the set, thus providing a more robust training data set. The remainder of the values that are present in the set of the positive training data can be pseudo-randomly altered or rearranged to produce the set of data elements in the first or second pseudo-random data.

The subset can be a continuous sequence of values, or alternatively can comprise a discontinuous sequence of values. In the latter case, the spacing between the discontinuous sequence of values can be replicated (i.e., fixed) in the set of data elements in the first and/or second pseudo random data. This can also be referred to as fixing or maintaining a "linear distance" between values.

In one particular application, the subset of values can correspond to a subsection of an amino acid sequence that is associated with a B-Cell epitope in an amino acid sequence in the positive training data. In conformational B-Cell epitopes, the subset of values in the protein associated with the B-Cell epitope may be discontinuous in the sequence, due to folding of the protein. In this case, fixing the values in the subset and the linear distance between these values preserves the structure of the B-Cell epitope in the pseudo-randomly generated examples, providing more useful negative training data.

Preferably, generating the first pseudo-random data and/or the second pseudo-random data comprises fixing certain values in a set of data elements in the positive training data and pseudo-randomly rearranging or altering remaining values to generate one or more sets of data elements. This is another way of generating more realistic negative training data.

The "certain values" can be selected in any appropriate way. In one example, the first and last values in a given set of data elements in the positive training data can be fixed. Alternatively, values in a given set of data elements in the positive training data associated with a special property of the set of data elements can be fixed. In one particular example, the set of data elements can represent an amino acid sequence of a protein, and the certain values of the data elements that form a B-Cell epitope can be fixed.

Preferably, the method comprises generating new pseudo-random data for each epoch during training and using the newly generated pseudo-random data as negative data in each respective epoch. In this way, the machine learning training can be performed based on a larger set of negative training data, leading to a more robust machine learning model.

More preferably, the method comprises replacing pseudo-random data used in each previous epoch with the newly generated pseudo-random data such that different negative data is used in each epoch. In this way, continuous re-training of a false-negative example in pseudo-random data generated in an earlier epoch is avoided, which could otherwise skew the machine learning model. Additionally, the machine learning model can be exposed to a greater number of negative training examples, improving the robustness of the trained model.

Preferably, the machine leaning model is a neural network, preferably a long short-term memory network, LSTM or a bi-directional long short-term memory network, BLSTM. In other embodiments, the machine learning model could be any type of machine learning model that performs a plurality of epochs or training iterations based on the same training data.

Preferably, in one or more epochs, the negative training data contains more examples than the positive training data, more preferably wherein a ratio of the number of sets in the negative training data to the number of sets in the positive training data is greater than or equal to 2:1, more preferably where the ratio is about 3:1. It has been found that using such ratios is advantageous for model performance without creating an undue burden on processing or memory requirements.

In some examples, the method comprises pre-generating pseudo-random data, and generating the first pseudo-random data and/or the second pseudo-random data comprises pseudo-randomly selecting from examples in the pre-generated pseudo-random data. This may provide better model performance in some scenarios.

According to a further aspect of the present invention, there is provided a non-transitory computer readable medium comprising executable instructions that, when executed by a computer, cause the computer to perform steps to train a machine learning model, the steps comprising: generating first pseudo-random data; performing a first pass of machine learning training, using positive training data and using the first pseudo-random data as negative training data, to complete a first epoch; generating second pseudo-random data; and performing a second pass of machine learning training, using the second pseudo-random data as negative training data and using the positive training data, to complete a second epoch.

According to a further aspect of the present invention, there is provided a system comprising one or more processors configured to train a machine learning model by performing steps comprising: generating first pseudo-random data; performing a first pass of machine learning training, using positive training data and using the first pseudo-random data as negative training data, to complete a first epoch; generating second pseudo-random data; and performing a second pass of machine learning training, using the second pseudo-random data as negative training data and using the positive training data, to complete a second epoch.

According to a further aspect of the present invention, there is provided a non-transitory computer readable medium comprising a machine learning model trained according to the method the first aspect of the invention.

According to a further aspect of the present invention, there is provided a method of use of a machine learning model trained according to the first aspect of the invention.

According to a further aspect of the present invention, there is provided a machine learning model trained using the method of the first aspect of the present invention.

## BRIEF DESCRIPTION OF DRAWINGS

Embodiments of the invention are now described, by way of example, with reference to the drawings, in which:

Figure 1 shows a schematic control diagram of a computer system for carrying out the invention;

Figure 2 shows a flowchart of a method of training a machine learning model in an embodiment of the invention;

Figure 3 shows a flowchart of a method of training a machine learning model in an embodiment of the invention;

Figure 4 is a flow diagram illustrating the steps of a method incorporating a model trained according to an embodiment of the invention;

Figure 5 is a flow diagram illustrating the steps of a method of training a first machine learning algorithm according to an embodiment of the invention;

Figure 6 is a flow diagram illustrating the steps of a method of training a second machine learning algorithm;

Figures 7(a) to 7(f) show the correlation results of the feature-feature pairs;

Figure 8 shows the Spearman's correlation coefficients for continuous features against (a) RSA, (b) UHSE and (c) LHSE;

Figure 9 shows the Kendall's correlation coefficients for categorical features against (a) RSA, (b) UHSE and (c) LHSE;

Figure 10 shows the Kendall's correlation coefficients for continuous features against (a) SS, (b) CBCE and (c) LBCE;

Figure 11 shows the Mutual Information criterion for categorical features against (a) SS, (b) CBCE and (c) LBCE;

Figure 12 schematically shows the structure of a Prediction model for RSA and HSE;

Figures 13(a) to (f) show cross-validation results for a RSA/HSE model;

Figures 14(a) to (f) show test results for a RSA/HSE model ;

Figures 15(a) to (f) show final training results for a RSA/HSE model;

Figures 16(a) to (f) illustrate CV results for a Secondary Structure (SS) prediction model according to the invention;

Figures 17(a) and (b) show the test results for SS model;

Figures 18(a) to (d) show final training results for the SS model;

Figures 19(a) and 19(b) show Paratope Analysis for CBCE;

Figure 20(a) schematically illustrates the architecture of a first machine learning algorithm for predicting CBCEs;

Figure 20(b) schematically illustrates the architecture of a first machine learning algorithm for predicting CBCEs, according to a further embodiment of the invention;

Figures 21(a) to (d) illustrate outlier analysis for CBCEs;

Figures 22(a) to (f) show results for the precision-recall metric for a first machine learning algorithm for predicting CBCEs, according to a further embodiment of the invention;

Figures 23(a) and (b) show results for the precision-recall metric for a first machine learning algorithm for predicting CBCEs;

Figures 24(a) and (b) show the results for the precision-recall metric for a first machine learning algorithm for predicting CBCEs;

Figure 25(a) and (b) show the results for the precision-recall metric for a first machine learning algorithm for predicting CBCEs;

Figures 26(a) to (c) show the results of final training for a first machine learning algorithm for predicting CBCEs;

Figures 27(a) and (b) show the results of final training for a first machine learning algorithm for predicting CBCEs;

Figures 28(a) to (d) illustrates outlier analysis for LBCEs;

Figure 29 is an example of a system suitable for implementing embodiments of the method is shown;

Figure 30 is an example of a suitable server; and

Figure 31 schematically illustrates a pipeline.

## DETAILED DESCRIPTION

*Machine Learning Training*

Figure 1 shows a schematic control diagram of an example system for carrying out the method of the invention. A computer 10 is provided and comprises a controller 102 and a memory 104. The controller 102 comprises one or more processors configured to execute instructions 106 stored in the memory 104. The instructions 106 can be executed by the controller 102 to operate a trainer module 108 and a pseudo-random number generator 110, which can be any suitable type of generator known in the art. The trainer module 108 is configured to train a machine learning model 112 that can be stored in the memory 104. Positive training data 114 for the machine learning model 112 can also be stored in the memory together with pseudo-random data 116. The computer 10 can be provided with a network interface 118 for performing the method of the invention remotely for a client device. Alternatively, the modules executed by the controller 102 and/or the data stored in the memory 104 can be provided in a distributed fashion across a network for providing machine learning training to a client device.

The machine learning model 112 is an algorithm configured to make predictions based on the positive training data 114 and negative training data about previously unseen test data. The machine learning model 112 can take test data representing a candidate subject, such as an image, a physical structure or an amino acid sequence, as input and output a probability that a given candidate has a particular property or characteristic. In the example methods discussed below, the machine learning model 112 is configured to predict whether a candidate amino acid sequence corresponds to a B-Cell epitope. A more detailed explanation of B-Cell epitopes is provided in subsequent subsections. In other embodiments, the method of the present invention can be applied to train a machine learning model configured to solve any other type of problem in related or unrelated fields.

In this example, the machine learning model 112 is a neural network, in particular a bi-directional long short-term memory network (BLSTM). In other examples, the machine learning model 112 can be a long short-term memory network, or any other suitable kind of machine learning model known in the art that can be trained over several epochs.

The positive training data 114 represents examples, also referred to as "samples", of a subject known to exhibit the particular characteristic or property that the machine learning model 112 is intended to identify in unseen data. The positive training data 114 can include samples verified in any suitable manner, such as manually by a human operator or empirically by prior experiment. The positive training data 114 can be of any suitable format for performing machine learning, such as a sets of data elements taking particular values, e.g. binary values, arranged in an array, vector, matrix or sequence.

In the example methods discussed further below, the positive training data 114 contains amino acid sequences, their common characteristic being that each sequence contains a B-Cell epitope. Further details regarding B-Cell epitopes are provided in subsequent subsections. The amino acid sequences in the positive training data 114 are encoded in one-hot encoded matrices. Each amino acid sequence is thus represented by a set of data elements in a matrix, each data element having a binary value indicating whether a particular amino acid is present in the sequence. Each amino acid sequence can also have an associated permutation vector indicating the order and number of each amino acid in the sequence. In other examples, the amino acid sequences could be encoded or represented by any other suitable data structure.

The pseudo-random data 116 comprises examples generated using the pseudo-random number generator 110. The machine learning model 112 is configured to treat these examples as not having the particular characteristic that is common among the samples in the positive training data 114. In other words, the trainer module 108 is configured to train the machine learning model 112 using the pseudo-random data 116 as negative training data. The random nature of each example in the pseudo-random data 116 means that it can be presumed with a high degree of certainty that a given pseudo-random example does not exhibit the common property in the positive training data 114.

The pseudo-random data 116 can similarly be of any suitable format for performing machine learning, such as a sets of data elements taking particular values, e.g. binary values, arranged in an array, vector, matrix or sequence. In the examples below, the pseudo-random data 116 is represented in the same manner as the positive training data 114, i.e., by one-hot encodings of amino acid sequences with associated permutation vectors.

Figures 2 and 3 show methods of training the machine learning model 112 according to an embodiment of the invention, which can be carried out by a computer such as the computer 10 of Figure 1. The methods of Figures 2 and 3 can be used to train a machine learning model adapted to any suitable field and, in particular, fields where verified negative data is not readily available.

For the purposes of illustration, the methods of Figures 2 and 3 are described using the specific example where the machine learning model 112 is configured to predict whether a protein comprises a B-cell epitope that is likely to instigate an immunogenic response. B-Cell epitopes can be discovered experimentally using X-ray crystallography on antigen-antibody structure complexes. However, it may not be possible to know with certainty that a given protein could not act as a B-Cell epitope for an unspecified or unknown antibody. Consequently, it may not be possible to provide a negative example of a protein that is, for certain, not (or does not include) a B-Cell epitope. This same issue of a lack of verified negative data can arise in many other scenarios, where such examples may be impossible to obtain (or impractical to obtain at the scale required for machine learning training).

The method of the present invention enables robust training of the machine learning model 112 despite the lack of available negative training samples.

Figure 2 shows a flowchart of a method 200 for training a machine learning model 112 according to an embodiment of the invention.

The method 200 begins at step 202 where the trainer module 108 obtains the positive training data 114 stored previously in the memory 104.

At step 204, the trainer module 108 uses the pseudo-random number generator 110 to generate pseudo-random amino acid sequences for use as negative training data. In this example, the model trainer 108 generates a plurality of "completely" random amino acid sequences. In other words, each value in the sequence can take the value of any amino acid. The total length of the sequence may also vary pseudo-randomly. Generating the pseudo-random data 116 in this way reduces the likelihood of generating false-negatives in the negative training data, leading to a more robust final model. The generated sequences may be stored as one-hot encoded matrices and associated permutation vectors, as described previously.

In one example, the trainer module 108 generates 10 pseudo-random amino acid sequences per positive sample in the positive training data 114. In another example, 30 pseudo-random amino acid sequences are generated per positive sample.

Having more negative examples than positive examples in the training data may be beneficial because there is often a more limited subset of examples that have a particular characteristic. Conversely, there can be an unlimited number of examples not having the characteristic. This applies to the specific case of recognising candidate B-Cell epitopes in amino acid sequences as well as in other applications. It has been found that a ratio of positive to negative examples of about 1 to 3 yields good performance of the machine learning model 112 without unduly increasing the processing time.

At step 206, the trainer module 108 trains the machine learning model 112 based on the full set of positive training data 114 and the full set of pseudo-random data 116 to complete one epoch training epoch. Depending on the size of the training datasets, the available memory and the processing capacity, each epoch may need to be processed in several batches. The skilled person would appreciate that training data can be processed by machine learning models several times to gradually improve the model quality. A cycle of training using all of the available training data is referred to as an "epoch", where each epoch can be split into a plurality of "batches" of training due to memory constraints.

In other embodiments, the model trainer 108 can complete several epochs (i.e. training iterations) based on the pseudo-random data 116 generated at step 204.

The model trainer 108 performs the training based on the positive training data 114 and the negative training data as known in the art, e.g., by updating weights in the machine learning model 112 to minimise a loss function.

Once the model trainer 108 has completed the desired number of epochs, the model trainer 108 proceeds to step 208.

In step 208, new pseudo-random data 116 is generated in the same manner as in step 204. The newly generated pseudo-random data 116 enables the machine learning model 112 to be exposed to a large number of negative examples, compared to machine learning training performed using a finite dataset.

In step 210, a further epoch is completed based on the positive training data 114 and negative training data comprised of the pseudo-random data 116 generated at step 208. As in step 206, the machine learning model 112 is trained by the model trainer 108 using the full set of positive training data 114 and the full set of pseudo-random data 116 in this latter epoch.

In other embodiments, several training epochs can be performed at step 210 based on the pseudo-random data 116 generated at step 208.

The model trainer 108 can be configured to replace pseudo-random data 116 of step 204 with the pseudo-random data 116 of step 208, such that new negative training data is used in the later epoch of step 210. Alternatively, the model trainer 108 can expand the samples used as negative data to include the samples generated at step 208. However, replacing the pseudo-random data 116 with newly generated data each training epoch is preferred because it avoids continuous re-sampling of false negatives, which could skew the machine learning model 112. In any case, at least two epochs are based on different pseudo-random data 116 as negative training data.

At step 212, steps 204 to 210 are repeated until the desired number of epochs are completed and the machine learning model 112 is fully trained. The total number of epochs can be about 50, 80, 100, or 200, in some example implementations. In other embodiments, a larger number of epochs in the range of 200 to 10,000 can be performed.

In other embodiments, the pseudo-random data generation of steps 204 and 208 can be performed by pseudo-randomly selecting some of a larger set of pseudo-randomly generated examples, generated in a preparatory step performed before the first epoch is completed.

Once the machine learning model 112 is trained, the machine learning model 112 can receive an input, such as a candidate amino acid sequence, in the same or a similar format as the positive training data 114 and the pseudo-random data 116. The machine learning model 112 can output a probability that the candidate has a particular characteristic, e.g., whether the amino acid sequence comprises a B-Cell epitope.

Figure 3 shows a flowchart of a method 300 for training a machine learning model 112 according to a further embodiment of the invention.

The method 300 begins at step 302 wherein the trainer module 108 obtains the positive training data 114 stored previously in the memory 104.

At step 304, the trainer module 108 uses the pseudo-random number generator 110 to generate pseudo-random amino acid sequences for use as negative training data. In this example, the model trainer 108 generates pseudo-random data in three different ways, represented in Figure 3 by steps 304a to 304c. The skilled person would appreciate these steps may also be undertaken in parallel.

In step 304a, a plurality of completely random sequences is generated in the same manner as step 204 of the method 200. In one example, 10 samples may be generated for each sample in the positive training data 114. This provides a portion of negative training data that is particularly unlikely to include false negatives.

In steps 304b and 304c, the trainer module 108 uses the pseudo-random number generator 110 to generate pseudo-random amino acid sequences by modifying each amino acid sequence in the positive training data 116. The modification is performed pseudo-randomly, such that the resulting data can still be presumed to be a negative sample with a high degree of accuracy. Using the resulting pseudo-random data 116 as negative training data has the benefit that the generated samples are more likely to represent realistic amino acid sequences.

In step 304b, the trainer module 108 generates pseudo-random data 116 by fixing one or more amino acids in each positive sample and pseudo-randomly rearranging the remaining amino acids to create permutations. In one example, the first and last amino acid of each positive sample is fixed and the remaining values can be shuffled into a different order. Alternatively, the remaining values can be completely randomised, i.e., can be replaced with any amino acid on a pseudo-random basis. In another example, a pseudo-random selection of values in each sequence of the positive samples can be fixed with the remaining values rearranged or randomised. 10 samples may be generated in this way per positive sample in the positive training data 114, such that there are 10 examples in the pseudo-random data 116 associated with each positive example. More or less pseudo-random samples can be generated per positive sample in other embodiments.

In step 304c, the trainer module 108 generates pseudo-random data 116 by fixing a characteristic in each sample of the positive training data. As in step 304b, 10 pseudo-random samples can be generated for use as negative data per positive sample, such that there are 10 examples in the negative training data associated with each positive sample. Other numbers of pseudo-random samples can be generated per positive sample in other embodiments.

The characteristic fixed in step 304c can be one or more of a length of a sequence, the inclusion of a particular subset of a sequence of amino acids, a total amount of amino acids in the sequence, or a linear distance between two parts of a sequence.

In this context, the term "linear distance" relates to the parts of an amino acid sequence in a particular positive sample that form the B-Cell epitope when the protein is folded. These parts may be discontinuous in the amino acid sequence, depending on the structure of the protein when folded. This is the case in conformational B-Cell epitopes, for instance. Therefore, fixing the "linear distance" refers to maintaining the relative distance in the sequence between two or more discontinuous parts of an amino acid sequence that form a B-Cell epitope.

One particular way of generating permutations in step 304c involves identifying those parts of an amino acid sequence that form a B-Cell epitope, then fixing the values (i.e., fixing those particular amino acids) that form the verified epitope in the positive sample. The linear distance between these amino acids is also kept constant. Using the pseudo-random generator 110, the model trainer 108 can pseudo-randomly shuffle the location of the fixed amino acids to other locations in the protein, or alternatively randomise the remaining amino acids in the sequence. This allows the model trainer 108 to generate pseudo-random permutations having a similar structure to the associated verified B-Cell epitope.

In other examples, the trainer module 108 can split each positive sample into groups of adjacent amino acids and shuffle the position of each group to create permutations. Other suitable characteristics could also be fixed, such as the relative frequency of particular amino acids that are more likely to be found in the positive training data 114.

In other use cases, any suitable characteristic or property of samples in the positive training data 114 can be fixed.

The trainer module 108 generates 10 pseudo-random samples per positive sample in the positive training data 114 in this way. A greater or lesser number of pseudo-random samples can be generated in other embodiments.

Once steps 304a to 304c have been completed, the pseudo-random data 116 can be used as negative training data. In a case where 10 pseudo-random samples are generated for each positive sample in each of steps 304a to 304b, the overall ratio of positive to negative examples would be 1:3.

At step 306, the trainer module 108 trains the machine learning model 112 based on the full set of positive training data 114 and the full set of pseudo-random data 116 of step 304 to complete at least one epoch. Depending on the size of the training datasets, the available memory, and available processing capacity, each epoch may need to be processed in several batches, as described previously. The training can be performed using known methods of executing machine learning training based on positive and negative data, as described previously.

The model trainer 108 can complete several epochs of training based on the pseudo-random data 116 generated at steps 304a to 304c. Preferably, however, the pseudo-random data 116 of step 304a to 304c is used in only one epoch and is replaced with newly generated pseudo-random data 116 every epoch to expose the machine learning model 112 to more negative data. Generating new pseudo-random data 116 for each epoch also avoids continuous re-sampling of false negatives, which could skew the machine learning model 112. In any case, at least two epochs are based on different pseudo-random data 116 as negative training data.

Once the model trainer 108 has completed the desired number of epochs, the model trainer 108 proceeds to step 308.

In step 308, the method 300 loops and steps 304 to 306 are repeated until the desired number of epochs are completed, after which the machine learning model 112 is fully trained. The total number of epochs can be about 50, 80, 100, or 200, in some example implementations. In other embodiments, a larger number of epochs in the range of 200 to 10,000 can be performed.

In other embodiments, the pseudo-random data generation of step 304 can be performed by pseudo-randomly selecting a subset of a larger set of pseudo-randomly generated examples, generated in a preparatory step performed before the first epoch is completed.

Once the machine learning model 112 is trained, the machine learning model 112 can receive an input, such as a candidate amino acid sequence, in the same or a similar format as the positive training data 114 and the pseudo-random data 116. The machine learning model 112 can output a probability that the candidate has a particular characteristic, e.g., whether the amino acid sequence comprises a B-Cell epitope.

*Background and Example Usage in a **B-Cell** Epitope Predictor*

1. Overview

The invention described above can be used to train a B-cell epitope (BCE) prediction algorithm ("first machine learning algorithm" or "first machine learning model"), for example using the method 200 or the method 300. The following description provides an overview of this example implementation of the trained BCE prediction algorithm.

The model accurately predicts BCEs on a given Ag from its primary sequence in combination with one or more 3D structure and/or surface characteristics of the Ag, with no requirement for the full 3D structure of the Ag as an input into the predictor. The model is trained using BLSTMs, which allows for the processing of the whole Ag sequence as one data-point, processed from both directions without the need for *k-mer* segmentation. Each amino-acid of the Ag is treated as a time-step in the network, whose characteristics can be affected by both preceding and succeeding amino-acids that flank the amino-acid in question, thus, allowing for the discovery of the contextual relationships between spatially distant amino-acids which could be important parts of conformational BCEs (CBCEs).

The first machine learning model is capable of discovering separate or independent CBCEs that may exist on a single Ag protein sequence. A particularly advantageous feature of the model is that it takes as input an Ag protein sequence and a binary "permutation vector" or "encoding" representing each single candidate CBCE (or LCBE), as well as the one or more 3D structure and/or surface characteristics of the Ag. The output of the model is a single probability indicating the likelihood that the input permutation vector is a *bone fide* CBCE. In other words, the user asks the question to the model if the specific query CBCE, encoded as a binary permutation vector on the given Ag sequence, is a true CBCE or not. However, the model may be used to provide a second output in the form of a probability vector with a probability for each amino-acid in the Ag sequence. The second output can be seen as the contribution of each amino acid to the CBCE in question, and may be used to compare the current approach with conventional models that predict on an "amino acid by amino acid" basis. The first machine learning algorithm is preferably trained on unbound 3D Ag protein structures prior to the Ag-Ab binding event, to learn the properties of BCEs on the Ag prior to the Ab binding event, in order to capture the *bone fide* ("true") BCEs on the query Ag protein sequence.

Structural characteristics of the Ag sequence are important for predicting CBCEs. However, embodiments of the present example negate the need for the complete 3D structure of the protein sequence to be experimentally measured or predicted, and then used as input into the algorithm. The first machine learning algorithm does not require the coordinates of each atom in each amino acid in the 3D protein sequence (i.e. it does not require the full 3D structure), and instead may predict BCEs using one or more structure and/or surface characteristics of the query protein, such as RSA, HSE and SS.

The "top-level" most important features such as RSA, HSE and SS, may be predicted with one or more trained machine learning algorithms ("second machine learning algorithm" or "second machine learning model"), typically receiving as input the amino acid sequence of the protein. In this way, structural and/or surface characteristics of the protein under investigation may advantageously be computed directly from the amino acid sequence, with no requirement for extra information to be sourced or input by the user. Those second algorithms use BLSTM networks also and are shown to have a high performance.

In this way, the first and second machine learning models may together form a "pipeline" which requires as input only the amino acid sequence of a query protein in order to predict whether that query protein comprises a true B-cell epitope. A schematic diagram of such a pipeline constituting the first and second machine learning models according to an embodiment of the example is shown in Figure 31.

Figure 4 is a flowchart S100 summarising the steps of a preferred embodiment of the present example. At step S101, the amino acid sequence of a protein to be analysed for the presence of B-cell epitopes is accessed. Typically the protein under investigation will comprise an antigen. Various techniques known to the skilled person may be used to access the amino acid sequence, for example the sequence may be downloaded from a bioinformatics repository such as UniProt.

At step S103, one or more (e.g. three-dimensional) structure and/or surface characteristics of the protein in an unbound state are accessed. Typically, these characteristics of the protein are predicted using one or more trained second machine learning model(s) (schematically illustrated at S300, and described below) that receives the amino acid sequence accessed in step S101 as input. The structure and/or surface characteristics typically include the secondary structure of the query protein and relative solvent accessibility. The half sphere exposure (both upper and lower half sphere exposure) may also be used. Values or categories for each of these characteristics are typically assigned to each amino acid of the sequence. However, other techniques for accessing the three-dimensional structure and surface characteristics of the query protein may be employed, such as database entries, X-ray crystallography or computational approaches to predicting full 3D protein folding structures (from which the structure and/or surface characteristics can be obtained).

At step S105, candidate encodings of one or more candidate BCEs on the query protein are generated. The candidate encodings are each typically in the form of a binary vector, with each data element in the binary vector corresponding to an amino acid of the amino acid sequence forming the respective candidate BCE. In such a binary "permutation vector" encoding, each amino acid of the sequence forming part of the candidate BCE is assigned a "1" and each amino acid of the sequence not forming part of the candidate BCE is assigned a "0". For example, for the exemplary amino acid sequence "ABCDEFG", a candidate conformational B-cell epitope constituted by the amino acids B, D, F and G may be encoded as the binary vector (0101011). In some embodiments, a candidate encoding for each possible candidate BCE on the protein may be generated. However, in order to reduce computational load, the search space may be limited based on physical priors (e.g. knowledge of a mutation point on a neoantigen of the input protein or receptor binding domain on the spike protein of SARS-CoV-2, etc) or on techniques such as deep reinforcement learning or related generative models.

In step S107, the amino acid sequence accessed in step 101, the one or more structure and/or surface characteristics accessed in step 103, and the candidate encodings generated in step S105, are input into a trained first machine learning model, which has been trained using BLSTMs . One or more physiochemical characteristics assigned to each amino acid of the amino acid sequence may also be used as input to the trained first machine learning model. The output of the trained first machine learning model is a probability that each of the candidate encoding(s) represents a true B-cell epitope on the query protein.

Figure 5 is a flow diagram S200 summarising the steps of a preferred embodiment of training the first machine learning model. At step S201, a plurality of protein complexes are accessed. These may be accessed from publicly available sources such as the Protein Data Bank (PDB) for CBCEs or Immune Epitope Database (IEDB) for LBCEs.

At step S203, the protein complexes accessed in step S201 are analysed to define true epitopes and antigens. Linear BCEs may be defined in the complexes of the IEDB. Further analysis may be required to define true conformational epitopes from protein complexes accessed from the PDB, and further details on these techniques are described herein.

At step S205, each antigen defined in step S203 as having a true B-cell epitope is mapped onto its amino acid sequence. At step S207, each true epitope is encoded onto the amino acid sequence of the corresponding antigen. In this way, a plurality of reference epitope encodings is generated, corresponding to the true epitopes. The reference encodings are typically encoded as binary vectors in the same manner as described above in step S105.

At step S209 of the method, three-dimensional structure and/or surface characteristics of the antigens in an unbound state are accessed, for example by prediction from the amino acid sequence of the antigen obtained in step S205. Typically, the structure and/or surface characteristics of the antigens are predicted using the trained second machine learning model(s) (schematically illustrated at 300). However, other techniques of accessing the structure and/or surface characteristics of the antigens in an unbound state may be used, for example using known computational approaches for predicting 3D protein structures. Typically, the characteristics include the secondary structure, half sphere exposure (both UHSE and LHSE), and relative solvent accessibility.

At step S211, a first reference dataset is generated. The first reference dataset comprises the amino acid sequences of the reference antigens (step S205), the encodings of the true epitopes (step S207) on the corresponding reference antigens, and the accessed (e.g. predicted) structure and/or surface characteristics of the reference antigens in an unbound state. In some embodiments the first reference dataset may further comprise one or more physiochemical characteristics assigned to each amino acid of the reference antigens.

At step S213, the first machine learning model is trained using the first reference dataset generated in step S211. Here, the first machine learning model is or comprises a BLSTM, and may be trained using techniques known in the art. By training the model using the first reference dataset generated through performing the steps S201 to S211, the first machine learning model is trained to learn a relationship between the reference encodings of the true epitopes, the structure and/or surface characteristic(s), and the corresponding true B-cell epitopes.

As discussed above with reference to Figures 4 and 5, in preferred embodiments the second machine learning model(s) (schematically shown at S300) is used to predict structure and/or surface characteristics both when training the first machine learning model (Figure 5), and when using the trained first machine learning model to predict the presence of B-cell epitopes on a protein of interest (Figure 4). A particularly advantageous feature of the use of the second machine learning model is that it is trained to predict structure and/or surface characteristics of the protein in an unbound state. In this way, the first machine learning model is configured to predict BCEs in their unbound state, i.e., having the three dimensional form and characteristics that the Ab "sees" and initiates binding upon.

Figure 6 is a flow diagram S300 summarising the steps of a preferred embodiment of training the second machine learning model.

At step S301, a plurality of second reference proteins in an unbound state (i.e., single protein structures) are accessed. These second reference proteins may be accessed from publicly available databases such as the PDB.

At step S303, the amino acid sequences of the second reference proteins are accessed.

At step S305, the three-dimensional structure and/or surface characteristics of the second reference proteins are accessed. Typically, these include the secondary structure, the relative solvent accessibility, and the upper and lower half sphere exposures. The DSSP algorithm *(*Wolfgang Kabsch and Christian Sander. Dictionary of protein secondary structure: Pattern recognition of hydrogen-bonded and geometrical features. Biopolymers, 22(12):2577-2637, 2018/11/30 December 1983) was used to compute the secondary structure and RSA, and the BioPython package *(*Peter JA Cock, Tiago Antao, Jeffrey T Chang, Brad A Chapman, Cymon J Cox, Andrew Dalke, Iddo Friedberg, Thomas Hamelryck, Frank Kauff, Bartek Wilczynski, et al. Biopython: freely available python tools for computational molecular biology and bioinformatics. Bioinformatics, 25(11):1422-1423, 2009*)* utilised to compute the UHSE and LHSE. The DSSP and BioPython algorithms may be applied to the structure files of the second reference proteins (e.g. obtained from the Protein Data Bank, PDB)

At step S307, a second reference dataset is generated using the amino acid sequences accessed in step S303, and the structure and/or surface characteristics of the second reference proteins accessed in step S305.. In some embodiments the second reference dataset may further comprise one or more physiochemical characteristics assigned to each amino acid of the second reference proteins.

At step S309, the second machine learning model is trained using the second reference dataset (e.g. to learn a relationship between one or more features of the amino acid sequences of the second reference proteins and the corresponding structure and/or surface characteristics). Here, the second machine learning model is or comprises a BLSTM, and may be trained using techniques known in the art. It will be appreciated that different second machine learning models may be trained to predict different respective structure and/or surface characteristics. For example, one second machine learning model may be trained to predict RSA and HSE metrics, and further second machine learning model trained to predict the secondary structure.

The various steps outlined in Figures 4-6 will be described in further detail in the following sections. Section 2 explains pre-training techniques used for all the models. Section 3 presents some preliminary analyses we performed to choose input features for the models. Section 4 presents the structural algorithms for RSA, HSE and SS used in the current BCE algorithms. Section 5 presents the structural algorithms for RSA, HSE and SS that are used as features in the BCE model. Section 6 presents an example of a first machine learning algorithm trained according to embodiments of the invention, used to predict CBCEs, while Section 7 presents an outline of a first model used to predict linear BCEs (LBCEs). In section 8 we present applications of the present example.

## 2 Pre-training Techniques

To ensure reproducible results and avoid the need of random seed in the networks used in our models, we used auto-encoders (aEs). aEs are types of NNs trained on compressed data which mirrors the inputs of the main model. More specifically, an AE takes an input at layer number i = 0 and processes it through an arbitrary number of layers, say $i = 1, \ldots, N$, which constitute the encoder part. It then processes it back through a mirrored structure of the encoder part, called the decoder part. Finally, it returns the output which is the same as the input. The intermediate layers may reduce or expand the dimensions of the input.

Each layer in the main network is converted into an AE, and trained on some compressed amount data. The pre-trained weights of the outer layers are used as constants to the subsequent aEs. This is repeated until all the main network layers are pre-trained. The pre-trained weights can then be used as starting points for the main training procedure.

The following procedure were performed once and the pre-trained weights were used on all the cross-validation folds, test of unseen data, and final model.

First, we randomly generated 10000 protein sequences of length between 50 to 1000 amino-acids. The actual amino-acids used was random as well. We then pre-trained each layer of the neural network as an individual AE on the whole generated data. The pre-training was done for maximum 1000 epochs for each AE, with the possibility of early stopping if the loss did not decrease after 10 epochs. The weights of the outer layers were copied to deeper aEs after pre-training, and were also kept constant during the pre-training of those. The last layer of the model (output) was not pre-trained at all.

This procedure was done once and the same pre-trained weights were used on all the validation procedures. This procedure was faster, it used much more data to pre-train and the same initial weights were used for all downstream analyses.

## 3 Preliminary Analysis and Feature Selection Techniques

We ran various preliminary analyses in order to see how different features correlate with the targets we wanted to predict, as well as with each other. The goal of those analyses was two-fold. First, we tried to find highly feature-targets correlations which would help us decide which features to use in order to predict the targets. Secondly, feature-feature correlations would help us subset the feature-target correlation space by keeping features which were not correlated with each other, thus, reducing the input space of the models. Note, however, that all the analysed using this selection technique mostly capture linear-relationships between feature-targets and feature-features.

3.1 First Method

For deciding which features will be used as input to predict different targets of the models we performed a correlation analysis using the features in Table 0. The analysis was done using the whole data for those models. Namely, the data-set used for those analyses was the ones described in section 4.1. This first method of analysis was run for the 3D structure prediction models and not for the CBCE or LBCE models.

3.1.1 Pre-processing continuous features

Continuous features provide a single value per amino-acid in the alphabet. To model the effect of neighbour amino-acids in a protein sequence, we averaged the values of each feature by scanning the protein sequence using a sliding window of a peptide length of 9 amino-acids. The average feature value was assigned to the middle amino-acid in the peptide. This was done for every feature on each protein sequence in the entire training and test datasets.

3.1.2 Correlation between continuous features and continuous targets

These analyses were done for RSA (relative solvent accessibility), UHSE (upper half-sphere exposure) and LHSE (lower half-sphere exposure). First, we pre-processed the continuous features as described in section 3.1.1. The values of the target variables were used as they were, that is not averaged, since their value already represents neighbours. The Spearman's correlation coefficient was computed for every window-averaged feature in each protein sequence and every target variable. Correlations from all the proteins were then averaged per feature-target pair. For all the three targets we then chose the 30 most correlated features (positively or negatively), and we chose features that were highly correlated with all the three targets.

3.1.3 Correlation between categorical features and continuous targets

These analyses were done for RSA, UHSE and LHSE. Categorical features provide a single value per amino-acid. Those features were used as they are, per-amino-acid in any given sequence. The point-biserial correlation coefficient between feature and target was computed per class. The targets were also used as they are, that is, no windows, for the same reason as in section 3.1.2. Correlations from all the proteins were then averaged per feature-target pair. The top highest (positively or negatively) correlated features with all the three targets were chosen as inputs in the model.

3.1.4 Correlation between continuous features and categorical targets

This analysis was done for SS. The same procedure was used as in Section 3.1.2 for the windows of the features. The only difference is that the target is categorical. For assessing the correlation with continuous features, we used the point-biserial correlation coefficient. Correlations from all the proteins were then averaged per feature-target pair. Top correlated features (positively or negatively) were used in the models.

3.1.5 Correlation between categorical features and categorical targets

This analysis was done for SS. For assessing the dependency between categorical features-targets we used the Chi-Square on each protein. We then did multiple hypothesis correction of the resulting p-values using the Benjamini-Hochberg procedure. Finally, we averaged their corrected p-values from all the sequences, for each feature-response pair, using the harmonic mean. The top most significant feature-target correlations were used in the models.

3.2 Second Method

For deciding which features will be used as input to predict different targets of the models we did a correlation analysis using the features in Table 0. Further information on each feature (e.g. characteristic) is given in the Appendix. The analysis was done using the whole data for those models. Namely, the data-set used for those analyses was the ones described in section 4.1 (for RSA, UHSE, LHSE, SS) and section 5.1 and 6.1 (for CBCE and LBCE). Which data we used depends on the model tested, accordingly. Moreover, for the CBCE and LBCE analyses, those two data-sets were merged.

Table 0: Table of Global Features

| Feature | Abbreviation | Feature | Abbreviation |
|---|---|---|---|
| Mass [25] | MassKnown | Refractivity [30] | REFRJones |
| Bulkiness [29] | BulkZimmerman | Normalized consensus hydrophobicity [31] | NCHPBCEisenberg |
| Hydrophilicity [32] | HPLCParker | Hydrophilicity [1] | HPLCHopp |
| Polarity [2] | POLGrantham | Average surrounding hydrophobicity [15] | ASHPLCManavaian |
| Composition [2] | COMPGrantham | Hydrophobicity of physiological L-alpha amino-acids [33] | HPBCOPLAAABlack |
| Molecular volume [2] | MOLVGrantham | Hydrophobicity [34] | HPBCFauchere |
| Recognition factors [17] | RECFFraga | Free energy of transfer from inside to outside of a globular protein [3] | FEOTITOJanin |
| Hydrophobicity [35] | OMHSweet | Membrane buried helix parameter [5] | MBHPRao |
| Hydropathicity [36] | HPTCKyte | Hydrophobicity [37] | HPBCTanford |
| Hydrophobicity [38] | HPBCAbraham | Antigenicity value X 10 [18] | AVX10Weilling |
| Hydrophobicity [19] | HPBCBull | Hydrophobicity indices at ph determined by HPLC [39] | HPBCPH75Cowan |
| Hydrophobicity [4] | HPBCGuy | Retention coefficient in HFBA [20] | RCHFBABrowne |
| Hydrophobicity [6] | HPBCMiyazawa | Transmembrane tendency [7] | TransTrendZhao |
| Hydrophobicity [40] | HPBCRoseman | Retention coefficient in HPLC, pH 7.4 [22] | RCHPLCPH74Meek |
| Hydration potential [21] | HPWolfenden | Molar fraction of accessible residues [3] | MOLFAR3220Janin |
| Hydrophobic constants [26] | HPLCWilson | Mean fractional area loss [9] | MFALRose |
| Hydrophobicity indices at ph 3.4 [39] | HPLCph34Cowan | Average area buried on transfer from standard state to folded protein [9] | AABOTRose |
| Mobilities on chromatography paper [41] | MOAAOCPAboder in | Conformational parameter for alpha helix [10] | CPFAH29Chou |
| Retention coefficient in TFA [20] | RCTFABrowne | Conformational parameter for beta-turn [10] | CPFBT29Chou |
| Retention coefficient in HPLC, pH 2.1 [22] | RCHPLCph21 Mee k | Conformational parameter for beta-sheet [14] | CPFBSDeleage |
| Molar fraction of buried residues [3] | MOLF20010BRJan in | Conformational parameter for coil [14] | CPFCDeleage |
| Proportion of residues buried [8] | PORB12Clothia | Normalized frequency for beta-sheet | NFFBSLevitt |
| Average *flexibility* index [23] | AFIBhaskaran | Conformational preference for total beta strand (antiparallel+parallel) [11] | CPFTBSAPLifson |
| Conformational parameter for beta-sheet [10] | CPFBS29Chou | Conformational preference for total beta strand [11] | CPFTBSLifson |

(continued)

| Feature | Abbreviation | Feature | Abbreviation |
|---|---|---|---|
| Conformational parameter for alpha helix [14] | CPFAHDeleage | Surface accessibility [13] | SASEmini |
| Conformational parameter for beta-turn [14] | CPFBTDeleage | Antigenicity [27] | AGCKolaskar |
| Normalized frequency for alpha helix [16] | NFFAHLevitt | Side chain classes [12, p.51] | SCClassKnown |
| Normalized frequency for beta-turn [16] | NFFBTLevitt | Side chain polarity [12, p51] | SCPolKnown |
| Conformational preference for antiparallel beta strand [11] | CPFABSLifson | Side chain charge [12, p51] | SCPolarityKnown |
| Overall amino-acid composition [28] | OAACMccaldon | Isoelectric points [25] | PLKnown |
| Relative mutability of amino-acids [24] | RMOAADayhoff | Van der Waals volume [25] | VanDerWaalsKnow n |
| Known Number of codon(s) coding for each amino-acid in universal genetic code [12] | NCFAAKnown | PK1 a-COOH [25] | PK1Known |
| Polarity [81] | POLZimmerman | PK2 a-+NH3 [25] | PK2Known |

3.2.1 Pre-processing continuous features

The same procedure as in section 3.1.1.

3.2.2 Feature-Feature correlations

First, we ran a feature-feature correlation analysis of all the features in Table 0. We did this on two different datasets, one for the one in section 5.1 and one for the merged one from sections 6.1 and 7.1. The purpose of those analysis was to identify high correlated feature pairs, such that if both features from a correlated pair were correlated with a response in a later analysis, only one of them will be used as input feature for the corresponding model. Thus, reducing the input features space.

We first pre-processed the continuous features as described in section 3.2.1. Categorical features were used as they are, that is, a single value per-amino-acid in every sequence. We then computed correlation metrics for every feature pair on every sequence. For continuous-continuous feature pairs we used Spearman's Correlation coefficient. For continuous-categorical features we used Kendall rank correlation coefficient, and for categorical-categorical we used Mutual information criterion. For every pair-wise feature coefficient population we removed outliers using the IQR (boxplot) method. The median of the whole population is then taken as the final coefficient for every pair-wise feature-feature correlation. We defined as correlated continuous-continuous feature pairs with median Spearman's correlation coefficient > 0.7, continuous-categorical feature pairs with Kendall rank correlation coefficient > 0.7, and categorical-categorical feature pairs with Mutual information > 2.

Figure 7 shows the correlation results of the feature-feature pairs for the RSA/HSE model. Figures 7(a) to (c) show feature-feature correlations using the data from 6.1 and 7.1. Figures 7(d) to (f) show feature-feature correlations using the data from 5.1. Black dots correspond to correlated pairs. The heat-maps are symmetric. Note that not all feature names are written in the x- and y-axis. For the 6.1 and 7.1 datasets, the features for RSA, UHSE, LHSE and SS were predicted using the current models (see 4). For the 5.1 dataset, those figures were the observed ones, from PDB. As we can see, for all datasets, continuous-continuous feature-pairs resulted in many correlated features. For continuous-categorical feature-pairs only a few were correlated, while no correlation was observed between categorical-categorical feature pairs.

3.2.3 Feature-response correlations

For each of the following feature-response correlation analyses, we followed the same procedure. For continuous features we did the same pre-processing as described in section 3.1.1, but we used windows of 0, 3, 7, 9 and 11. We computed the correlations based on every window for each feature-response pair in every sequence of the corresponding data. Moreover, we removed outliers using the IQR (box-plot) method, and then computed the median of each correlation population.

We merged all the correlations from every window for each feature into one population, we also removed outliers with the same method, and finally we recomputed the median of that global population. The final "pooled" median was used to rank the features from highest to lowest correlated with the corresponding response. Categorical responses as well as predicted RSA, UHSE and LHSE were used as they are, that is, no windows.

Each of the following analyses gave us an indication of highly correlated feature-response pairs. We chose the top correlated features with the responses in such a way that if two features were correlated with the response and also with each other (using the results from section 3.2.3), only the one feature with the highest correlation with the response was used.

3.2.3.1 Correlation between continuous features and continuous targets

These analyses were done for RSA, UHSE and LHSE. We used data from 5.1. The metric used here was Spearman's Correlation coefficient. See Figure 8 for the results, and section 5 for the models and the features they used.

Figure 8 shows the Spearman's correlation coefficients for continuous features against (a) RSA, (b) UHSE and (c) LHSE. On each figure, the x-axis is the correlation, the y-axis are the feature names, sorted from highest to lowest absolute correlation of the median of the population. Each plot is from all windows.

3.2.3.2 Correlation between categorical features and continuous targets

These analyses were done for RSA, UHSE and LHSE. We used data from 5.1. The metric used here was Kendall rank correlation coefficient. See Figure 9 for the results, and section 5 for the models and the features they used.

Figure 9 shows the Kendall's correlation coefficients for categorical features against (a) RSA, (b) UHSE and (c) LHSE. On each figure, the x-axis is the correlation, the y-axis are the feature names, sorted from highest to lowest absolute correlation of the median of the population. Each plot is from all windows.

3.2.3.3 Correlation between continuous features and categorical targets

These analyses were done for SS, CBCE and LBCE. We used data from 5.1 (for SS) and merged 6.1 and 7.1 (for CBCE and LBCE). The metric used here was Kendall rank correlation coefficient. See Figure 10 for the results, and section 5, 6 and 7 for the models and the features they used.

Figure 10 shows the Kendall's correlation coefficients for continuous features against (a) SS, (b) CBCE and (c) LBCE. On each figure, the x-axis is the correlation, the y-axis are the feature names, sorted from highest to lowest absolute correlation of the median of the population. Each plot is from all windows.

3.2.3.4 Correlation between categorical features and categorical targets

These analyses were done for SS, CBCE and LBCE. We used data from 5.1 (for SS) and merged 6.1 and 7.1 (for CBCE and LBCE). The metric used here was Mutual information criterion. See Figure 11 for the results, and section 5, 6 and 7 for the models and the features they used.

Figure 11 shows the Mutual Information criterion for categorical features against (a) SS, (b) CBCE and (c) LBCE. On each figure, the x-axis is the correlation, the y-axis are the feature names, sorted from highest to lowest absolute correlation of the median of the population. Each plot is from all windows.

4 Predicting 3D features ("Second machine learning algorithm")

We created two BLSTM models to predict structure and surface characteristics of proteins from their native sequences. Those models are later used in the first machine learning model, to predict structural and surface features of the Ag before the binding event with the Ab takes place. For structural characteristics we chose to create a model for the SS, and for surface characteristics we chose RSA, UHSE and LHSE.

4.1 Data preparation for the 3D features

To accurately predict the SS, RSA, UHSE and LHSE from a native protein sequence, we used all available high quality 3D protein structures from the PDB from all available organisms. The goal of these models was to predict the most pertinent surface and structural characteristics of single proteins, that are not affected by binding to any other protein interaction, including Ab binding. Therefore, we only kept structures that are not bound by any other structure or molecule. Structures containing more than one copy of the same molecule, but slightly different conformation, are kept in the data. We filtered the structures and kept only those with $\leq$ 3 Angstrom resolution, ensuring that every atom of each amino-acid is mapped with coordinates, and with protein chains longer than 200 amino-acids.

After filtering, the data-base consisted of 41592 total structures. Those structures contained 70489 protein sequences which resulted in 53524 unique sequences. Subsequences of longer sequences were kept as different data point entries. The reason being that their 3D conformational folding properties may be different because of their shorter length.

The DSSP algorithm was used to compute the SS and the RSA for each molecule in each structure file. DSSP computes the following secondary structure classes for a protein sequence; $\alpha$-helix, 310-helix, $\pi$-helix, isolated $\beta$-bridge, $\beta$-strand, turn, bend and coil. We merged those classes into three super-classes; Helices ($\alpha$-helix, 310-helix and $\pi$-helix), Strands (isolated $\beta$-bridge and $\beta$-strand) and Coils (turn, bend and coil). Finally, the BioPython package was used in to compute the UHSE and LHSE.

At the end of the filtering, each amino-acid in the data base was assigned a value for the RSA, UHSE, LHSE and a class for the SS. To create a unique database, the mean per amino-acid was taken for RSA, UHSE, LHSE among identical sequences. Finally, amino-acids of identical sequences but with different SS classes, were assigned to the coil class.

4.2 Prediction model for RSA and HSE struct 3d rsa hse m2 model)

We chose to create a single BLSTM model for predicting all surface features. More specifically, the model predicts RSA, UHSE and LHSE from a primary protein sequence. Although LHSE may not give useful information about the surface position of an amino-acid, it may help predicting UHSE more accurately, since both together are forming a probability metric around each amino-acid.

This is a BLSTM model which takes as inputs a batch of features, each computed per amino-acid from each input sequence and predicts a three-way output. For each protein sequence given as input, a value for each RSA, UHSE and LHSE is predicted per amino-acid.

For all the three outputs the individual losses were calculated on the basis of the mean square error (MSE), but in principle the loss is not limited to this function. The global model loss was the weighted sum of the individual losses with weights: 50, 100 and 125 for RSA, UHSE and LHSE, respectively. The weights were decided by first training the model without them and then see the differences in the magnitude of the three losses. The weights contributed in such a way that the three losses gave the same contribution to the global model loss.

Figure 12 shows a schematic simplified architecture of the model. As shown, the first layer is the input layer. The input layer may comprise one or more input nodes, for example to receive the input features discussed below. Each input node is coupled to a respective masking node in a masking layer. Similarly, each masking node is coupled to a respective BLSTM node in a BLSTM layer. Each BLSTM node is coupled to a concatenation node in a concatenation layer. Thus, a plurality of outputs from the nodes of the preceding BLSTM layer are concentrated together in the concatenation layer. The concatenation layer can be coupled to one or more further BLSTM layers, each comprising a corresponding BLSTM node. The final of these BLSTM nodes is coupled to an output layer, which in this case comprises a plurality of output nodes corresponding, respectively, to the RSA, UHSE and LHSE output predictions.

Note: the input features were computed per amino-acid as they are, not averaged by windows.

4.2.1 Preliminary Analysis and Features Used

See procedure in section 3.1 for the procedure. The 14 features that we chose to use in the model (including the protein-sequence in binary representation) can be seen in Table 1.

Table 1: Features used in the struct 3d rsa hse m2 model.

| Feature | \| Abbreviation | Feature | Abbreviation |
|---|---|---|---|
| Polarity [2] | POLGrantham | Free energy of transfer from inside to outside of a globular protein [3] | FEOTITOJanin |
| Hydrophobicity [4] | HPBCGuy | Membrane buried helix parameter [5] | MBHPRao |
| Hydrophobicity [6] | HPBCMiyazawa | Transmembrane tendency [7] | TransTrendZhao |
| Proportion of residues buried [8] | PORB12Clothia | Mean fractional area loss [9] | MFALRose |

(continued)

| Feature | Abbreviation | Feature | Abbreviation |
|---|---|---|---|
| Conformational parameter for beta-sheet [10] | CPFBS29Chou | Conformational preference for total beta strand [11] | CPFTBSLifson |
| Side chain classes [12, p51] | SCClassKnown | Surface accessibility [13] | SASEmini |
| Side chain polarity [12, p51] | SCPolKnown | Binary representation of protein sequence | BPS |

4.2.2 Pre-training

See section 2.1.

4.2.3 Cross Validation (CV) results

We used- 80%of the data on 5-fold cross-validation to assess the performance of the model. See Figure 13 for the Spearman's correlation coefficient metric. The models performed well, without overfitting and had high performance for all the evaluation metrics. Moreover, the model demonstrated high stability, with only a small variation observed between different CV runs.

Figure 13 shows the results for RSA/HSE model. Figures 13(a)-(c) show the Spearman's correlation coefficients, and 12(d)-(f) the Mean Square Error (MSE) losses. The x-axis is the epochs, the y-axis is the metric values. The plots are the average of 5-fold CVs including Gaussian CI. The blue line is for training and the red (dashed) for validation sets.

4.2.4 Test results

We used the remaining ~ 20% of the data as test data to assess the performance of the model on completely unseen data. See Figure 14 for the Spearman's correlation coefficient metric. The models performed well, without overfitting and had high performance for all evaluation metrics.

Figure 14 shows the results for RSA/HSE model. (a)-(c): Spearman's correlation coefficients. (d)-(f): MSE losses. The x-axis is the epochs, the y-axis is the metric values. The solid line is for the training and the dotted for the validation set.

4.2.5 Final Training

The final training was done on the entire dataset. Using the optimal number of epochs determined by the cross-validation procedure. See Figure 15 for the Spearman's correlation coefficient metric. The models seem to perform well for all metrics. The model for trained for 70 epochs, chosen based on the CV and test-set analysis.

Figure 15 shows the results for RSA/HSE model. (a)-(c): Spearman's correlation coefficients (d)-(f): MSE losses. The x-axis is the epochs, the y-axis is the metric values.

4.3 Prediction model for SS (struct 3d ss m3 model)

We chose to create a BLSTM model for predicting a three-tier class output for SS. The model uses the categorical cross-entropy loss to assign one of the following classes to each amino-acid in its input sequence; Helices, Strands and Coils. The architecture of the model is substantially the same as that illustrated in igure 11, with a single output node in the output layer. Note: the input features were computed per amino-acid as they are not averaged over sliding windows.

4.3.1 Preliminary Analysis and Features Used

See procedure in section 3.1. The 8 features that we chose to use in the model (including the protein-sequence in binary representation) can be seen in Table 2.

Table 2: Features used in the struct 3d ss m3 model.

| Feature | Abbreviation | Feature | Abbreviation |
|---|---|---|---|
| Conformational parameter for coil [14] | CPFCDeleage | Average surrounding hydrophobicity [15] | ASHPLCManavaian |

(continued)

| Feature | Abbreviation | Feature | Abbreviation |
|---|---|---|---|
| Conformational preference for total beta strand (antiparallel+paral lel) [11] | CPFTBSAPLifson | Normalized frequency for beta-sheet [16] | NFFBSLevitt |
| Conformational parameter for alpha helix [14] | CPFAHDeleage | Side chain classes [12, p.51] | SCClassKnown |
| Normalized frequency for beta-turn [16] | NFFBTLevitt | Binary representation of protein sequence | BPS |

4.3.2 CV results

We used ~ 80%of the data on 5-fold cross-validation to assess the performance of the model. See Figure 16 for the precision recall metric. The model seems to perform well, without significant overfit and gives hight performance for all metrics. Moreover, small variation is observed between different CV runs for the loss. Finally, for demonstration purposes, the models were trained for 70 epochs with slight overfit after 60.

Figure 16 illustrates the results for SS model. Figures 16(a)-(e) show PR curves. The x-axis is the TPR, the y-axis is the PPV. Figure 16(f) shows categorical cross-entropy loss. The x-axis is the epochs, the y-axis is the loss values. The plot is the average of 5-fold CVs including Gaussian Cl. The blue line is for training and the red (dashed) for validation sets.

4.3.3 Test results

We used the rest ~ 20% of the data as test data to assess the performance on completely unseen data. See Figure 17 for the precision-recall metric. The model performed well, without significant overfitting and had high performance for all evaluation metrics. The model was trained for 100 epochs, chosen based on the CV analysis, so slightly more epochs were required than the CV analysis. Figure 17 shows the results for SS model. Figure 17(a): PR curves. The x-axis is the TPR, the y-axis is the PPV. Figure 17(b): Categorical cross-entropy loss. The x-axis is the epochs, the y-axis is the loss values. The solid line is for the training and the dotted for the validation set.

4.3.4 Final Training

The final training was done on the whole data. Using the optimal number of epochs determined by the cross-validation procedure. See Figure 18 for the F1 metric. The final trained model performed well, without significant overfitting and gave high performance for all metrics. The model for trained for 100 epochs, chosen based on the CV analysis, so slightly more epochs than the CV and test-set analysis.

Figure 18 shows results for SS model. Figure 18(a)-(c): F1 metrics. The x-axis is the epochs, the y-axis is the F1 value. Figure 18(d): Categorical cross-entropy loss. The x-axis is the epochs, the y-axis is the loss values.

5 Predicting 3D features ("Second Machine Learning Algorithm") (Version 2)

5.1 Data preparation for the 3D features (Version 2)

Same as Section 4.1. The only difference is that for the SS class there was a misplacement of one of the classes. Namely, the isolated β-bridge class was given to the Coil super-class, while here it is moved correctly to the Strand super-class. This small detail only affects some parts of the SS models data.

5.2 Prediction model for RSA and HSE (Version 2) (rsa hse m4 v1 model)

Same as section 4.2, only the preliminary analysis has changed and the input features. The model is deeper as well. The architecture of the model is substantially the same as that illustrated in Figure 12. Note: the input features were computed per amino-acid as they are, not averaged over sliding windows. The global model loss was the weighted sum of the individual ones as in the previous model. However, the weights this time were: 1, 10 and 125 for RSA, UHSE and LHSE, respectively. The weights were decided by first training the model without them, and see the differences in the magnitude of the three losses.

5.2.1 Preliminary Analysis and Features Used

See section 3.2 for the procedure. The 19 features that we chose to use in the model (including the protein-sequence in binary representation) can be seen in Table 3.

Table 3: Features used in the rsa hse m4 v1 model.

| Feature | Abbreviation | Feature | Abbreviation |
|---|---|---|---|
| Recognition factors [17] | RECFFraga | Antigenicity value X 10 [18] | AVX10Weilling |
| Hydrophobicity [19] | HPBCBull | Retention coefficient in HFBA [20] | RCHFBABrown e |
| Hydration potential [21] | HPWolfenden | Retention coefficient in HPLC, pH 7.4 [22] | RCHPLCPH74 Meek |
| Average area buried on transfer from standard state to folded protein [9] | AABOTRose | Mean fractional area loss [9] | MFALRose |
| Conformational parameter for beta-turn [10] | CPFBT29Cho u | Conformational parameter for alpha helix [10] | CPFAH29Chou |
| Average *fle*xibility index [23] | AFIBhaskara n | Side chain polarity [12, p51] | SCPolKnown |
| Normalized frequency for alpha helix [16] | NFFAHLevitt | Side chain classes [12, p51] | SCClassKnown |
| Relative mutability of amino-acids [24] | RMOAADayh *off* | Isoelectric points [25] | PLKnown |
| Known Number of codon(s) coding for each amino-acid in universal genetic code [12] | NCFAAKnown | PK2 a-+NH3 [25] | PK2Known |
| Binary representation of protein sequence | BPS | | |

5.2.2 Pre-training

See section 2.2.

5.2.3 CV results

We used ~ 80% of the data on 5-fold cross-validation to assess the performance of the model.

5.2.5 Test results

We used the rest ~ 20% of the data as test data to see the performance on completely unseen data.

5.2.6 Final Training

The final training was done on the whole data. Using the optimal number of epochs determined by the cross-validation procedure.

5.3 Prediction model for SS (Version 2) (ss3 m6 v1 model)

Similar to the results described section 4.3. The architecture of the model is substantially the same as that illustrated in Figure 12. Note: the input features were computed per amino-acid as they are, not averaged by windows.

5.3.1 Outliers

Same as section 4.3.

5.3.2 Preliminary Analysis and Features Used

See section 3.2 for the procedure. The 19 features that we chose to use in the model (including the protein-sequence in binary representation) can be seen in table 4.

Table 4: Features used in the ss3 m6 v1 model.

| Feature | Abbreviation | Feature | Abbreviation |
|---|---|---|---|
| Molecular volume [2] | MOLVGrantham | Recognition factors [17] | RECFFraga |
| Hydrophobicity [19] | HPBCBull | Antigenicity value X 10 [18] | AVX10Weilling |
| Hydrophobic constants [26] | HPLCWilson | Molar fraction of accessible residues [3] | MOLFAR3220Janin |
| Retention coefficient in TFA [20] | RCTFABrowne | Conformational parameter for coil [14] | CPFCDeleage |
| Proportion of residues buried [8] | PORB12Clothia | Side chain classes [12, p51] | SCClassKnown |
| Conformational parameter for beta-turn [14] | CPFBTDeleage | Antigenicity [27] | AGCKolaskar |
| Relative mutability of amino-acids [24] | RMOAADayhoff | Side chain polarity [12, p51] | SCPolKnown |
| Overall amino-acid composition [28] | OAACMccaldon | Isoelectric points [25] | PLKnown |
| PK2 a-+NH3 [25] | PK2Known | PK1 a-COOH [25] | PK1Known |
| Binary representation of protein sequence | BPS | | |

5.3.3 Pre-training

See section 2.2.

5.3.4 CV results

We used ~ 80% of the data on 5-fold cross-validation to assess the performance of the model.

5.3.5 Test results

We used the rest ~ 20% of the data as test data to see the performance on completely unseen data.

5.3.6 Final Training

The final training was done on the whole data. Using the optimal number of epochs determined by the cross-validation procedure.

6 First Machine Learning Model (CBCEs)

For predicting CBCEs we used BLSTMs based models. This allowed us to model the whole protein sequences bidirectionally as one observation, without the need of segmenting it. Therefore, distant, and contextual amino-acid relationships are captured by the model. Moreover, the use of BLSTMs allowed us to train different protein lengths simultaneously. The main goal of our models was to create non-Ab specific CBCE predictors. This was achieved by the manner we prepared the training data described in section 6.1.

6.1 Data preparation

For modelling CBCEs we downloaded non-obsolete protein complexes from the PDB. We allowed structures of any resolution and organisms, as long as they had at least three different protein chains. The reason for this is that two of the chains may be the $V_H$ and $V_L$ chains of an Ab, and the third chain may be an Ag. Of course, there may be multiple Abs or Ags in one PDB structure.

We created a local database using all immunoglobulin V-, D- and J-region genes from the IMGT (The international ImMunoGeneTics information system) database. Those genes were downloaded for both $V_H$ and $V_L$ chains, from all the available organisms, that is; human, mouse, rhesus monkey, rabbit and rat. The IMGT provides information about the $V_H$ and $V_L$ chains of an Ab, and its paratope regions (the CDRs [Complementary determining region of the antibody] and FRs [framework region of antibody] of each chain).

To identify the Abs in this local database we used IgBlast *(*Jian Ye, Ning Ma, Thomas L Madden, and James M Ostell. Igblast: an immunoglobulin variable domain sequence analysis tool. Nucleic Acids Res, 41 (Web Server issue): W34-40, Jul 2013*)* for protein sequences on the IMGT database. For identifying the paratope in a chain we used the Kabat system that the IgBlast provides (G Johnson and T T Wu. Kabat database and its applications: 30 years after the first variability plot. Nucleic acids research, 28(1):214-218, 01 2000*)*. We considered $V_H$ and $V_L$ chains as valid, only if at least their CDR1 and CDR2 were found by IgBlast. Since CDR3 is more difficult to map, we allowed it to be missing in the search. We then aligned all the protein chains of each structure to that database. Structures were filtered out if they did not have at least one chain mapped as a valid $V_H$ and one as a valid $V_L$. Any other chain that was not mapped as $V_H$ or $V_L$ was assumed to be an Ag chain. We considered as valid Ag chains, those chains that were longer than 100 amino-acids and were not bound by any other chain other than a $V_H$ or $V_L$ chain. Finally, only structures including at least one $V_H$, $V_L$ and Ag chains were taken to further analyses.

We then paired $V_H$ and $V_L$ chains in each structure to recreate valid Abs. In case there were multiple $V_H$ and $V_L$ chains in one single structure, we measured the mean distance from every atom on each $V_H$ to every atom on each $V_L$ chain. $V_H$ and $V_L$ chains with the minimum mean atom distance were assigned as pairs and assumed to belong to one single Ab. Stand-alone $V_H$ or $V_L$ chains were not considered on the downstream analysis as they could not define a complete Ab. A paratope analysis provided information about the contacts formed with each Ag. Two amino-acids were assumed to be in contact if any of their atoms were located within a certain probe distance from each other. We computed the total number of Ag amino-acids that form contacts with each paratope's parts, using probe distances of 4, 6 and 8 Angstroms. Figure 19(a) shows the normalised mean of those contacts (x-axis shows the amino acids, y-axis shows the paratope parts). Most of the contacts are made with CDR1 and CDR2 of the $V_H$s and CDR1 and CDR3 of the $V_L$s. The absence of CDR3 on $V_H$s may be due to the difficulty of identifying it using IgBlast. Moreover, the FR regions do not seem to form as many contacts as the CDR regions, as expected. Additionally, the standard deviation of the total contacts per amino-acid and paratope part is relatively low in Figure 19(b), indicating that similar number of contacts are made between different Ags and Abs. This could be an indication of the possibility of predicting CBCEs without any further information about the actual Abs. Figure 19(b) shows the STD for number of contacts. X-axis shows the amino-acids, y-axis shows the paratope parts.

For each $V_H$ and $V_L$ chain pair we defined a single CBCE. This was done by first identifying atoms on any Ag whose distance from the CDRs regions of any $V_H$ and $V_L$ chain pair was $\leq$ 4 Angstroms. The amino-acids that those atoms belonged to were defined as contacts between the Ag and the Ab, that is, they defined the CBCE. Multiple CBCEs from different Abs could be defined on the same Ag. Finally, structures that did not define any CBCE within the 4 Angstrom distance were discarded.

Observed CBCEs were also mapped to similar Ag. Undiscovered CBCEs could increase the false negative rate of the prediction models. To decrease the possibility of assigning undiscovered CBCEs as negative data we copied observed CBCEs to similar Ags. First we identified clusters of similar Ags using BlastPlus with > 90% similarity and at most 2 gaps. CBCEs were copied from an Ag in a cluster to all the other Ags in the same cluster, as long as their corresponding position and distancing was exactly the same based on the mapping from BlastPlus.

Lastly, we created a unique Ag local database. Duplicated Ags were completely removed from the data. On the other hand, Ag sequences that were subsequences of longer Ags were kept in the data and treated as different observations. The resulted database consisted of 1003 Ag sequences in FASTA format and 12968 CBCEs, of which 6986 were unique. Each Ag sequence was associated with at least one CBCE.

### 6.2 CBCE models summary

We have created two main CBCE predictors, namely model 13 conf v1 and model 17 conf v1. There are two differences between the models. Firstly, the negative data generator is different, see section 6.3 for details. Secondly, the model 13 conf v1 model gives two outputs, while model 17 conf v1 gives one. Both models take as input features, among others, a permutation vector. Every sequence in the data is associated with at least one true CBCE, those CBCEs are turned into binary 2D vectors and are given as input to the models. The common output of the two models is a probability, so both models are basically asking the question: "Is this permutation vector, on this specific sequence, a true CBCE or not?". The second output of the model 13 conf v1 model is the permutation vector itself. This part of the model works as an AE, where it returns a probability per amino-acid. This output can be seen as a contribution of each amino-acid in the sequence to the specific CBCE in question.

The goal of those models was to predict CBCEs on the protein Ag sequence, before the Ab binding event took place. The dataset used, described in section 6.1, contains the linear protein sequence and the true CBCEs. Therefore, there is no information about any structural or surface characteristic of those protein sequences. However, we need information about how each protein sequence's secondary structure and surface would look like before the binding event. Therefore, we used our prediction models for RSA, UHSE and LHSE (model struct 3d rsa hse m2 res) and SS (model struct 3d ss m3 res1) described in section 4 to predict those characteristics in every protein in the dataset.

There are three main differences of our models compared to the currently existing publicly available CBCE algorithms. Firstly, we use BLSTMs which allows us to capture relationships between distant and contextual amino-acids which may constitute a CBCE on a native folded 3D protein structure. On the other hand, the other algorithms are segmenting the protein sequences in k-mers and thus, not capturing these important relationships. Secondly, the training dataset is created in such a way to predict true CBCEs before the binding event takes place. This difference brings the model conceptually closer to modelling the reality of Ab binding. As the 3D folded protein structure is exactly what an Ab sees and triggers the binding, and not what an Ab has already seen and bound on.

Thirdly, the currently existing publicly available CBCE algorithms require an experimentally measured, or potentially predicted, 3D protein Ag structure in order to predict epitopes. Our first machine learning model does not require the 3D coordinates of every atom in each amino acid on the Ag or Ab. Finally, our model addresses the epitope question in a conceptually very different manner, but in a manner that is more fined tuned to capture bone fide ("true") BCEs. We predict probabilities of stand-alone CBCEs on each Ag as a pair or independent class, and therefore multiple CBCEs on the same protein sequence can be discovered and assessed independently. On the other hand, other algorithms provide probabilities per amino-acid, without the ability of separation between different CBCEs.

Figure 20(a) schematically shows the architecture of the model 13 conf v1 model. The architecture of the model is substantially the same as that shown in Figure 12. Here, the output layer comprises two output nodes. The BCE amino acid output (BCE_aa) is the actual permutation output, where binary cross-entropy loss was used. The BCE perm output is a probability vector indicating if the input permutation is a true CBCE (second position on vector) or not (first position on vector). Figure 20(b) shows the architecture of the model 17 conf v1 model. The BCE perm represents the same output as the previous model. Note: the input features were computed per amino-acid as they are, not averaged by windows.

For both models, the BCE perm output is computed from amino-acid values. The last layer of the output is a Dense layer with sigmoid activation. Such that, for each protein sequence, a value in e [0, 1] is returned per amino-acid. Then a 2-class probability vector is computed for that sequence as:

$$[1 - \sum_{i=0}^{N} x_i/N, \sum_{i=0}^{N} x_i/N],$$

where N is the length of the sequence and xi is the dense layer output on amino-acid at position i on the sequence. This 2-class probability vector is then used in the binary cross-entropy loss.

The model 13 conf v1 model had weighted global loss. The global model loss was the weighted sum of the individual ones, that is BCE perm and BCE aa, with weights: 1, and 1, respectively. Different weights could be tested as well.

### 6.3 Negative Data

If we were to model the CBCEs as per amino-acid, then the positive data would be every amino-acid in a sequence which is a part of any true CBCE, and the negative data would be any other amino-acid on the same sequence. However, since we model the whole CBCE as a single permutation vector of a given protein sequence, the response to this vector is a single probability (that is output BCE perm from section 6.2). Since we only have observed true positive CBCEs from the data in section 6.1, we do not have any permutation corresponding to negative data.

We developed a solution to improve the performance of the models, whereby negative permutation data was generated in a manner which resample the ground truth as close as possible. To cover a large spectrum of negative data and make the models more robust, we generated negative permutations for every sequence in the data, on every epoch during training. The two different generation schemes are described in the following sections.

#### 6.3.1 CBCE model (model 13 conf v1)

For this model we generated completely random permutations. For each sequence in the training and validation data we generated 10 completely random permutations and assumed that they are not true CBCEs. Both the total number of amino-acids and the placement of those in the given sequence were random. New permutations were generated on each epoch.

The advantage of this method is that because of the complete randomness, it is quite unlikely that any generated CBCE permutation will be false negative. However, the disadvantage is that the randomly generated CBCE permutations may be extremely different than the true CBCE permutations.

6.3.2 CBCE model (model 17 conf v1)

For this model we applied three different types of negative data generating methods. For each sequence in the training and validation data we generated 30 completely random permutations, 10 from each method, and assumed that they are not true CBCEs. The first method is like the one described in 6.3.1. The second and third methods were applied on every true CBCE of the given protein sequence. The second method kept the first and last amino-acid of a given true CBCE at the correct position, while it randomly shuffled the internal CBCE amino-acids indicators inside the region. The third method kept the total amount of amino-acids of a true CBCE, as well their linear distance, constant. It then randomly shifted the whole true CBCE on other parts of the protein. New permutations were generated on each epoch from every method.

The advantage of this method is that the randomly generated CBCE permutations cover both extremely similar and extremely different true CBCE permutations. This will probably lead to that the algorithm is more robust to both positive and negative data. On the other side, the disadvantage of the method is that the false negatives may increase . Leading to lower performance on new data.

6.4 Outliers

We removed outliers before any training of either model. We did this by applying Isolation forests (IF) on the total amino-acids of each observed CBCE in our data from section 5.1. We run the algorithm for every observed amount of total amino-acids as the max samples argument in the IF algorithm from the sklearn Python package *(*F. Pedregosa, G. Varoquaux, A. Gramfort, V. Michel, B. Thirion, O. Grisel, M. Blondel, P. Prettenhofer, R. Weiss, V. Dubourg, J. Vanderplas, A. Passes, D. Cournapeau, M. Brucher, M. Perrot, and E. Duchesnay. Scikit-learn: Machine learning in Python. Journal of Machine Learning Research, 12:2825-2830, 2011*.)* The most common minimum and maximum borders from all the runs were taken as global borders for inliers.

Figure 21 shows the total amount of amino-acids from all the observed CBCEs. Figures 21(a) and 21(b) show, respectively, density and box-plot of observed amount of total amino-acids in the true CBCEs. Figures 21(c) and 21(d) shown log-scales of Figures 21(a) and 21(b) respectively. Where we see that the median amount is at 10 amino-acids. After the outlier analysis, we found that valid CBCEs should contain a total amount of amino-acids in range, which is lower than what discussed in section 1.3. However, those studies did not perform any outlier detection or removal of their observed CBCEs. As we can see from Figure 21, the maximum amount of total amino-acids before outlier removal is around 30 to 35, which corresponds with that reported in the previous studies. The outliers were finally removed from the data, and the models were trained without them.

6.5 Preliminary Analysis and Features Used

See section 3.2 for the procedure. The 14 features that we chose to use in the models (including the protein-sequence in binary representation and the permutation vectors) is shown in in Table 5. Note that the RSA and SS were predicted by models struct 3d rsa hse m2 res and struct 3d ss m3 res1, respectively, described in section 4. The same features were used for both model model 13 conf v1 and model 17 conf v1.

Table 5: Features used in the model 13 conf v1 and model 17 conf v1 models.

| **Feature** | **Abbreviation** | **Feature** | **Abbreviation** |
|---|---|---|---|
| Bulkiness [29] | BulkZimmerman | Polarity [2] | POLGrantham |
| Molar fraction of buried residues [3] | MOLF2001OBRJani n | Conformational parameter for beta-turn [10] | CPFBT29Chou |
| Average flexibility index [23] | AFIBhaskaran | Normalized frequency for beta-sheet [16] | NFFBSLevi*tt* |
| Normalized frequency for alpha helix [16] | NFFAHLevitt | Side chain classes [12, p51] | SCClassKnown |
| Known Number of codon(s) coding for each amino-acid in universal genetic code [12] | NCFAAKnown | Side chain polarity [12, p51] | SCPolKnown |

(continued)

| Feature | Abbreviation | Feature | Abbreviation |
| --- | --- | --- | --- |
| Binary representation of protein sequence | BPS | 3-Class Secondary Structure | SS3 |
| Relative Solvent Accessibility | RSA | Permutation Vectors | PERM |

6.6 Pre-training

See section 2.2.

6.7 CV results

We defined as "age" of the protein Ag in our data the date at which the most recent CBCE was registered to the protein sequence. We used ~ 94% of the "oldest" proteins in the data on a 5-fold cross-validation in order to assess the performance of the model. We used custom callbacks in Tensorflow to allow for early stopping in case the loss did not decrease anymore.

6.7.1 Model model 13 conf v1

Figure 22 shows results for the precision-recall metric. The model performed well, without significant overfitting and had high performance for all evaluation metrics. Moreover, a small variation is observed between different CV runs for the loss. The models were trained for around 466 epochs and apparent bumps in the loss may indicate that the random generator create negative CBCE permutations which were similar to the positives. However, their negative effect became smaller as the epochs proceeded further.

Figure 22 illustrates results for CBCE model 13 conf v1. Figure 22 (a)-(e): PR curves. The x-axis is the TPR, the y-axis is the PPV. Figure 22(f): Weighted cross entropy loss. The x-axis is the epochs, the y-axis is the loss values. The plot is the average of 5-fold CVs including Gaussian Cl. The blue line is for training and the red (dashed) for validation sets.

6.7.2 Model model 17 conf v1

Figure 23 shows results for the precision-recall metric. The model seems to perform well, without significant overfit before 250 epochs and gives high performance for all metrics. Moreover, small variation is observed between different CV runs for the loss. The models were trained for 455 epochs.

Figure 23 illustrates results for CBCE model 17 conf v1. Figure 23 (a)-(e): PR curves. The x-axis is the TPR, the y-axis is the PPV. Figure 23(f): Weighted cross entropy loss. The x-axis is the epochs, the y-axis is the loss values. The plot is the average of 5-fold CVs including Gaussian Cl. The blue line is for training and the red (dashed) for validation sets.

6.8 Test results

We used ~ 6% of the "newest" proteins in the data in order to see the performance on completely unseen data. The reason for keeping the newest data as test set was because the same data was used for comparison with other algorithms. This way we ensure that those algorithms were not trained on that data. The training on the following models was done on the "oldest" ~ 94% of the data.

6.8.1 Model model 13 conf v1

Figure 24 shows the results for the precision-recall metric. The model performed well, without significant overfitting and gave high performance for all evaluation metrics. The model for trained for 466 epochs, chosen based on the CV analysis, so slightly more epochs than the CV analysis.

Figure 24 illustrates the results for CBCE model 13 conf v1. Figure 24(a): PR curves. The x-axis is the TPR, the y-axis is the PPV. Figure 24(b): Overall model loss. The x-axis is the epochs, the y-axis is the loss values. The solid line is for the training and the dotted for the validation set.

6.8.2 Model model 17 conf v1

Figure 25 shows the results for the precision-recall metric. The model seems to perform well, without significant overfit and gives high performance for all metrics. The model for trained for 455 epochs, chosen based on the CV analysis, so slightly more epochs than the CV analysis.

Figure 25 illustrates the results for CBCE model 17 conf v1. Figure 25(a): PR curve. The x-axis is the TPR, the y-axis is the PPV. Figure 25(b): Overall model loss. The x-axis is the epochs, the y-axis is the loss values. The solid line is for the training and the dotted for the validation set.

6.9 Final Training

The final training was done on the whole data. Using the optimal number of epochs determined by the cross-validation procedure.

6.9.1 Model model 13 conf v1

Figure 26 shows the results for the F1 metric. The model seems to perform well, without significant overfit and gives hight performance for all metrics. The model for trained for 500 epochs, chosen based on the CV analysis.

Figure 26 shows the results for CBCE model 13 conf v1. Figure 26(a)-(b): F1 metrics. The x-axis is the epochs, the y-axis is the F1 value. Figure 26(c): Overall model loss. The x-axis is the epochs, the y-axis is the loss values.

6.9.2 Model model 17 conf v1

Figure 27 shows the results for the F1 metric. The model seems to perform well, without significant overfit and gives high performance for all metrics. The model for trained for 700 epochs, chosen based on the CV analysis.

Figure 27 shows the results for CBCE model 17 conf v1. Figure 27(a): F1 metric. The x-axis is the epochs, the y-axis is the F1 value. Figure 27(b): Overall model loss. The x-axis is the epochs, the y-axis is the loss values.

7 First Machine Learning Model (LBCEs)

For predicting LBCEs we use BLSTMs models as well. This allows us to model the whole protein sequences as one observation, without the need of segmenting it. Therefore, distant amino-acid relationships should be able to be captured by the model. Moreover, the use of BLSTMs allows us to train different protein lengths simultaneously. Therefore, the model type here is the same as the one for the CBCEs in section 6. The main goal of our models was to create non-Ab specific LBCE predictors. This is achieved by the way we prepare the training data as described in section 7.1.

7.1 Data preparation

For modelling LBCEs we downloaded LBCE data from IEDB We downloaded all non-obsolete assays from the IEDB, from all organisms.

Since the main goal of our models was to create non-Ab specific LBCE predictors, we considered all positive LBCEs for our database. That is, if a LBCE was tested positive against at least one Ab, we included it in the database. Each Ag ID was then mapped to the universal protein resource (UniProt), in order to retrieve the protein sequence in fasta format. Assays were discarded if their IDs were non-mappable, or if their IDs were mappable but the coordinates of the corresponding LBCE did not give the same sequence on the retrieved Ag sequence.

Observed LBCEs were also mapped to similar Ag, as we did in section 6.1 for the CBCEs. Undiscovered LBCEs could increase the false negative rate of the prediction models. In an attempt to decrease the possibility of assigning undiscovered LBCEs as negative data we copied observed LBCEs to similar Ags. First we identified clusters of similar Ags using BlastPlus with > 90% similarity and at most 2 gaps. LBCEs were copied from an Ag in a cluster to all the other Ags in the same cluster, as long as their corresponding linear coordinates were exactly the same based on the mapping from BlastPlus.

Lastly, we created a unique Ags database. Duplicated Ags were completely removed the data. On the other hand, Ags sequences that were sub-sequences of longer Ags were kept in the data and treated as different observations. The resulted database constituted of 4695 Ag sequences in fasta format and 177782 CBCEs from which 63363 were unique. Each Ag sequence was associated with at least one LBCE.

7.2 LBCE model summary

Every sequence in the data is associated with at least one true LBCE. The model takes as input features, among others, a permutation vector. This vector is the LBCEs which are turned into binary 2D vectors and are given as input to the model. The output of the model is a probability, so the model is basically asking the question; "Is this permutation vector, on this specific sequence, a true LBCE or not?". Basically, the permutation vectors are the same as described in section 6.2, where the only difference is that they represent LBCEs and not CBCEs.

The goal of those models was to predict LBCEs before the binding event took place. The dataset used, described in section 7.1, contains the linear protein sequence and the true LBCEs. Therefore, there is no information about any structural or surface characteristic of those protein sequences. However, we need information about how each protein sequence's surface would look like before the binding event. Therefore, we used our prediction model for RSA (model struct 3d rsa hse m2 res), described in section 4, in order to predict those characteristics in every protein in the dataset.

There are three main differences of our models with the publicly available LBCE algorithms. Firstly, we use BLSTMs which allows us to capture relationships between distant amino-acids which may constitute a LBCE. On the other hand, the other algorithms are segmenting the protein sequences and thus, breaking such bonds. Secondly, the training dataset is created in such a way in order to predict true LBCEs before the binding event takes place. Which is exactly what an Ab sees and triggers the binding, and not what an Ab has already seen and bound on. Finally, we predict probabilities of stand-alone LBCEs, and therefore multiple LBCEs on the same protein sequence can be discovered and separated. On the other hand, other algorithms provide probabilities per amino-acid, without the ability of separation between different LBCEs.

The architecture of the model 12 linear v1 model has substantially the same structure as the model depicted in Figure 20(b). The bce output is a probability vector indicating if the input permutation is a true LBCE (second position on vector) or not (first position on vector). The bce output is computed from amino-acid values as described in section 6.2. Note: the input features were computed per amino-acid as they are, not averaged by windows.

## 7.3 Negative Data

We followed a similar procedure as in section 6.3.1. The only difference here is that we ensured that the randomly generated LBCEs were unbroken. Let N be the length of a given protein sequence. We first generate the total amount X of amino-acids that a random LBCE will have from the uniform distribution in [0,N]. Then we generated the starting position i of this LBCE from the uniform distribution in [0,N - X]. Finally, we assigned all amino-acids in [i, i + X] as the ones corresponding this random LBCE. A total amount of 10 randomly generated LBCEs was generated pre sequence, for every epoch.

## 7.4 Outliers

We removed outliers before any training of the model 12 linear v1 model. We followed exactly the same procedure as in section 6.4, and the data we used was the amount of total amino-acids of each observed LBCE in our data from section 7.1.

Figure 28 shows the total amount of amino-acids from all the observed LBCEs. Where we see that the median amount is at 17 amino-acids. After the outlier analysis, we found that valid LBCEs should contain a total amount of amino-acids in range [5, 30]. The outliers were finally removed from the data, and the models were trained without them.

Figure 28 shows outlier analysis for LBCEs. Figures 28(a) and (b) show, respectively, density and box-plot of observed amount of total amino-acids in the true LBCEs. Figures 28(c) and (d) show, log-scales of Figures 28(a) and 28(b) respectively.

## 7.5 Preliminary Analysis and Features Used

See section 3.2 for the procedure. The 13 features that we chose to use in the model (including the protein-sequence in binary representation and the permutation vectors) can be seen in Table 6. Note that the RSA was predicted by the model struct 3d rsa hse m2 res described in Section 4.

Table 6: Features used in the model 12 linear v1 model.

| Feature | Abbreviation | Feature | Abbreviation |
|---|---|---|---|
| Retention coefficient in HPLC, pH 7.4 [22] | RCHPLCPH74Me ek | Membrane buried helix parameter [5] | MBHPRao |
| Hydrophobic constants [26] | HPLCWilson | Normalized frequency for beta-sheet [16] | NFFBSLevitt |
| Side chain classes [12, p51] | SCClassKnown | Antigenicity [27] | AGCKolaskar |
| Normalized frequency for beta-turn [16] | NFFBTLevitt | Side chain polarity [12, p51] | SCPolKnown |

(continued)

| Feature | Abbreviation | Feature | Abbreviation |
|---|---|---|---|
| Binary representation of protein sequence | BPS | 3-Class Secondary Structure | SS3 |
| Relative Solvent Accessibility | RSA | Permutation Vectors | PERM |
| PK1 a-COOH [25] | PK1Known | | |

## 8 Applications

The precise prediction of B-cell epitopes (in particular 3D conformational B-cell epitopes) leads to major improvements in diagnostics, drug design and vaccine development in the fields of autoimmunity, infectious-disease, and cancer. Examples are provided below.

### 8.1 Therapeutic antibody mapping

The binding event of an antibody to an antigen is a key event in activating the humoral B Cell immune response against any pathogenic threat, such as viral and bacterial infections, in addition to cancer antigens in malignant tumour cells. B Cell epitope mapping is essential in therapeutic antibody development. The prediction of true B-cell epitopes by the present invention not only provides a functional understanding of the critical residues involved antibody-antigen binding; it also aids in the selection of therapeutically relevant antibodies to the prioritized predicted epitopes for further development as therapeutic antibodies.

### 8.2 Vaccine development

In antibody driven vaccine design, *in silico* predictions are performed to predict BCEs that could trigger humoral immune responses. Previous *in silico* guided vaccine-design initiatives tend to selectively apply BCE prediction with a focus on surface-exposed sites among the antigens. However, by using the approach of the present invention (in particular training on unbound protein structures and the use of candidate BCE encodings ("permutation vectors") mapped to the antigen protein sequence), we can identify with precision, BCEs to guide Ab based vaccine design with far higher fidelity. BCE vaccines guided by the approach of the present invention can advantageously identify at scale specific antibody epitope interactions capable of avoiding undesirable immune responses, in addition to potentially generating long lasting, potent and universal immunity. Moreover, the method described here bypasses the need for years of target discovery and laborious expensive time-consuming work at a fraction of the cost and time.

### 8.3 immune-diagnostics and immune monitoring

Prediction of B cell epitopes is of direct help to the design of immuno-diagnostic and immune monitoring of therapy strategies and reagents. Integrating prediction approaches for the accurate and comprehensive detection of BCEs can greatly facilitate diagnosis. One example of this would be the rapid and accurate detection of BCE epitopes on the spike protein of beta-coronaviruses, allowing for the diagnosis of COVID in a manner that can distinguish infections caused by common cold viruses from the SARS-CoV-2 virus causing COVID. Along the same vein, the BCE prediction technology can be used to identify immunodominant or humoral reactive epitopes in tumour associated antigens or neoantigens in immuno-diagnostic and immune monitoring clinical applications.

### 8.4 Example Systems

Figure 29 schematically illustrates an example of a system suitable for implementing embodiments of the method. The system 1100 comprises at least one server 1110 which is in communication with a reference data store 1120. The server may also be in communication with an automated peptide synthesis device 1130, for example over a communications network 1140.

In certain embodiments the server may obtain, for example using from the reference data store, an amino acid sequence of one or more query proteins. The server may then predict whether the query protein comprises a BCE that is likely to instigate a binding event with an antibody (e.g. instigate an immunogenic response).

Query proteins predicted to contain one or more BCEs may be sent to the automated peptide synthesis device 1130 to synthesise the query protein or parts thereof. Techniques for automated peptide synthesis are well known in the art and it will be understood that any known technique may be used. Typically, the query protein or epitope is synthesized using standard solid phase synthetic peptide chemistry and purified using reverse-phase high performance liquid chromatography before being formulated into an aqueous solution. If used for vaccination, prior to administration the peptide solution is usually admixed with an adjuvant before being administered to the patient.

Peptide synthesis technology has existed for more than 20 years but has undergone rapid improvements in recent years to the point where synthesis now takes just a few minutes on commercial machines. For brevity we do not describe in detail such machines but their operation would be understood to one skilled in the art and such conventional machines may be adapted to receive a candidate region or epitope from the server.

The server may comprise the functions described above to identify query proteins predicted to contain one or more viable BCEs. It will of course be understood that these functions may be subdivided across different processing entities of a computer network and different processing modules in communication with one another.

The techniques for identifying BCE-containing proteins may integrate into a wider ecosystem for customised vaccine development. Example vaccine development ecosystems are well known in the art and are described at a high-level for context, but for brevity we do not describe the ecosystem in detail.

In an example ecosystem, a first, sample, step may be to isolate DNA from a tumor biopsy and matched healthy tissue control. In a second, sequence, step, the data is sequenced and the variants identified i.e. the mutations. In an immune profiler step the associated mutated peptides may be generated «in silico».

Using the associated mutated peptides, and the techniques described here, a candidate protein may be predicted and selected and target epitopes identified for vaccine design. That is, the candidate peptide sequence is chosen based on its predicted binding affinity determined using the technique described herein.

The target epitopes are then generated synthetically using conventional techniques as described above. Prior to administration the peptide solution is usually admixed with an adjuvant before being administered to the patient (vaccination). In alternatives, the target epitopes can be engineered into DNA or RNA, or engineered into the genome of a bacteria or virus, as with any conventional vaccine.

The proteins predicted by the methods described herein may also be used to create other types of vaccine other than peptide based vaccines. For example the proteins (or predicted epitopes therein) could be encoded into the corresponding DNA or RNA sequence and used to vaccinate the patient. Note that the DNA is usually inserted in to a plasmid construct. Alternatively, the DNA can be incorporated into the genome of a bacterial or viral delivery system (can be RNA also - depending on the viral delivery system) - which can be used to vaccinate the patient - so the manufactured vaccine in a genetically engineered virus or bacteria which manufactures the targets post immunisation in the patient i.e. in vivo.

An example of a suitable server 1110 is shown in Figure 30. In this example, the server includes at least one microprocessor 1200, a memory 1201, an optional input/output device 1202, such as a keyboard and/or display, and an external interface 1203, interconnected via a bus 1204 as shown. In this example the external interface 1203 can be utilised for connecting the server 1110 to peripheral devices, such as the communications networks 1140, reference data store 1120, other storage devices, or the like. Although a single external interface 1203 is shown, this is for the purpose of example only, and in practice multiple interfaces using various methods (e.g. Ethernet, serial, USB, wireless or the like) may be provided.

In use, the microprocessor 1200 executes instructions in the form of applications software stored in the memory 1201 to allow the required processes to be performed, including communicating with the reference data store 1120 in order to receive and process input data, and/or with a client device to receive sequence data for one or more query proteins, and to generate immunogenic potential predictions according to the methods described above. The applications software may include one or more software modules, and may be executed in a suitable execution environment, such as an operating system environment, or the like.

Accordingly, it will be appreciated that the server 1200 may be formed from any suitable processing system, such as a suitably programmed client device, PC, web server, network server, or the like. In one particular example, the server 1200 is a standard processing system such as an Intel Architecture based processing system, which executes software applications stored on non- volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement. Accordingly, whilst the term server is used, this is for the purpose of example only and is not intended to be limiting.

Whilst the server 1200 is a shown as a single entity, it will be appreciated that the server 1200 can be distributed over a number of geographically separate locations, for example by using processing systems and/or databases 1201 that are provided as part of a cloud based environment. Thus, the above described arrangement is not essential and other suitable configurations could be used.

9. Appendix

[1] T P Hopp and K R Woods. Prediction of protein antigenic determinants from amino acid sequences. Proceedings of the National Academy of Sciences of the United States of America, 78(6):3824-3828, 06 1981.

[2] R. Grantham. Amino acid difference formula to help explain protein evolution. Science, 185(4154):862, 09 1974.

[3] JOEL JANIN. Surface and inside volumes in globular proteins. Nature, 277:491 EP -, 02 1979.

[4] H R Guy. Amino acid side-chain partition energies and distribution of residues in soluble proteins. Biophys J, 47(1):61-70, Jan 1985.

[5] J. K. Mohana Rao and Patrick Argos. A conformational preference parameter to predict helices in integral membrane proteins. Biochimica et Biophysica Acta (BBA) - Protein Structure and Molecular Enzymology, 869(2):197-214, 1986.

[6] Sanzo Miyazawa and Robert L. Jernigan. Estimation of effective interresidue contact energies from protein crystal structures: quasi-chemical approximation. Macromolecules, 18(3):534-552, 03 1985.

[7] Gang Zhao and Erwin London. An amino acid "transmembrane tendency" scale that approaches the theoretical limit to accuracy for prediction of transmembrane helices: relationship to biological hydrophobicity. Protein science : a publication of the Protein Society, 15(8):1987-2001, 08 2006.

[8] Cyrus Chothia. The nature of the accessible and buried surfaces in proteins. Journal of Molecular Biology, 105(1):1-12, 1976.

[9] GD Rose, AR Geselowitz, GJ Lesser, RH Lee, and MH Zehfus. Hydrophobicity of amino acid residues in globular proteins. Science, 229(4716):834, 08 1985.

[10] P Y Chou and G D Fasman. Prediction of the secondary structure of proteins from their amino acid sequence. Adv Enzymol Relat Areas Mol Biol, 47:45-148, 1978.

[11] Shneior Lifson and Christian Sander. Antiparallel and parallel -strands differ in amino acid residue preferences. Nature, 282(5734):109-111, 1979.

[12] M. Cooper and Robert E. Hausman. The cell : a molecular approach, volume 4. Washington, DC ASM Press, 2007.

[13] EA Emini, J V Hughes, D S Perlow, and J Boger. Induction of hepatitis a virus-neutralizing antibody by a virus-specific synthetic peptide. Journal of virology, 55(3):836-839, 09 1985.

[14] G. Deleage and B. Roux. An algorithm for protein secondary structure prediction based on class prediction. Protein Engineering, Design and Selection, 1 (4):289-294, 08 1987.

[15] P. MANAVALAN and P. K. PONNUSWAMY. Hydrophobic character of amino acid residues in globular proteins. Nature, 275(5681):673-674, 1978.

[16] Michael Levitt. Conformational preferences of amino acids in globular proteins. Biochemistry, 17(20):4277-4285, 10 1978.

[17] Serafin Fraga. Theoretical prediction of protein antigenic determinants from amino acid sequences. Canadian Journal of Chemistry, 60(20):2606-2610, 2019/03/01 1982.

[18] Gjalt W. Welling, Wicher J. Weijer, Ruurd van der Zee, and Sytske Welling-Wester. Prediction of sequential antigenic regions in proteins. FEBS Letters, 188(2):215-218, 2019/02/28 1985.

[19] Henry B. Bull and Keith Breese. Surface tension of amino acid solutions: A hydrophobicity scale of the amino acid residues. Archives of Biochemistry and Biophysics, 161(2):665-670, 1974.

[20] C. A. Browne, H. P. J. Bennett, and S. Solomon. The isolation of peptides by high-performance liquid chromatography using predicted elution positions. Analytical Biochemistry, 124(1):201-208, 1982.

[21] R. Wolfenden, L. Andersson, P. M. Cullis, and C. C. B. Southgate. Affinities of amino acid side chains for solvent water. Biochemistry, 20(4):849-855, 02 1981.

[22] J L Meek. Prediction of peptide retention times in high-pressure liquid chromatography on the basis of amino acid composition. Proceedings of the National Academy of Sciences of the United States of America, 77(3):1632-1636, 03 1980.

[23] R. BHASKARAN and P. K. PONNUSWAMY. Positional flexibilities of amino acid residues in globular proteins. International Journal of Peptide and Protein Research, 32(4):241-255, 2019/02/28 1988.

[24] Schwartz R.M. Dayhoff, M.O. and B.C. Orcutt. A model of evolutionary change in proteins, volume 5. Atlas of Protein Sequence and Structure. Natl. Biomed. Res. Found., Washington DC, 1978.

[25] David L. (David Lee) Nelson and Michael M. Cox. Lehninger principles of biochemistry. Principles of biochemistry. W.H.Freeman, New York, seventh edition, edition, 2017.

[26] K JWilson, A Honegger, R P Stotzel, and G J Hughes. The behaviour of peptides on reverse-phase supports during high-pressure liquid chromatography. The Biochemical journal, 199(1):31-41, 10 1981.

[27] A. S. Kolaskar and Prasad C. Tongaonkar. A semi-empirical method for prediction of antigenic determinants on protein antigens. FEBS Letters, 276(1-2):172-174, 2018/12/07 December 10, 1990.
[28] Peter McCaldon and Patrick Argos. Oligopeptide biases in protein sequences and their use in predicting protein coding regions in nucleotide sequences. Proteins: Structure, Function, and Bioinformatics, 4(2):99-122, 2019/02/28 1988.
[29] J.M. Zimmerman, Naomi Eliezer, and R. Simha. The characterization of amino acid sequences in proteins by statistical methods. Journal of Theoretical Biology, 21(2):170-201, 1968.
[30] Daniel D. Jones. Amino acid properties and side-chain orientation in proteins: A cross correlation approach, volume 50. 04 1975.
[31] D. Eisenberg, E. Schwarz, M. Komaromy, and R.Wall. Analysis of membrane and surface protein sequences with the hydrophobic moment plot. Journal of Molecular Biology, 179(1):125-142, 1984.
[32] J. M. R. Parker, D. Guo, and R. S. Hodges. New hydrophilicity scale derived from high-performance liquid chromatography peptide retention data: correlation of predicted surface residues with antigenicity and x-ray-derived accessible sites. Biochemistry, 25(19):5425-5432, 09 1986.
[33] Shaun D. Black and Diane R. Mould. Development of hydrophobicity parameters to analyze proteins which bear post- or cotranslational modifications. Analytical Biochemistry, 193(1):72-82, 1991.
[34] J L. Fauchere and V Pliska. Hydrophobic parameters II of amino acid side-chains from the partitioning of N-acetyl-amino acid amides, volume 18. 01 1983.
[35] Robert M. Sweet and David Eisenberg. Correlation of sequence hydrophobicities measures similarity in three-dimensional protein structure. Journal of Molecular Biology, 171(4):479-488, 1983.
[36] Jack Kyte and Russell F. Doolittle. A simple method for displaying the hydropathic character of a protein. Journal of Molecular Biology, 157(1):105-132, 1982.
[37] Charles. Tanford. Contribution of hydrophobic interactions to the stability of the globular conformation of proteins. Journal of the American Chemical Society, 84(22):4240-4247, 11 1962.
[38] Donald J. Abraham and Albert J. Leo. Extension of the fragment method to calculate amino acid zwitterion and side chain partition coefficients. Proteins: Structure, Function, and Bioinformatics, 2(2):130-152, 2019/02/28 1987.
[39] Richard Cowan and R GWhittaker. Hydrophobicity indices for amino acid residues as determined by HPLC, volume 3. 01 1990.
[40] Mark A. Roseman. Hydrophilicity of polar amino acid side-chains is markedly reduced by flanking peptide bonds. Journal of Molecular Biology, 200(3):513-522, 1988.
[41] A. A. Aboderin. An empirical hydrophobicity scale for alpha-amino-acids and some of its applications. International Journal of Biochemistry, 2:537-544, 1971.

## Claims

1. A method of training a machine learning model, comprising

    generating first pseudo-random data;
    performing a first pass of machine learning training, using positive training data and using the first pseudo-random data as negative training data, to complete a first epoch;
    generating second pseudo-random data; and
    performing a second pass of machine learning training, using the second pseudo-random data as negative training data and using the positive training data, to complete a second epoch.

2. The method of claim 1, wherein the positive training data contains examples verified as having a first characteristic, and wherein training the machine learning model is performed using only the positive training data and pseudo-random data assumed not to have the first characteristic as negative training data.

3. The method of claim 1 or claim 2, wherein the machine learning model is configured to predict whether a protein comprises a B-cell epitope that is likely to instigate a binding event with an antibody, based on an input comprising an amino acid sequence of the protein.

4. The method of any of the preceding claims, wherein a set of data elements in the first pseudo-random data or the second pseudo-random data has a second characteristic that is shared with one or more sets of data elements in the positive training data.

5. The method of any of the preceding claims, wherein generating the first pseudo-random data and/or generating the second pseudo-random data comprises pseudo-randomly modifying one or more sets of data elements in the positive training data.

**6.** The method of claim 5, wherein a set of data elements in the first pseudo-random data and/or the second pseudo-random data includes a subset of values that are present in a set of the positive training data.

**7.** The method of claim 5 or claim 6, wherein generating the first pseudo-random data and/or the second pseudo-random data comprises fixing certain values in a set of data elements in the positive training data and pseudo-randomly altering remaining values to generate one or more sets of data elements.

**8.** The method of any of the preceding claims, comprising generating new pseudo-random data for each epoch during training and using the newly generated pseudo-random data as negative data in each respective epoch, preferably comprising replacing pseudo-random data used in each previous epoch with the newly generated pseudo-random data such that different negative data is used in each epoch.

**9.** The method of any of the preceding claims, wherein the first machine leaning model is a neural network, preferably a long short-term memory network, LSTM or a bi-directional long short-term memory network, BLSTM.

**10.** The method of any of the preceding claims, wherein, in one or more epochs, the negative training data contains more examples than the positive training data, more preferably wherein a ratio of the number of sets in the negative training data to the number of sets in the positive training data is greater than or equal to 2:1, more preferably where the ratio is about 3:1.

**11.** A non-transitory computer readable medium comprising executable instructions that, when executed by a computer, cause the computer to perform steps to train a machine learning model, the steps comprising:

generating first pseudo-random data;
performing a first pass of machine learning training, using positive training data and using the first pseudo-random data as negative training data, to complete a first epoch;
generating second pseudo-random data; and
performing a second pass of machine learning training, using the second pseudo-random data as negative training data and using the positive training data, to complete a second epoch.

**12.** A system comprising one or more processors configured to train a machine learning model by performing steps comprising:

generating first pseudo-random data;
performing a first pass of machine learning training, using positive training data and using the first pseudo-random data as negative training data, to complete a first epoch;
generating second pseudo-random data; and
performing a second pass of machine learning training, using the second pseudo-random data as negative training data and using the positive training data, to complete a second epoch.

**13.** A non-transitory computer readable medium comprising a machine learning model trained according to the method of any of claims 1 to 10.

**14.** Use of a machine learning model trained using the method of any of claims 1 to 10.

**15.** A machine learning model trained using the method of any of claims 1 to 10.

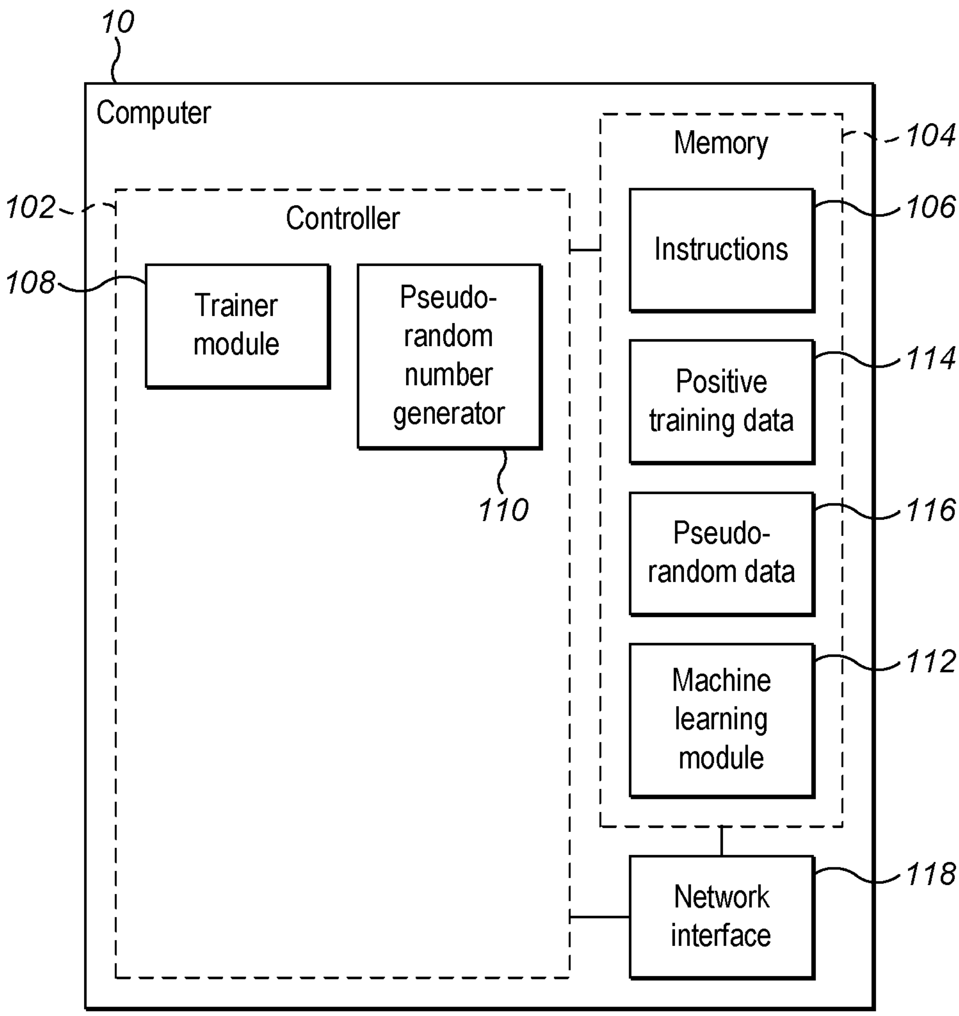
10
Computer
Memory
104
102
Controller
106
Instructions
108
Trainer
module
Pseudo-
random
number
generator
114
Positive
training data
110
116
Pseudo-
random data
112
Machine
learning
module
118
Network
interface

FIG. 1

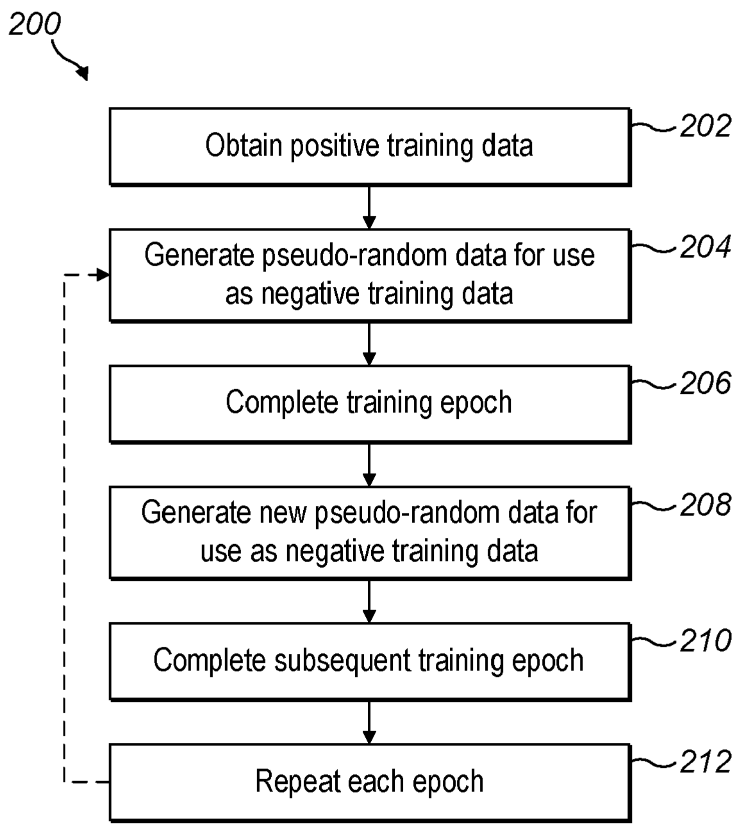
200
Obtain positive training data
202
Generate pseudo-random data for use as negative training data
204
Complete training epoch
206
Generate new pseudo-random data for use as negative training data
208
Complete subsequent training epoch
210
Repeat each epoch
212

FIG. 2

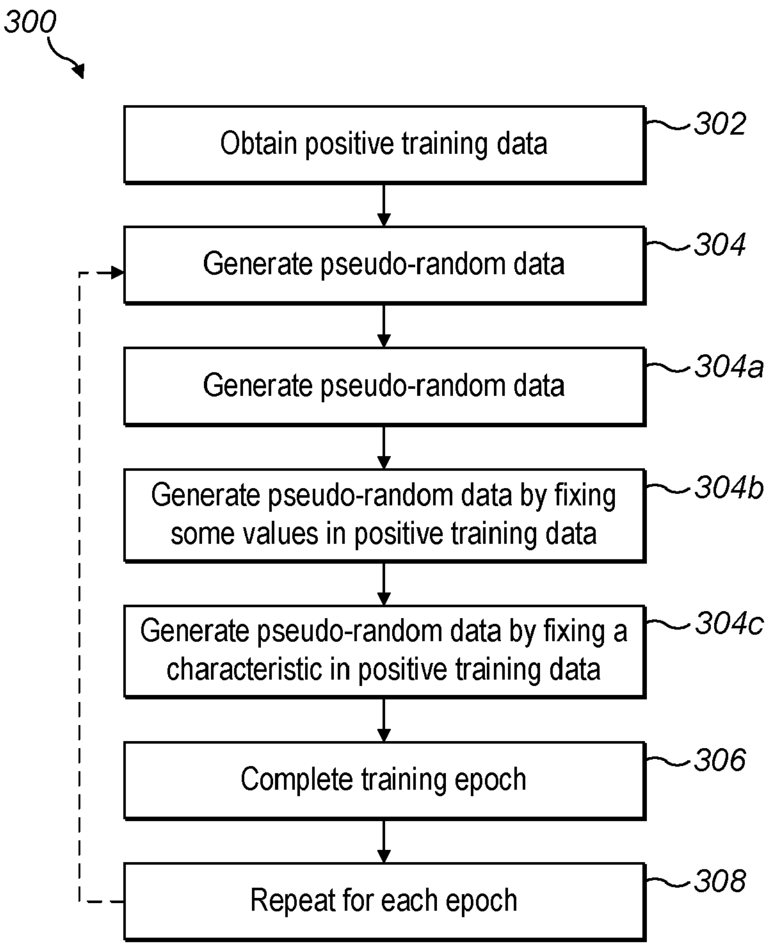
300
Obtain positive training data
302
Generate pseudo-random data
304
Generate pseudo-random data
304a
Generate pseudo-random data by fixing some values in positive training data
304b
Generate pseudo-random data by fixing a characteristic in positive training data
304c
Complete training epoch
306
Repeat for each epoch
308

FIG. 3

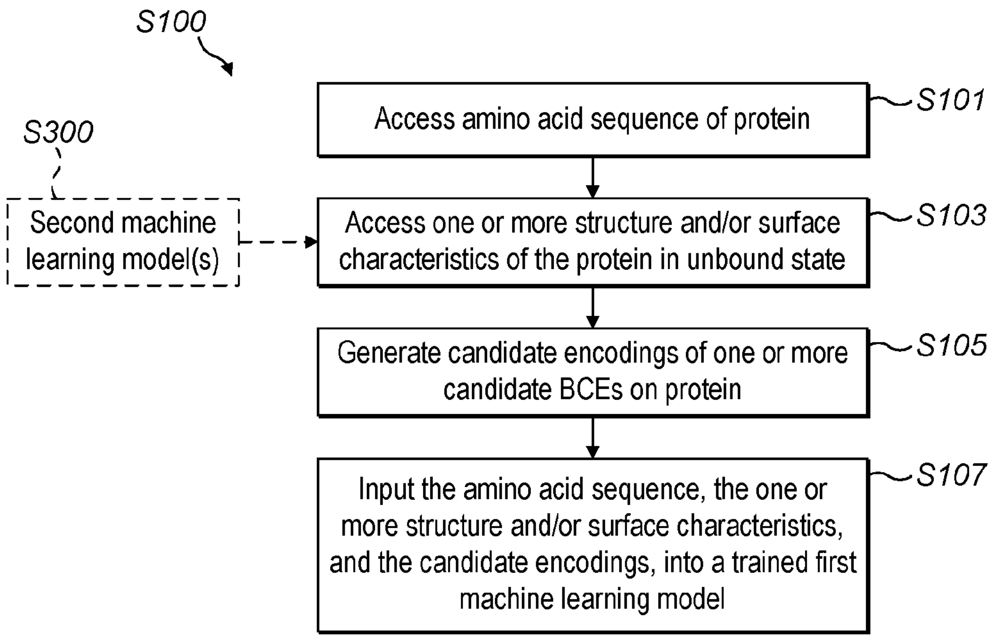
S100
S300
Second machine learning model(s)
Access amino acid sequence of protein
S101
Access one or more structure and/or surface characteristics of the protein in unbound state
S103
Generate candidate encodings of one or more candidate BCEs on protein
S105
Input the amino acid sequence, the one or more structure and/or surface characteristics, and the candidate encodings, into a trained first machine learning model
S107

FIG. 4

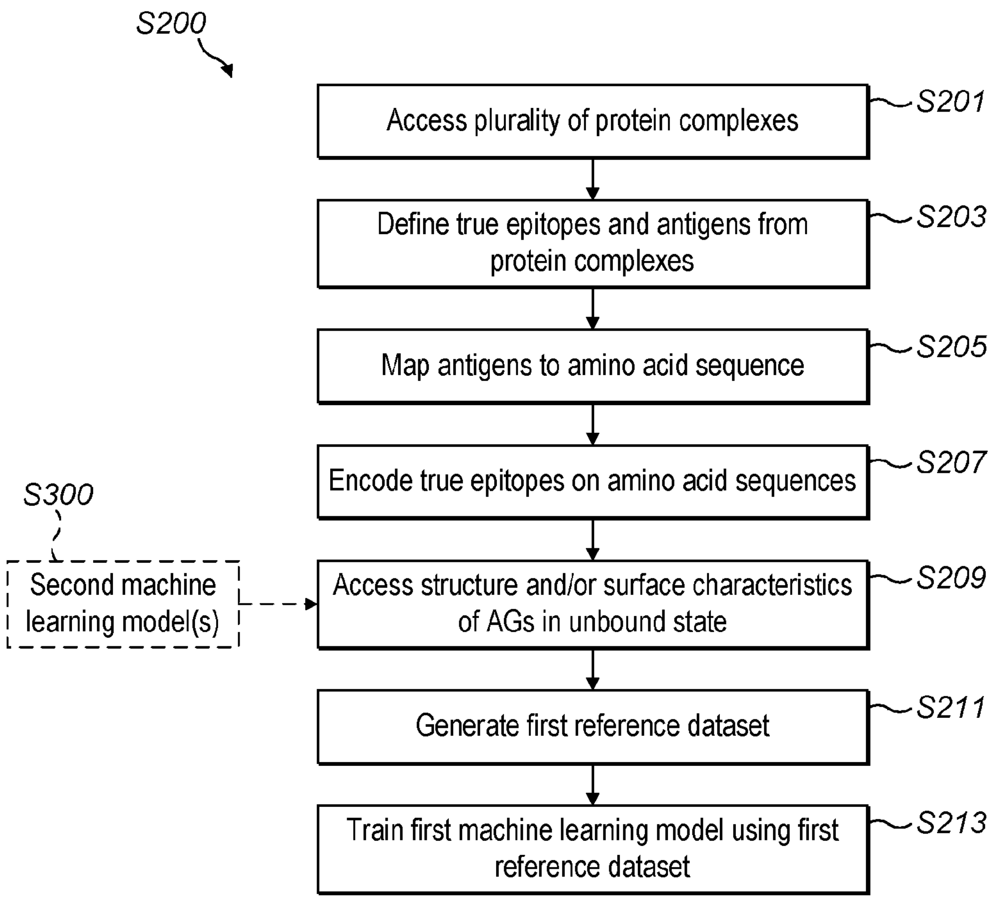
S200
Access plurality of protein complexes
S201
Define true epitopes and antigens from protein complexes
S203
Map antigens to amino acid sequence
S205
Encode true epitopes on amino acid sequences
S207
S300
Second machine learning model(s)
Access structure and/or surface characteristics of AGs in unbound state
S209
Generate first reference dataset
S211
Train first machine learning model using first reference dataset
S213

FIG. 5

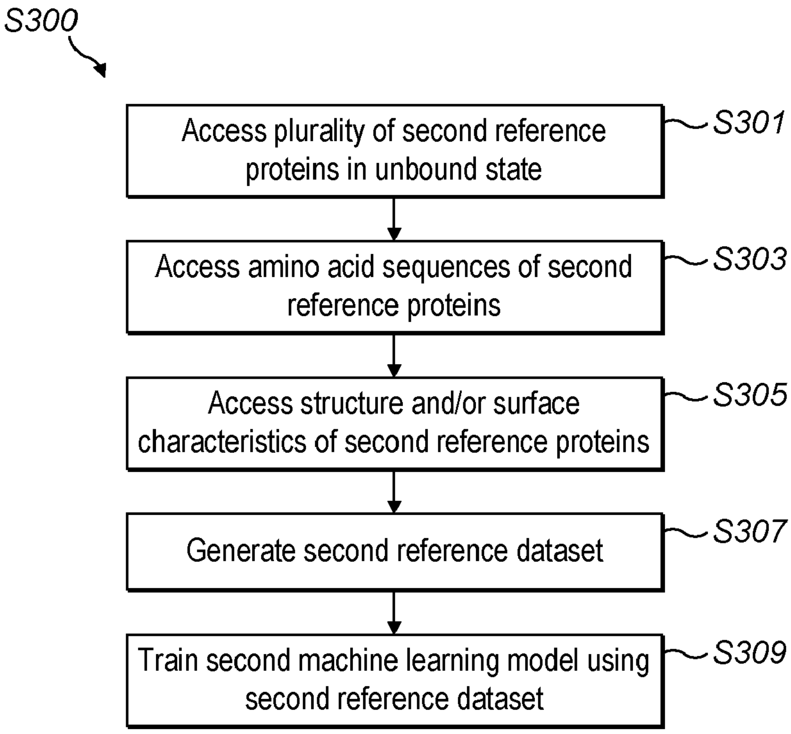
S300
Access plurality of second reference proteins in unbound state
S301
Access amino acid sequences of second reference proteins
S303
Access structure and/or surface characteristics of second reference proteins
S305
Generate second reference dataset
S307
Train second machine learning model using second reference dataset
S309

FIG. 6

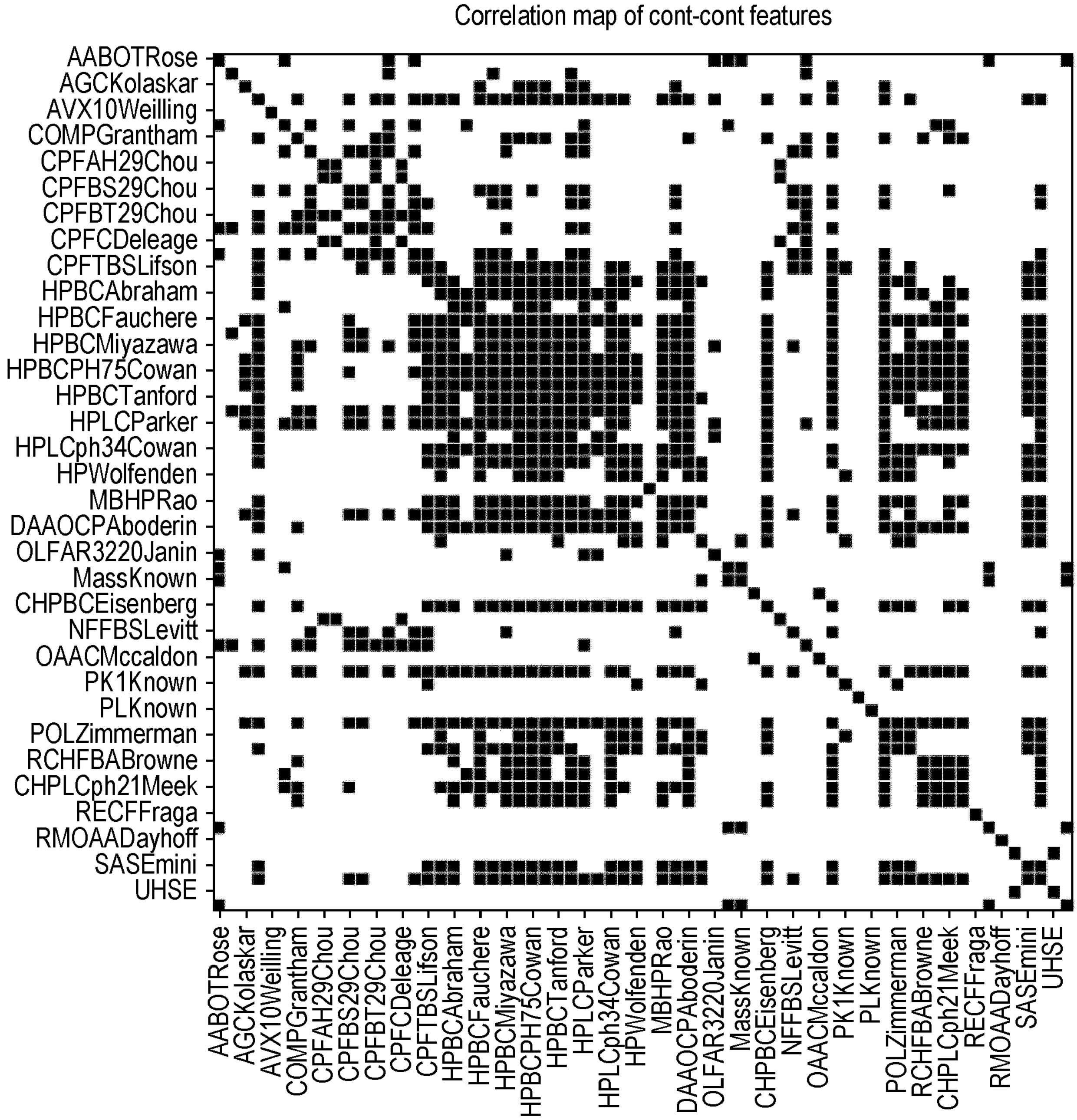


Continuous-continuous feature pairs median Spearman's correlation

*FIG. 7(a)*

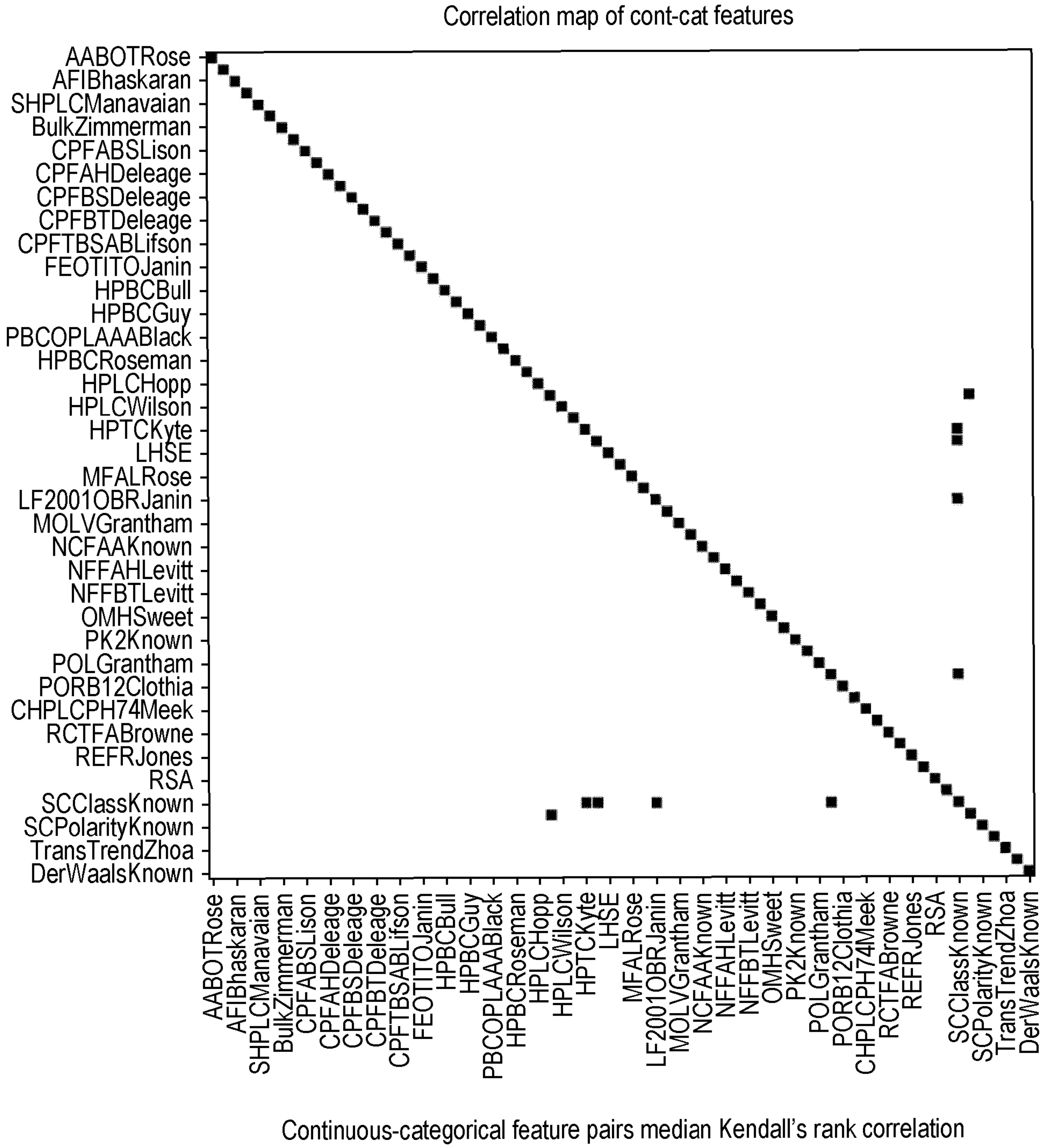
Correlation map of cont-cat features
AABOTRose
AFIBhaskaran
SHPLCManavaian
BulkZimmerman
CPFABSLison
CPFAHDeleage
CPFBSDeleage
CPFBTDeleage
CPFTBSABLifson
FEOTITOJanin
HPBCBull
HPBCGuy
PBCOPLAAABlack
HPBCRoseman
HPLCHopp
HPLCWilson
HPTCKyte
LHSE
MFALRose
LF2001OBRJanin
MOLVGrantham
NCFAAKnown
NFFAHLevitt
NFFBTLevitt
OMHSweet
PK2Known
POLGrantham
PORB12Clothia
CHPLCPH74Meek
RCTFABrowne
REFRJones
RSA
SCClassKnown
SCPolarityKnown
TransTrendZhoa
DerWaalsKnown
Continuous-categorical feature pairs median Kendall's rank correlation

*FIG. 7(b)*

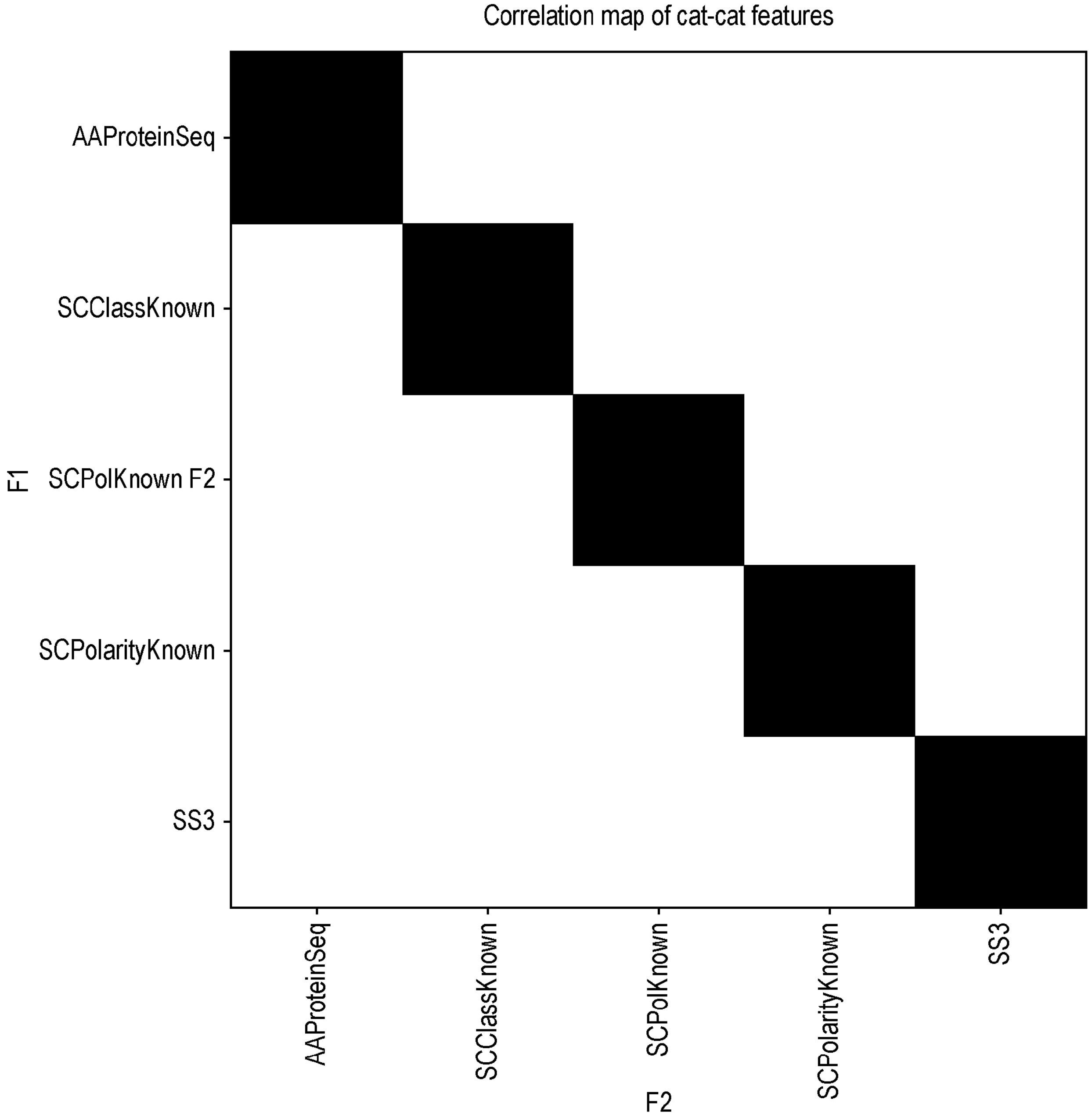


Categorical-categorical feature pairs median mutual information

*FIG. 7(c)*

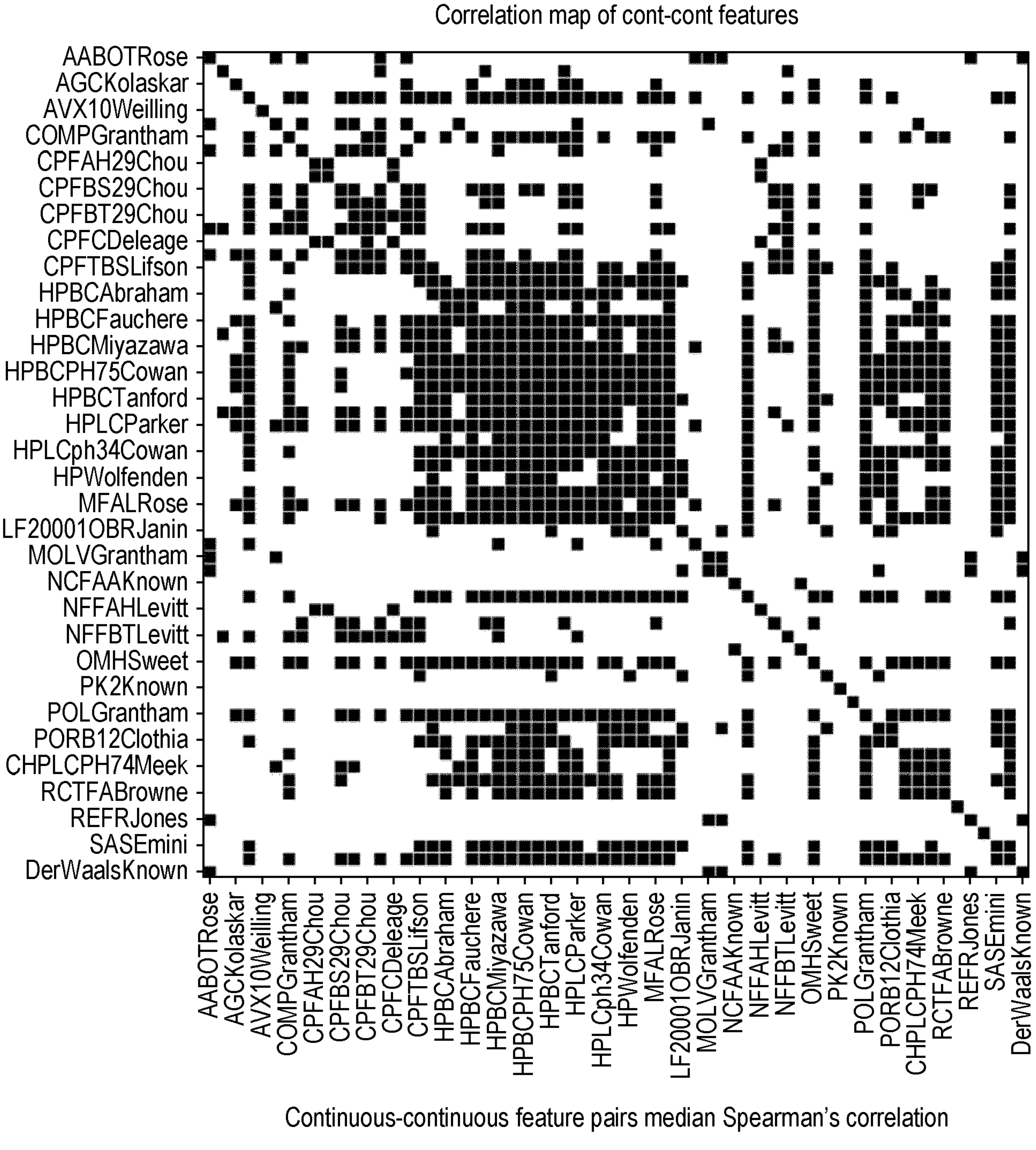
Correlation map of cont-cont features
AABOTRose
AGCKolaskar
AVX10Weilling
COMPGrantham
CPFAH29Chou
CPFBS29Chou
CPFBT29Chou
CPFCDeleage
CPFTBSLifson
HPBCAbraham
HPBCFauchere
HPBCMiyazawa
HPBCPH75Cowan
HPBCTanford
HPLCParker
HPLCph34Cowan
HPWolfenden
MFALRose
LF20001OBRJanin
MOLVGrantham
NCFAAKnown
NFFAHLevitt
NFFBTLevitt
OMHSweet
PK2Known
POLGrantham
PORB12Clothia
CHPLCPH74Meek
RCTFABrowne
REFRJones
SASEmini
DerWaalsKnown
Continuous-continuous feature pairs median Spearman's correlation

FIG. 7(d)

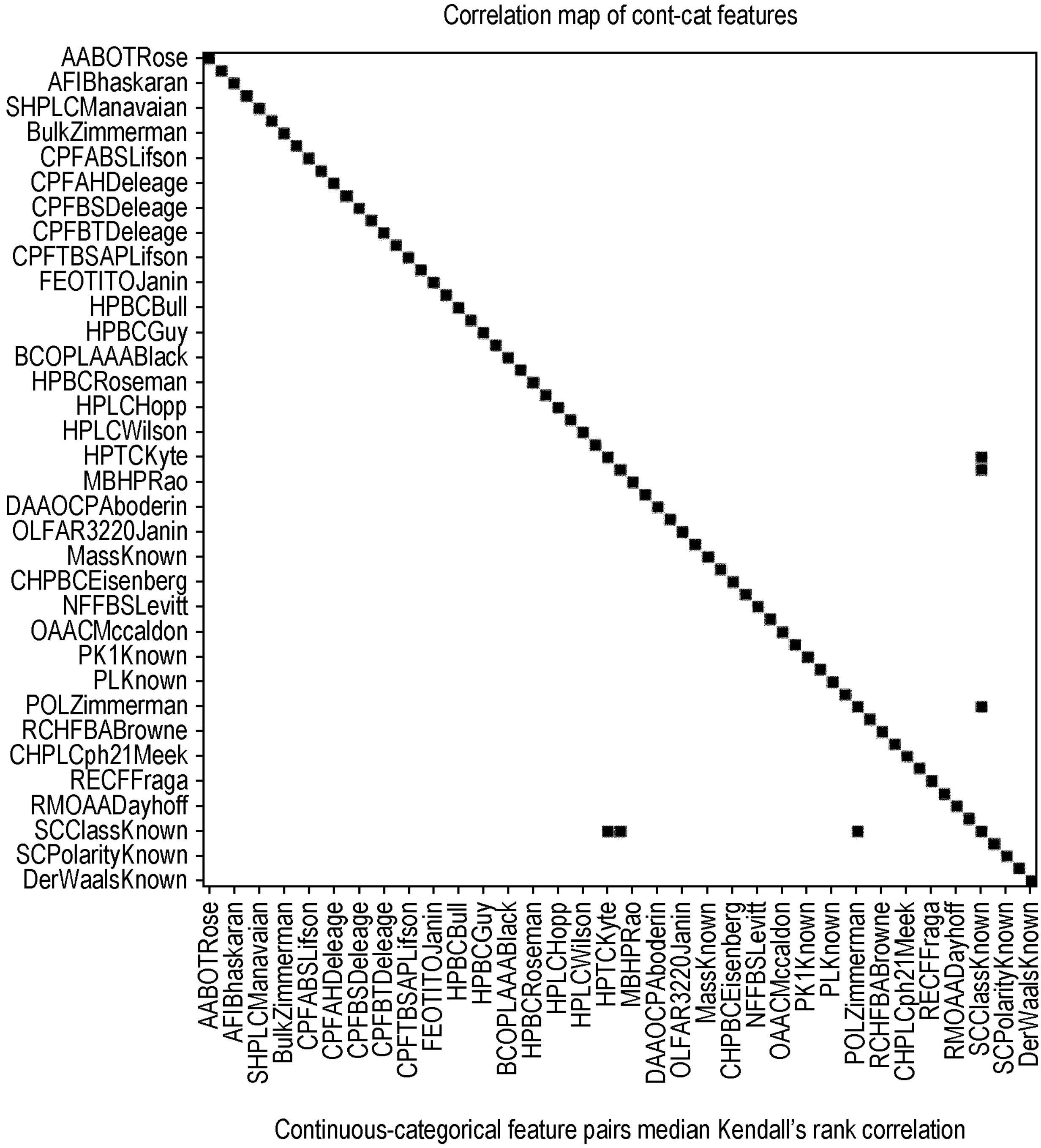
Correlation map of cont-cat features
AABOTRose
AFIBhaskaran
SHPLCManavaian
BulkZimmerman
CPFABSLifson
CPFAHDeleage
CPFBSDeleage
CPFBTDeleage
CPFTBSAPLifson
FEOTITOJanin
HPBCBull
HPBCGuy
BCOPLAAABlack
HPBCRoseman
HPLCHopp
HPLCWilson
HPTCKyte
MBHPRao
DAAOCPAboderin
OLFAR3220Janin
MassKnown
CHPBCEisenberg
NFFBSLevitt
OAACMccaldon
PK1Known
PLKnown
POLZimmerman
RCHFBABrowne
CHPLCph21Meek
RECFFraga
RMOAADayhoff
SCClassKnown
SCPolarityKnown
DerWaalsKnown
Continuous-categorical feature pairs median Kendall's rank correlation

FIG. 7(e)

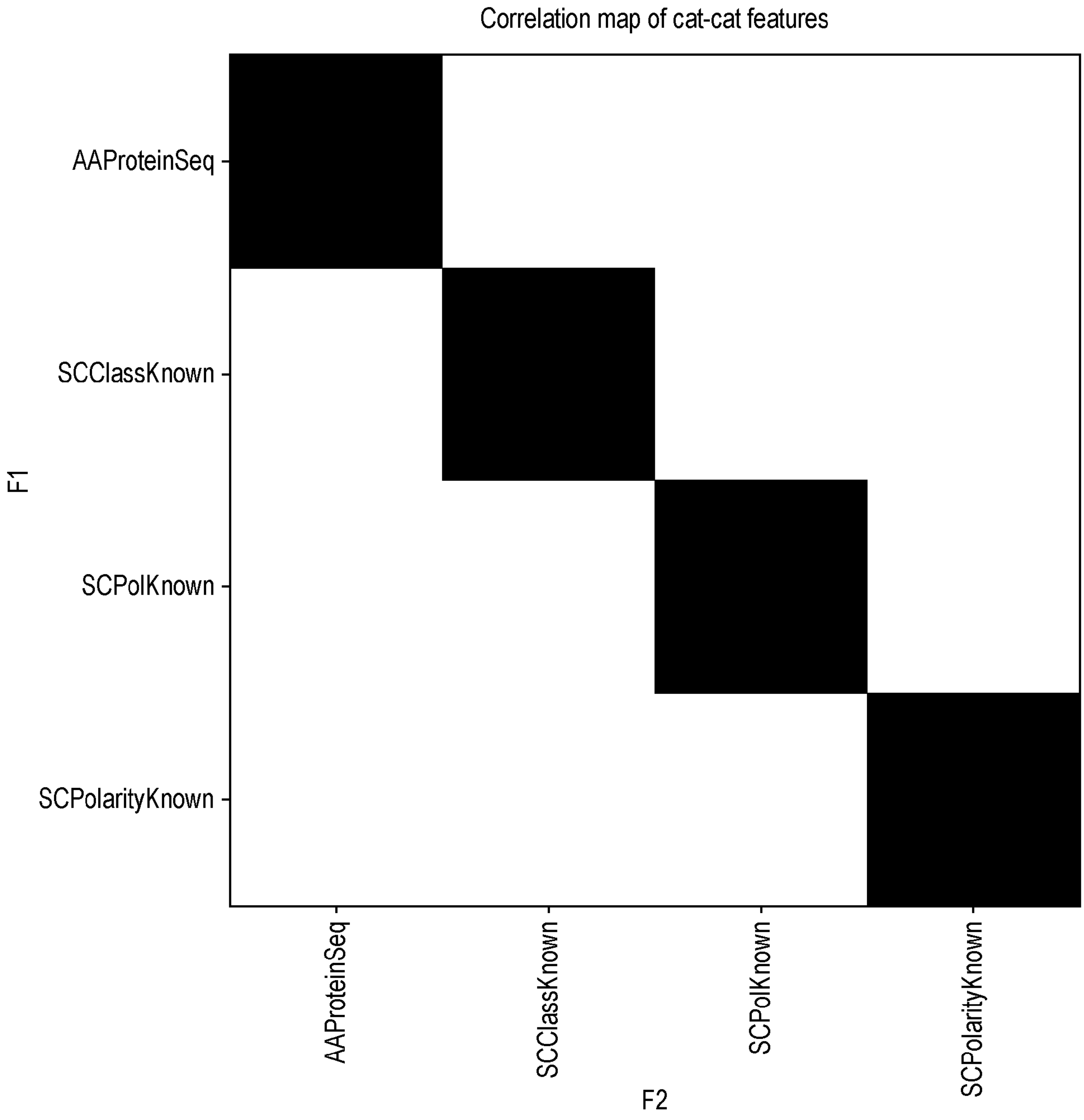


Categorical-categorical feature pairs median mutual information

*FIG. 7(f)*

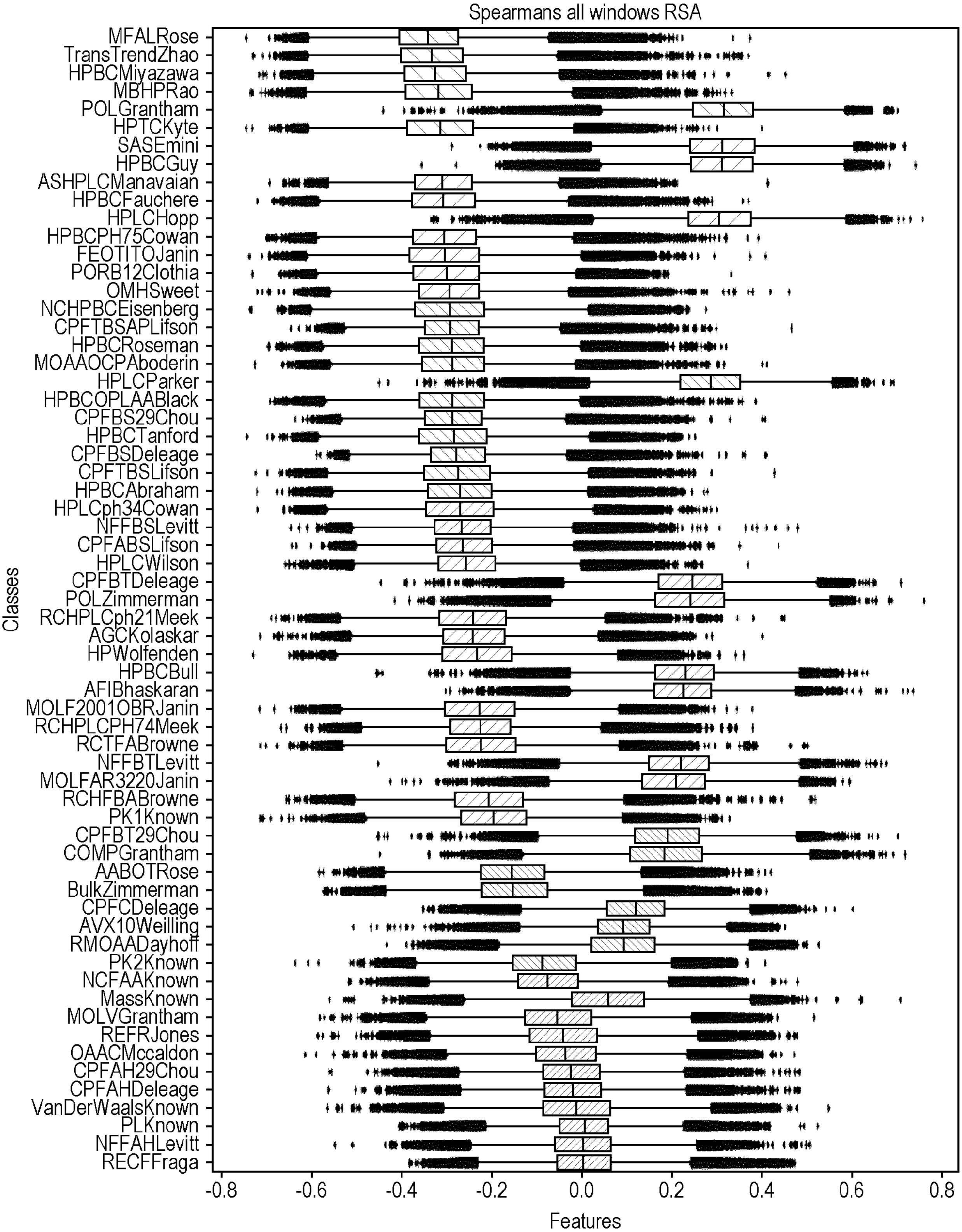


Continuous features with RSA using median Spearman's correlation

*FIG. 8(a)*

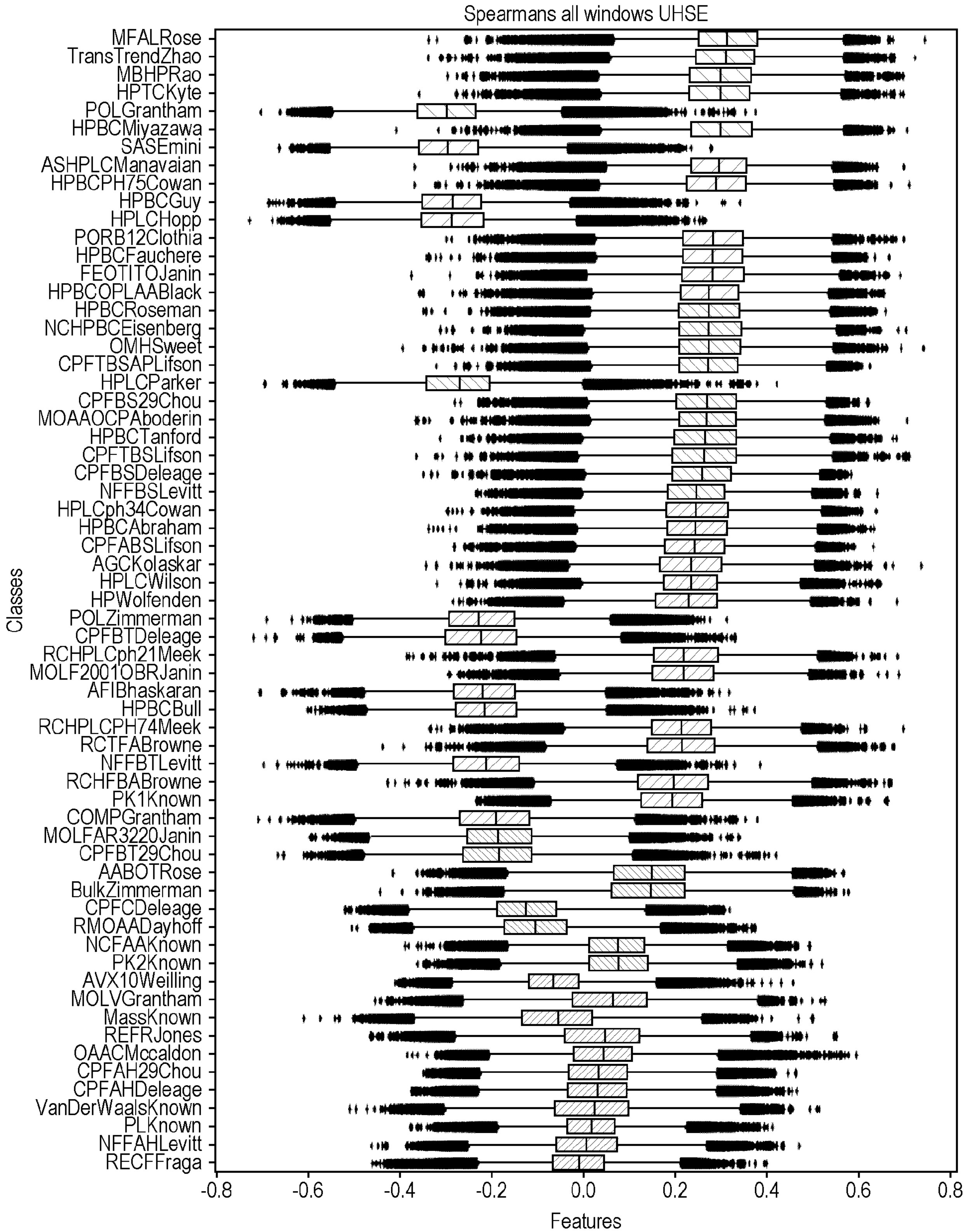


Continuous features with UHSE using median Spearman's correlation

*FIG. 8(b)*

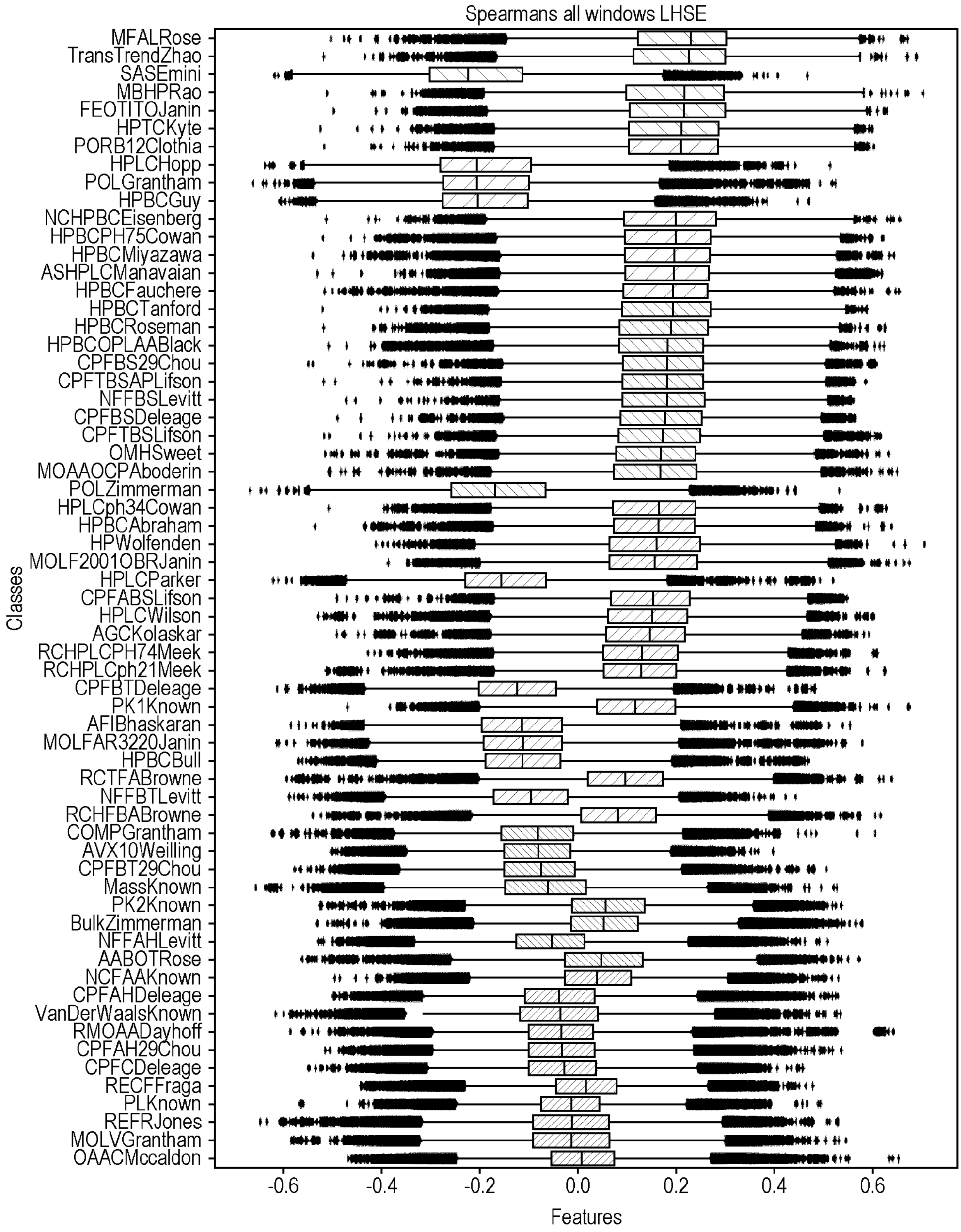


Continuous features with LHSE using median Spearman's correlation

*FIG. 8(c)*

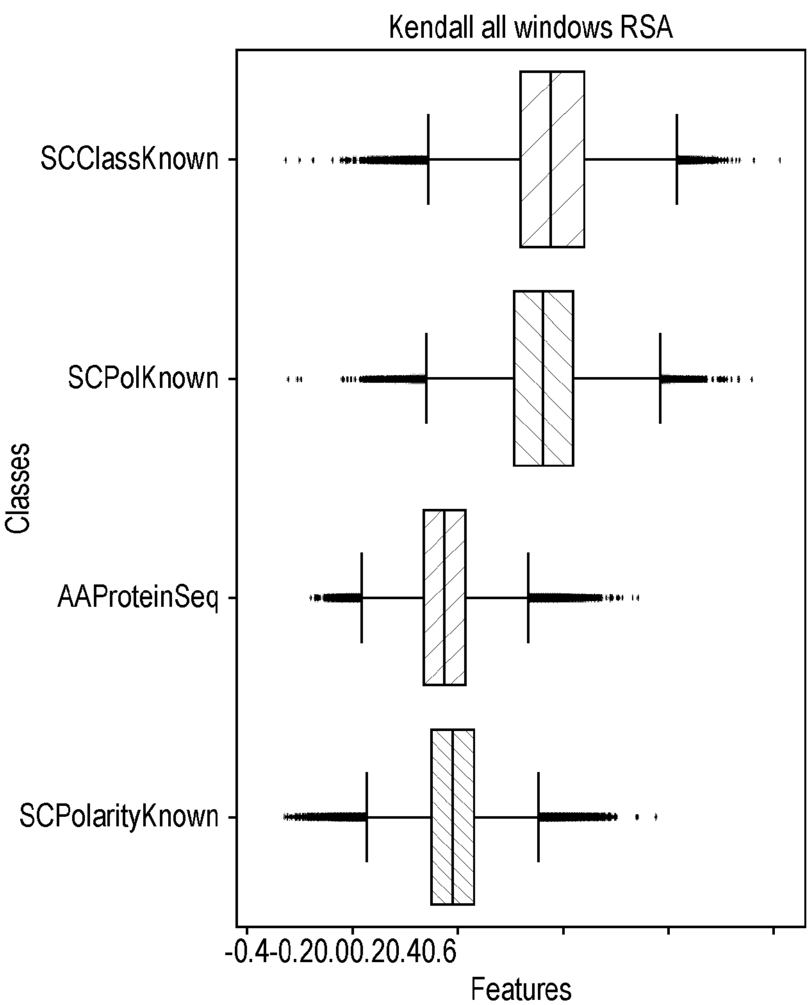


Categorical features with RSA using median Kendall's rank correlation

FIG. 9(a)

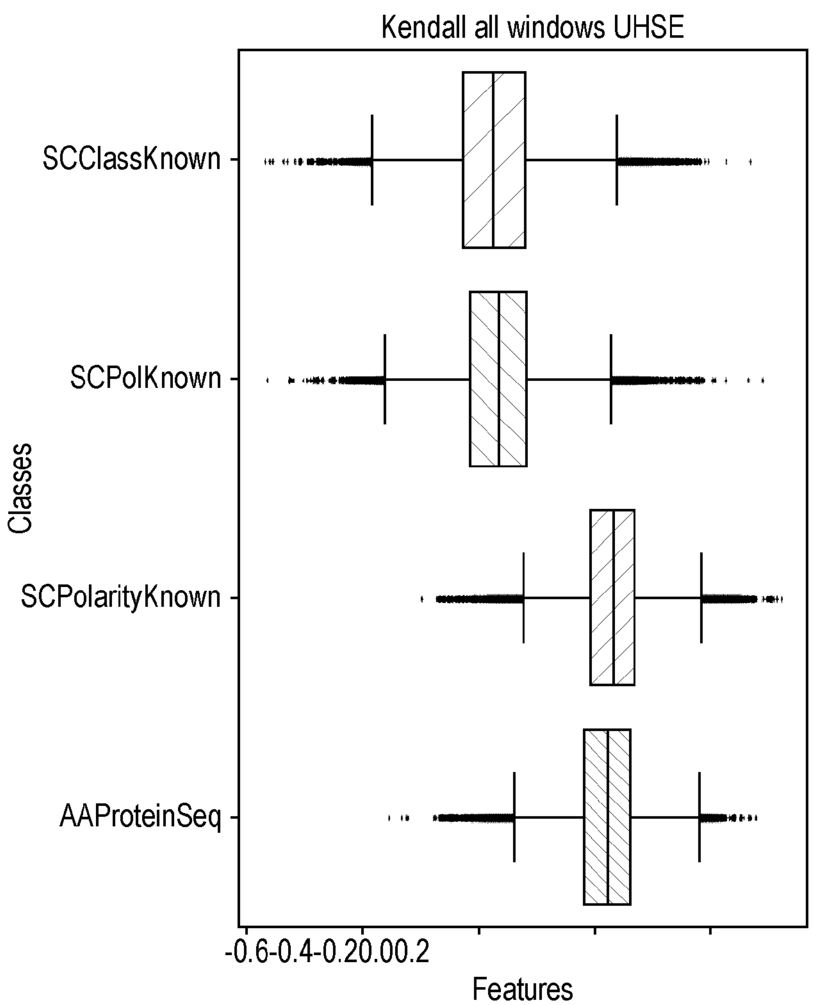


Categorical features with UHSE using median Kendall's rank correlation

FIG. 9(b)

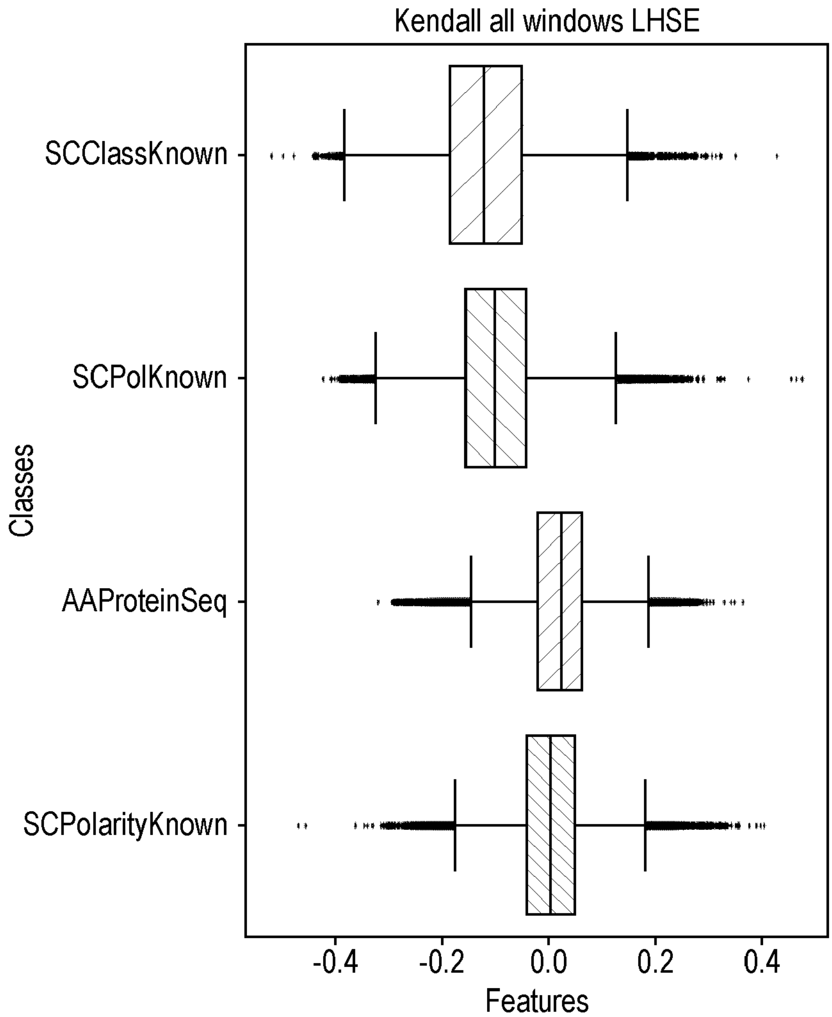


Categorical features with LHSE using median Kendall's rank correlation

*FIG. 9(c)*

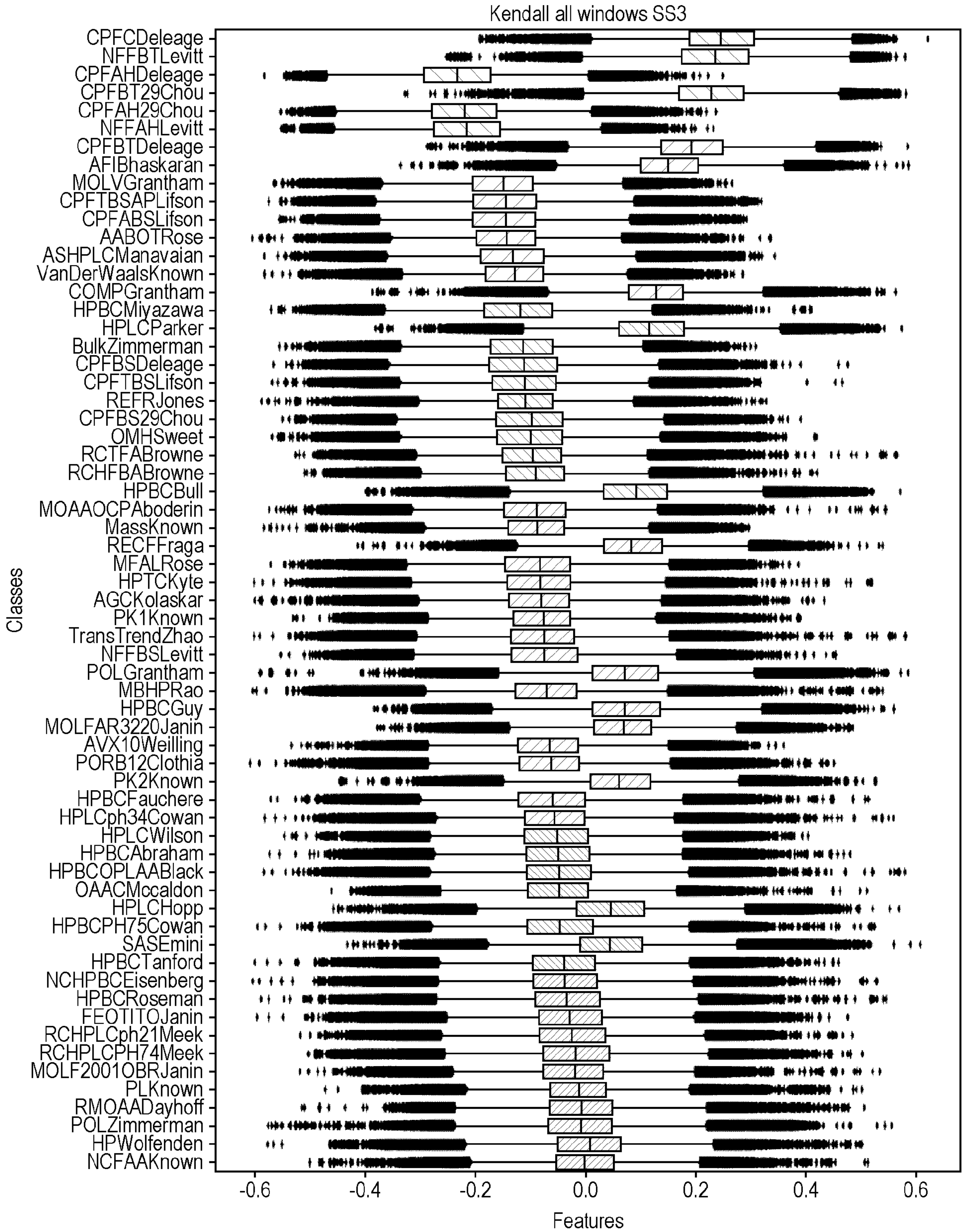


Continuous features with SS using median Kendall's rank correlation

*FIG. 10(a)*

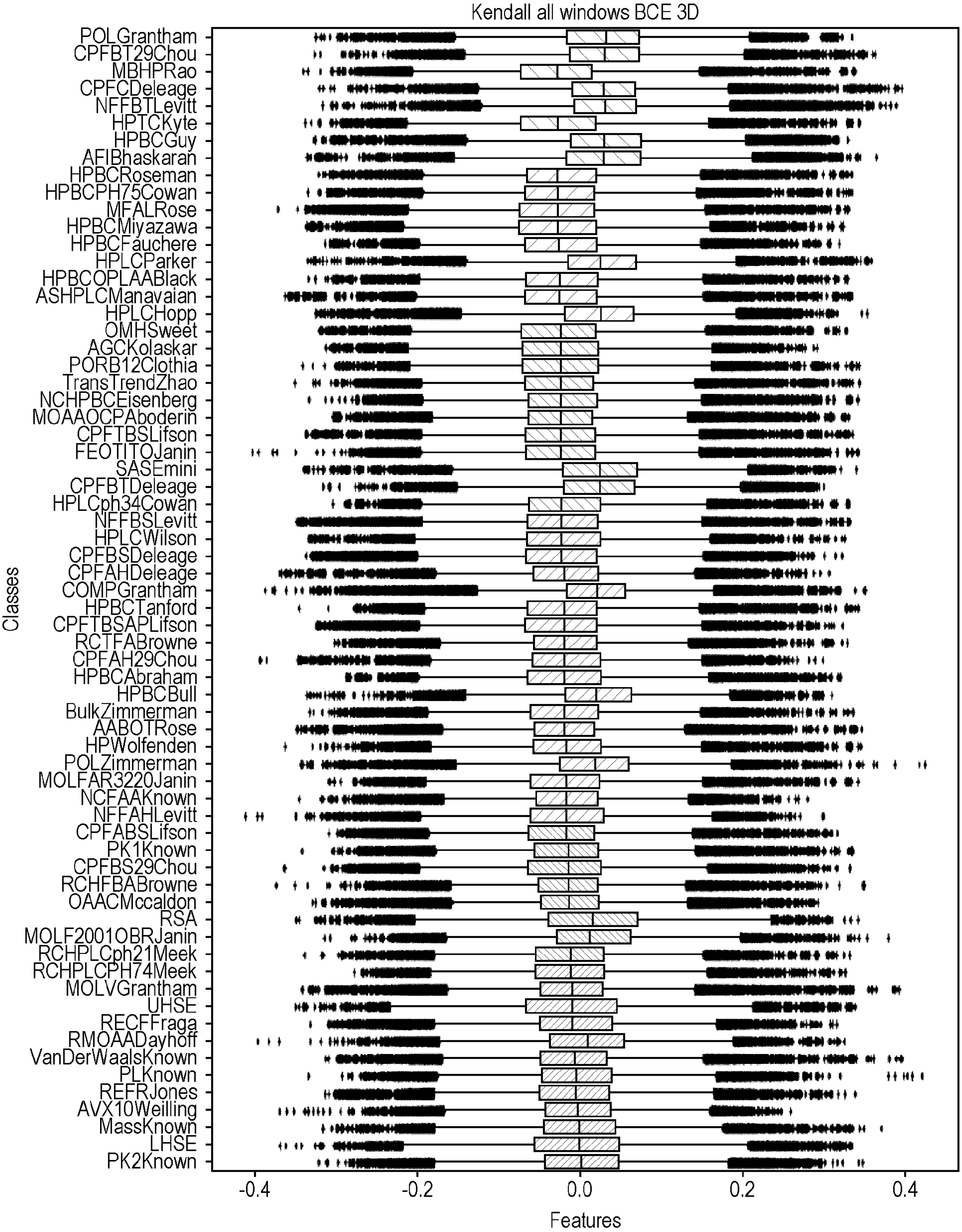


Continuous features with CBCE using median Kendall's rank correlation

*FIG. 10(b)*

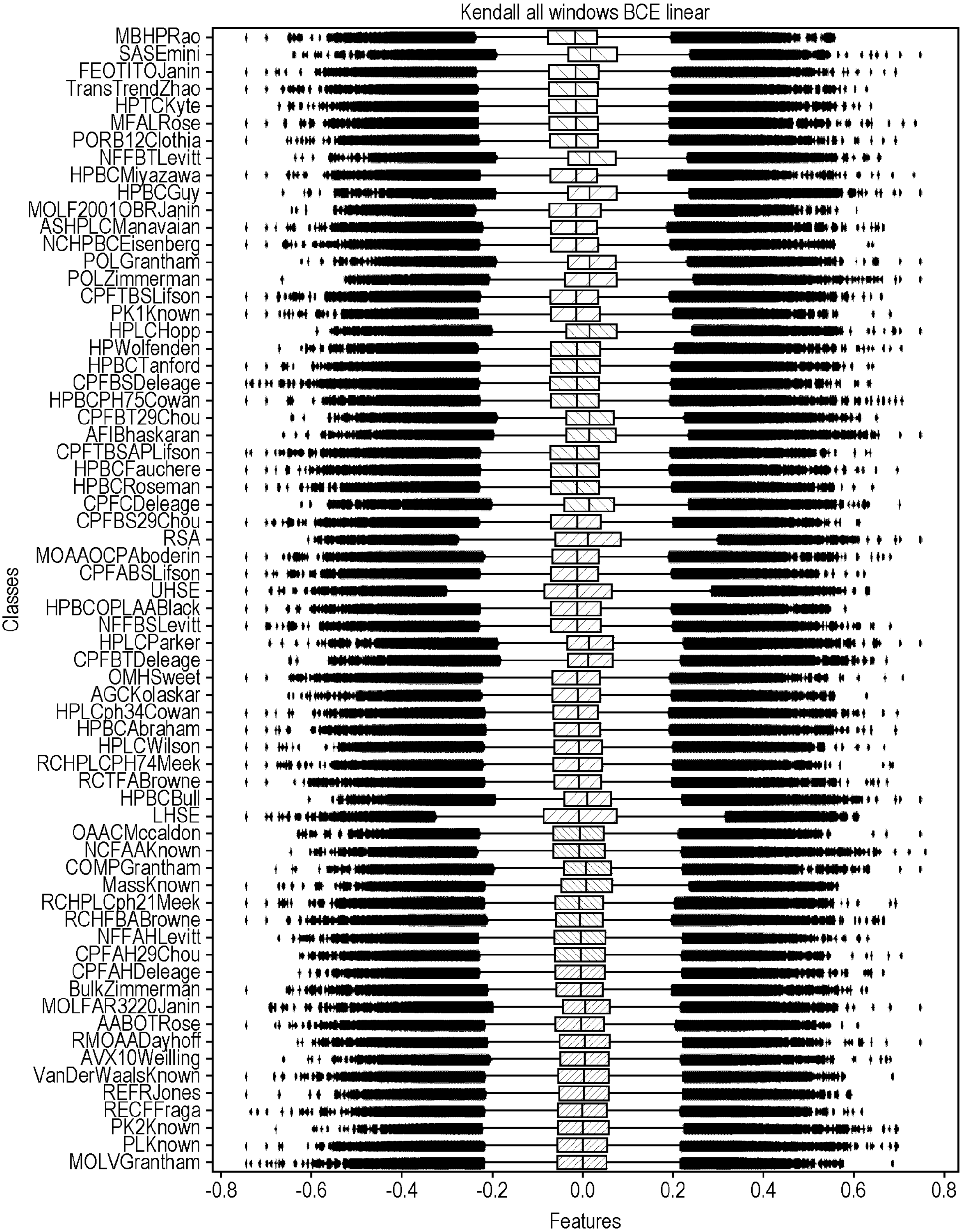


Continuous features with LBCE using median Kendall's rank correlation

*FIG. 10(c)*

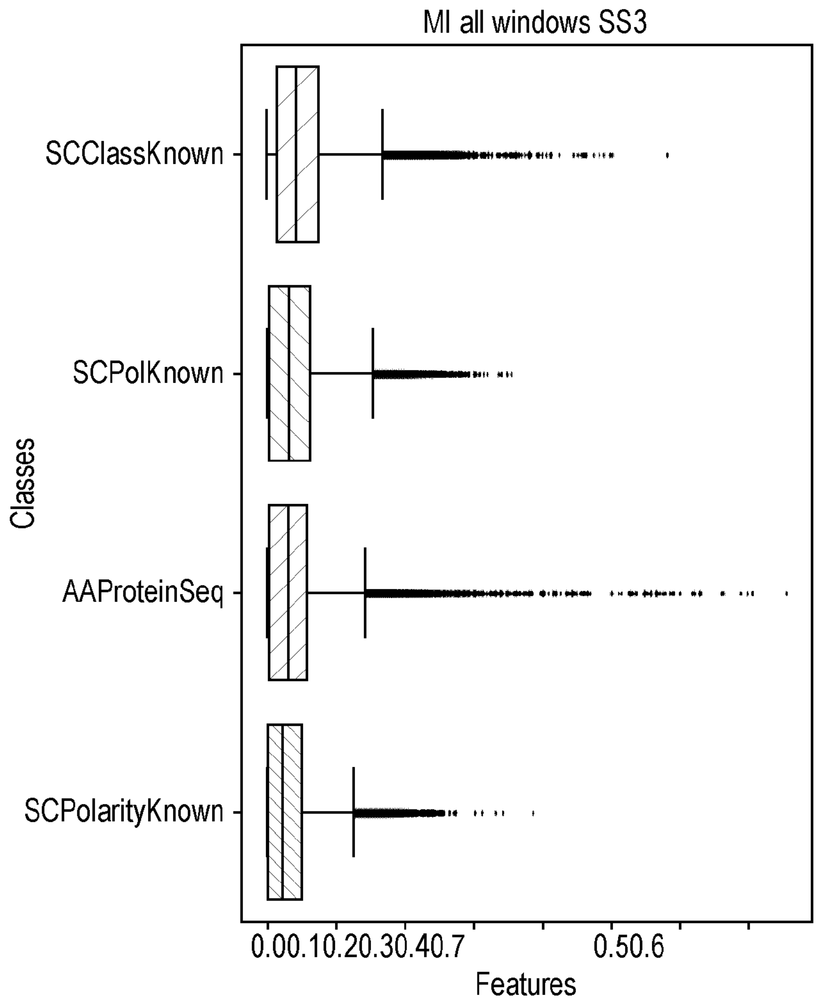


Categorical features with SS using
median Kendall's rank correlation

*FIG. 11(a)*

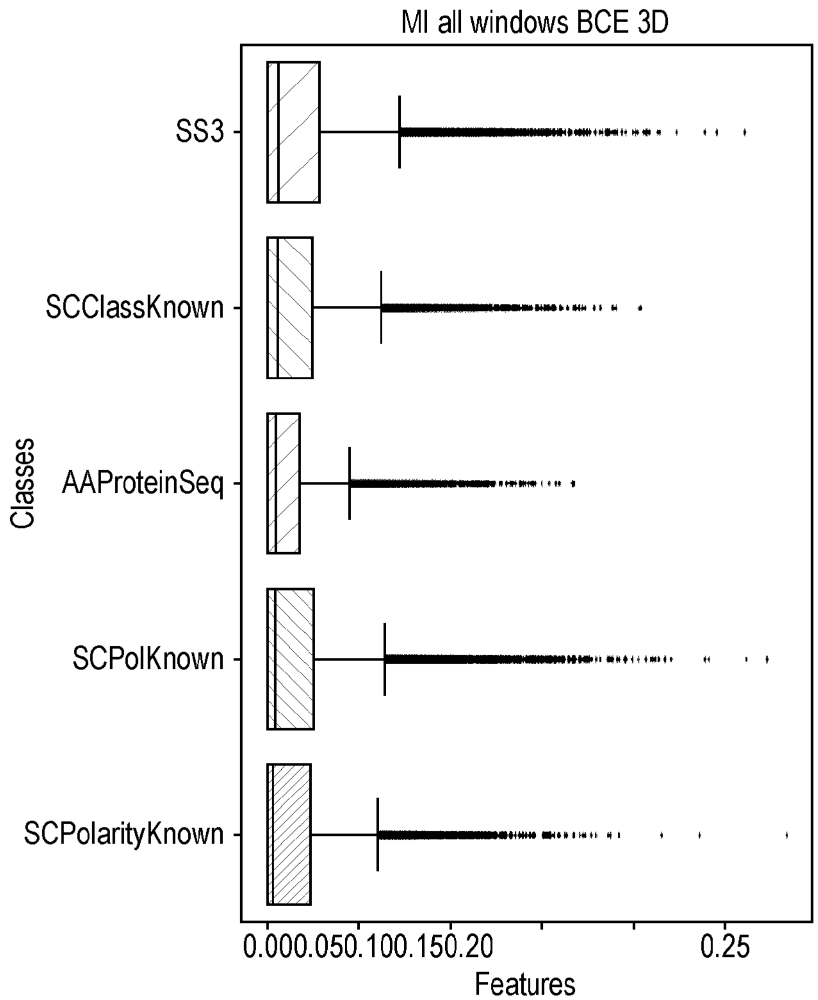


Categorical features with CBCE using
median Kendall's rank correlation

*FIG. 11(b)*

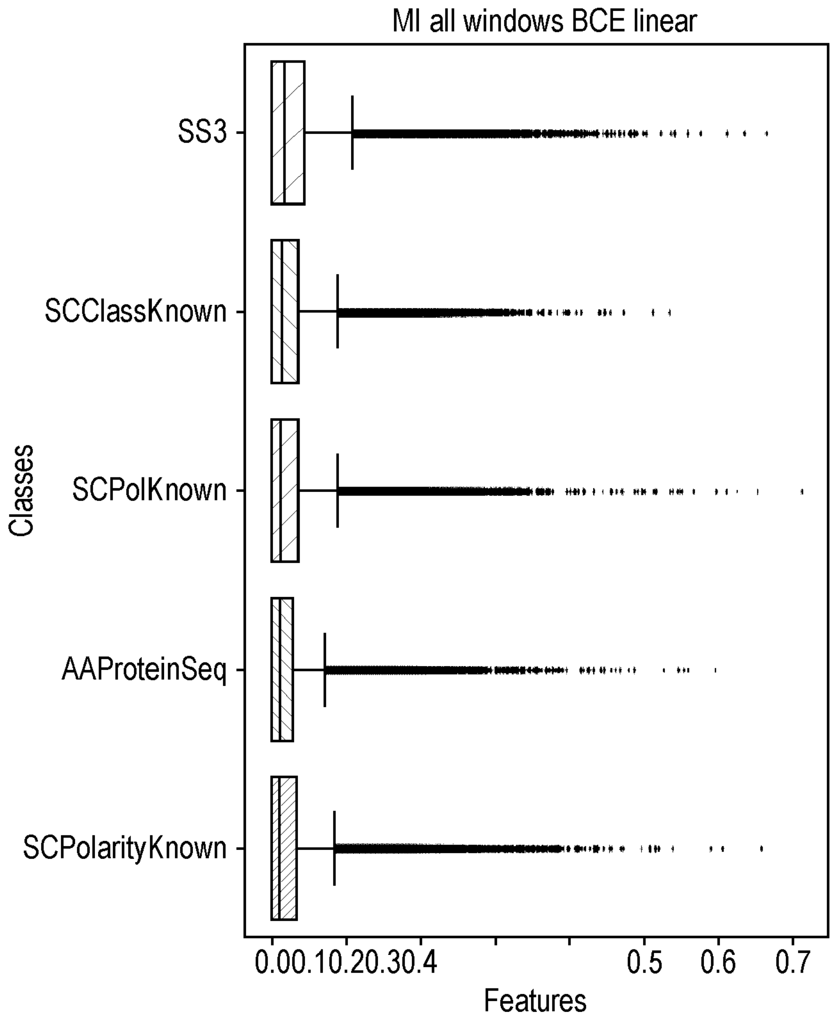


Categorical features with LBCE using median Kendall's rank correlation

FIG. 11(c)

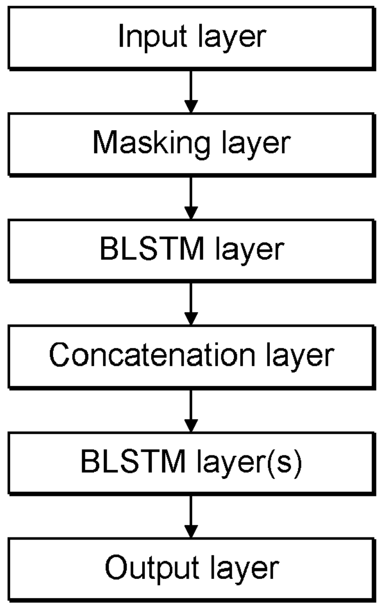


FIG. 12

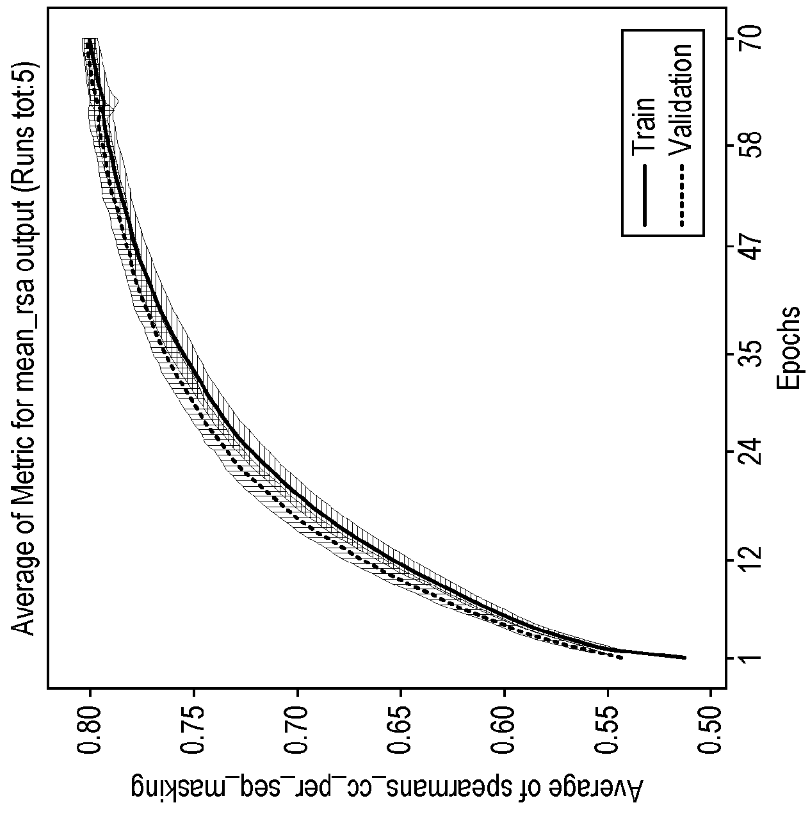


Spearman's correlation coefficient for RSA per sequence

FIG. 13(a)

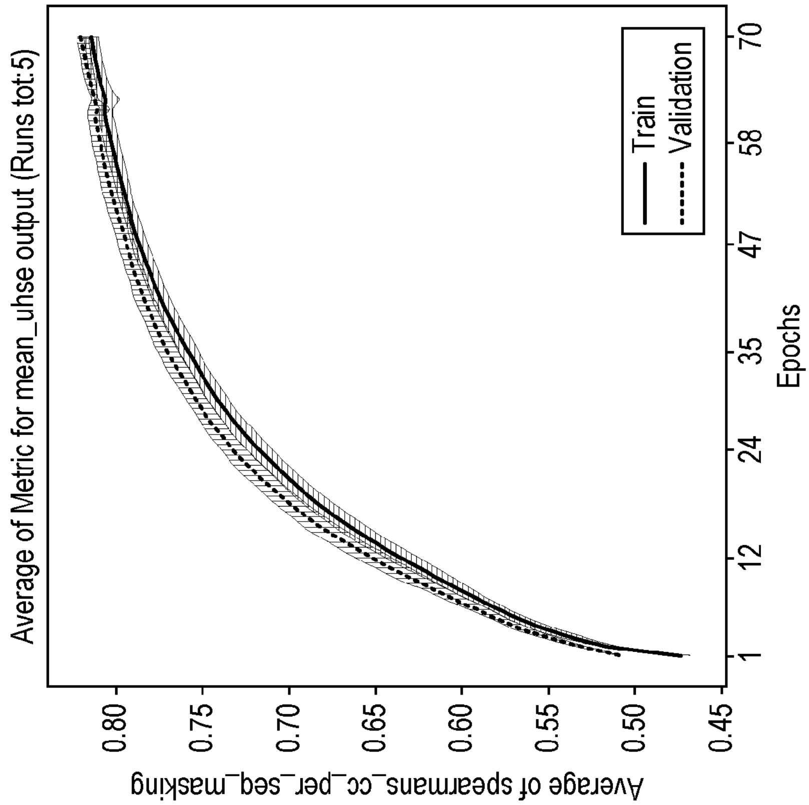


Spearman's correlation coefficient for UHSE per sequence

FIG. 13(b)

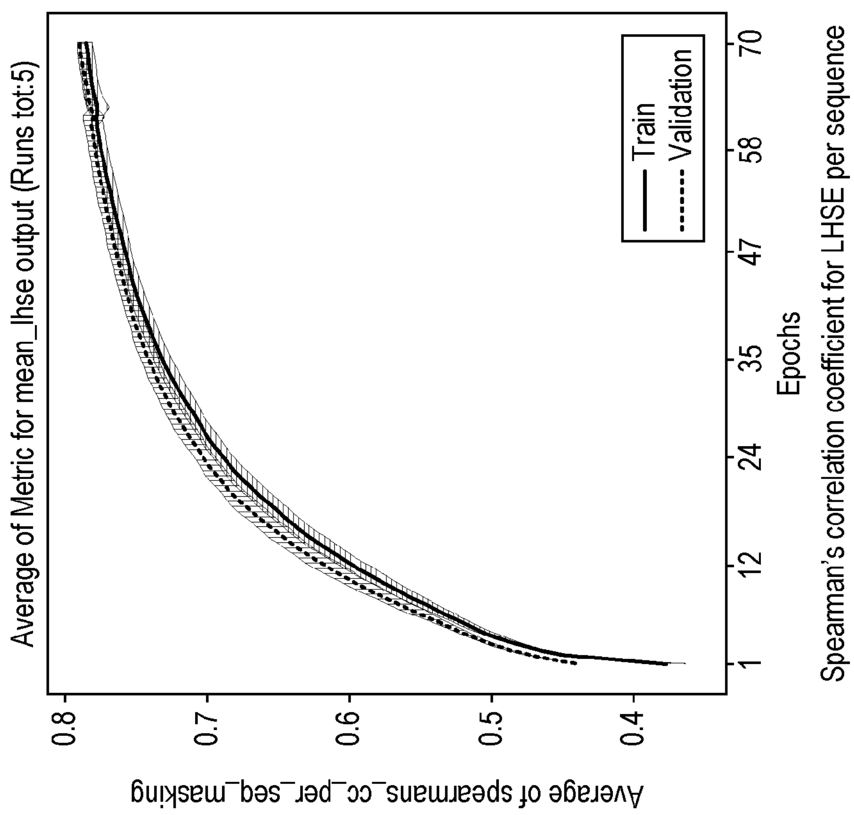


FIG. 13(c)

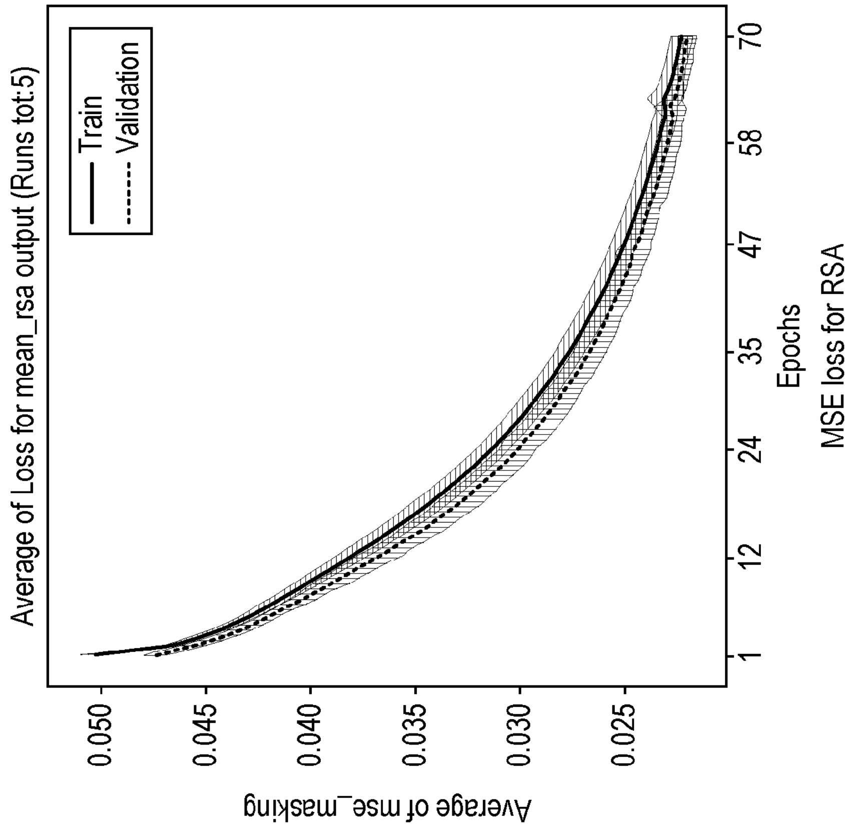


FIG. 13(d)

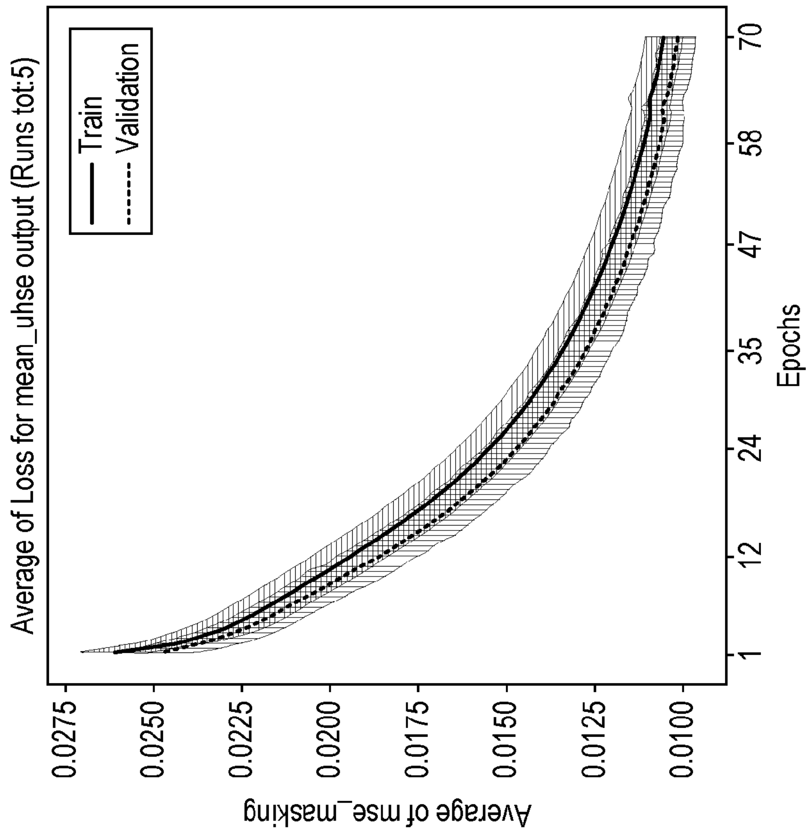


MSE loss for UHSE

*FIG. 13(e)*

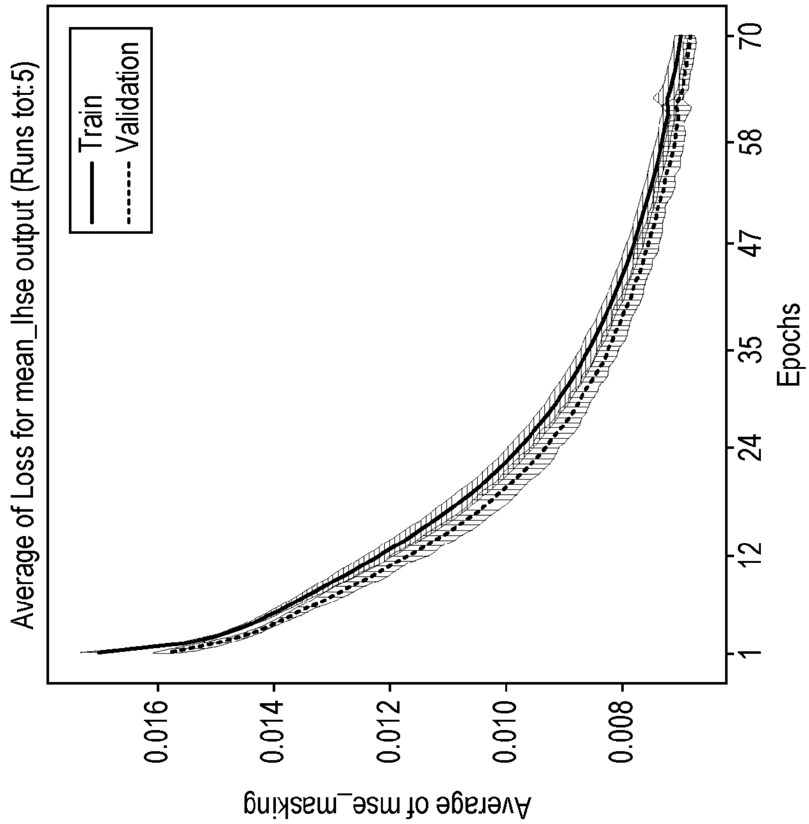


MSE loss for LHSE

*FIG. 13(f)*

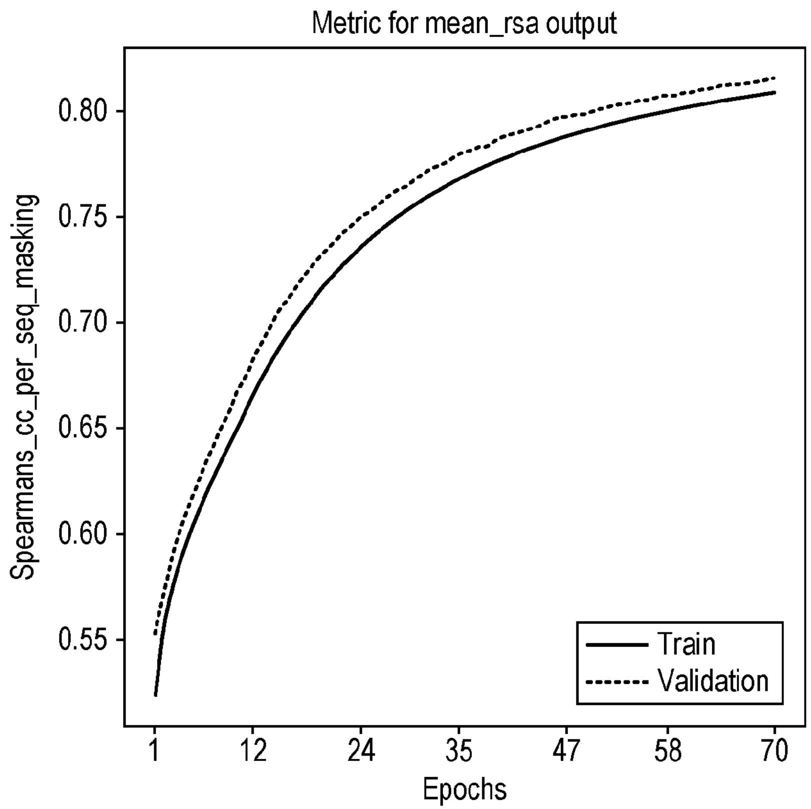


Spearman's correlation coefficient for RSA per sequence

FIG. 14(a)

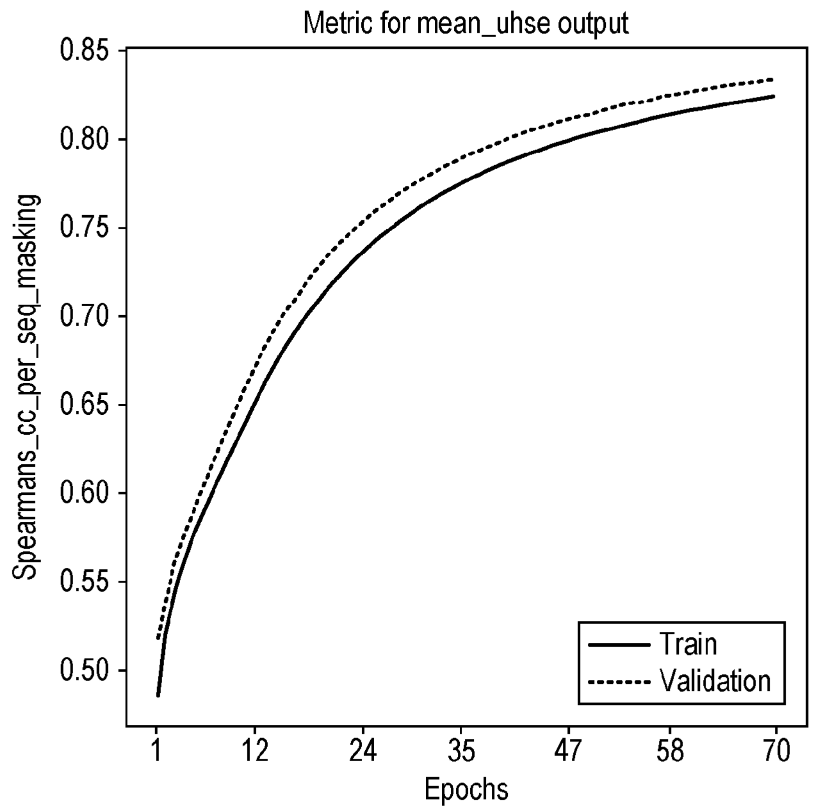


Spearman's correlation coefficient for UHSE per sequence

FIG. 14(b)

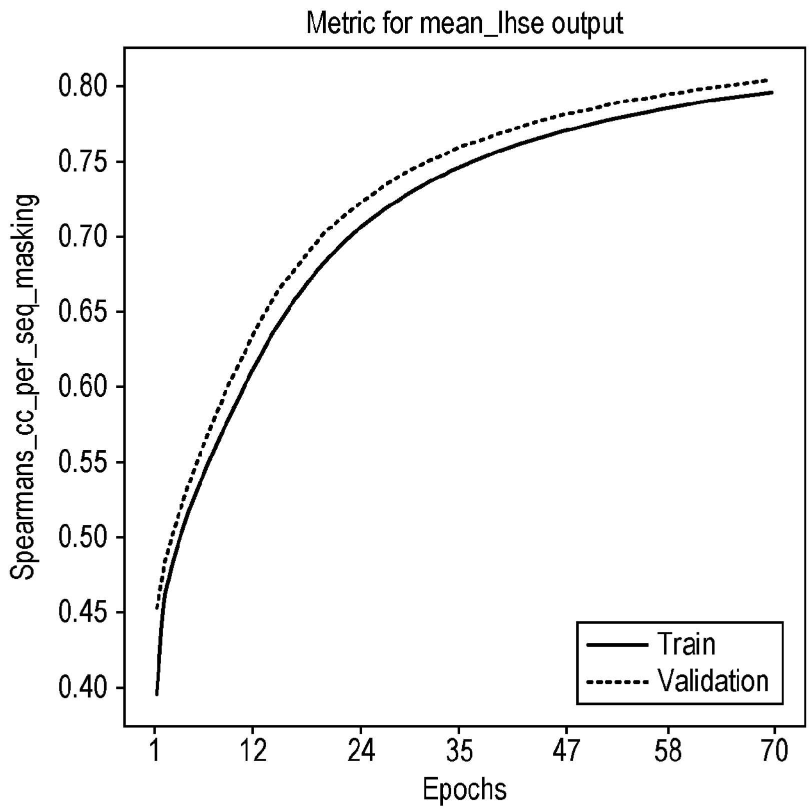


Spearman's correlation coefficient for LHSE per sequence

FIG. 14(c)

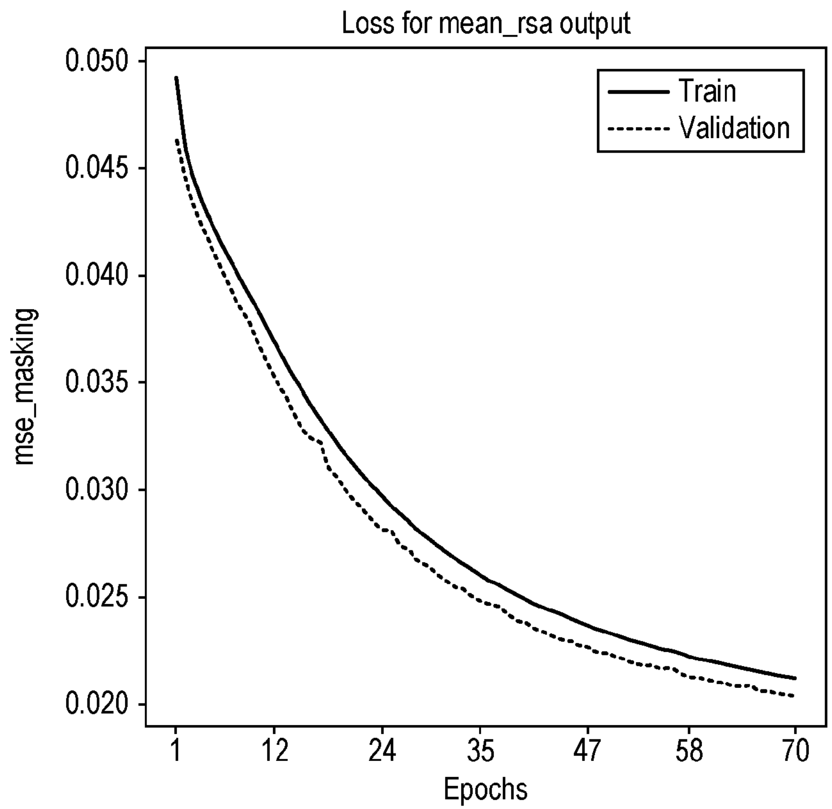


MSE loss for RSA

FIG. 14(d)

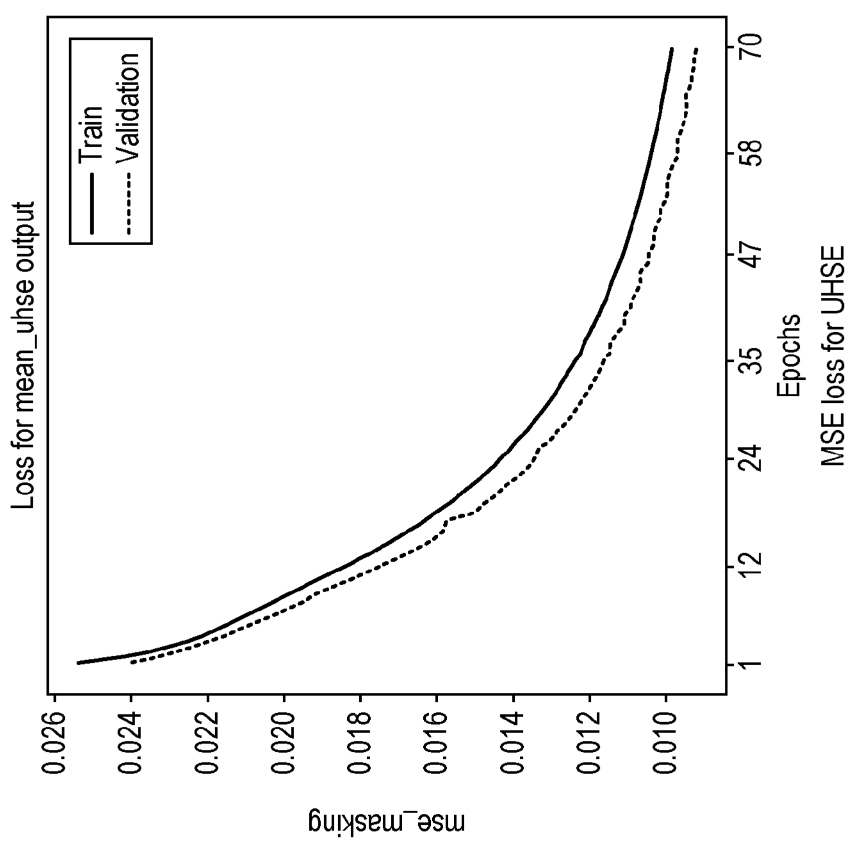


FIG. 14(e)

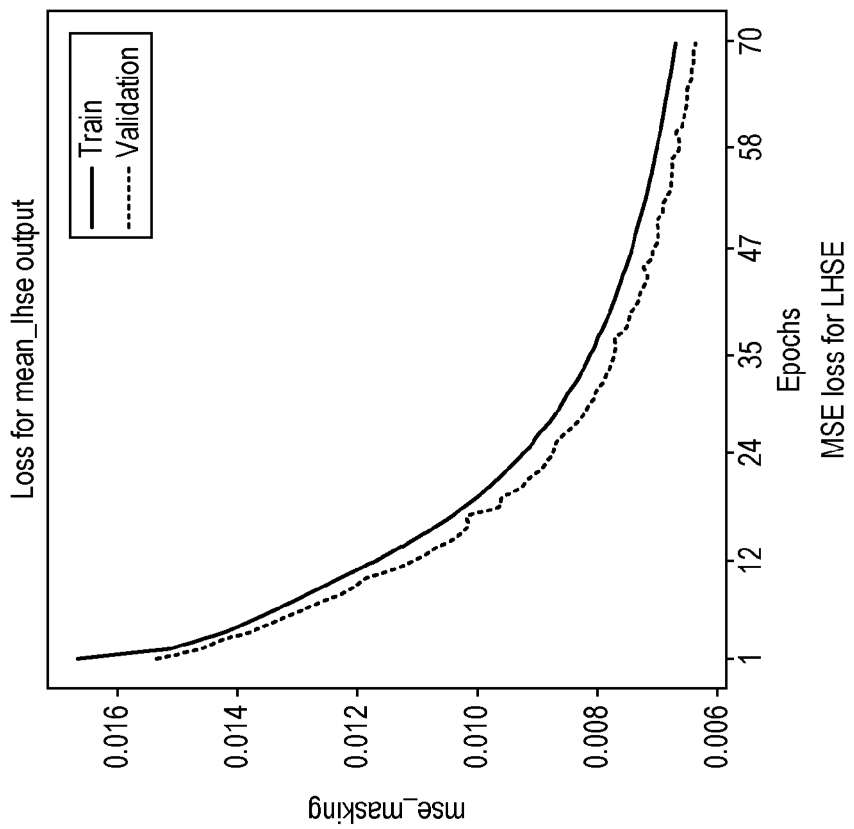


FIG. 14(f)

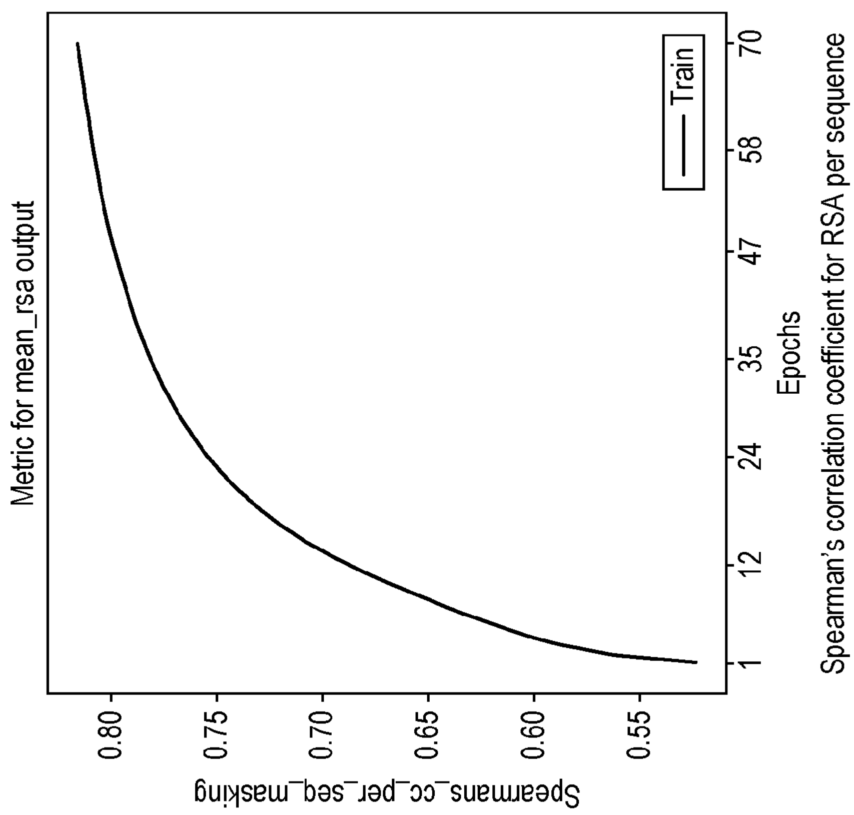


FIG. 15(a)

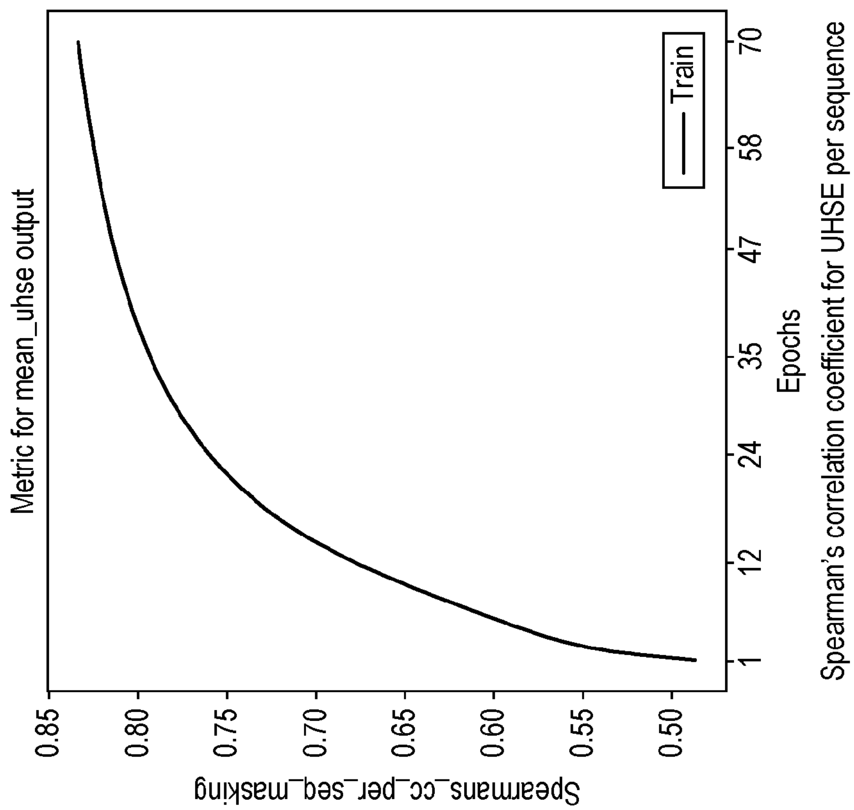


FIG. 15(b)

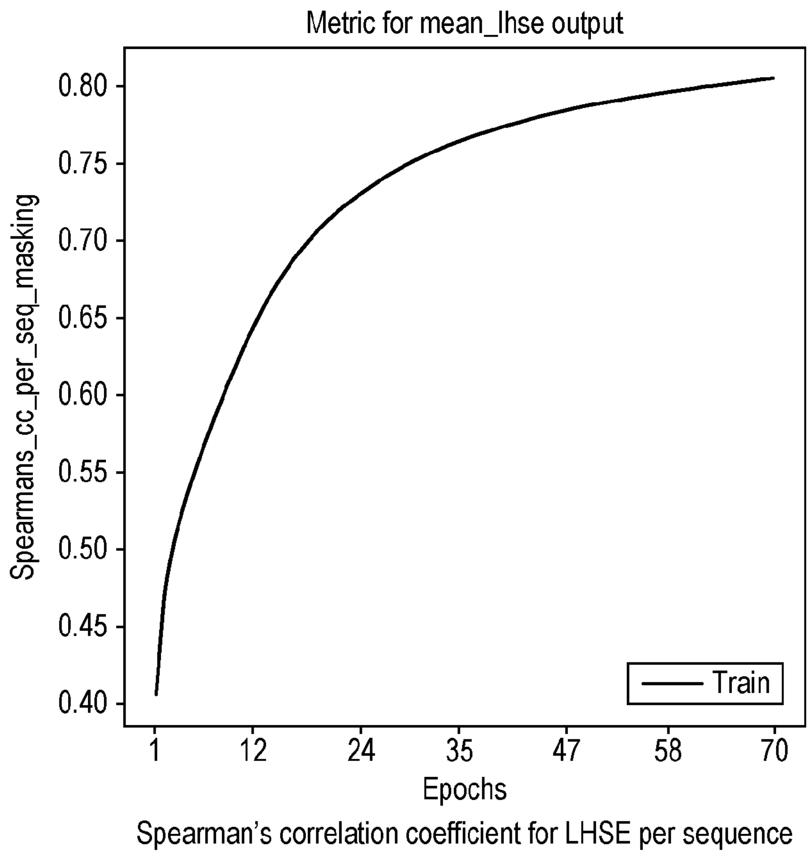


FIG. 15(c)

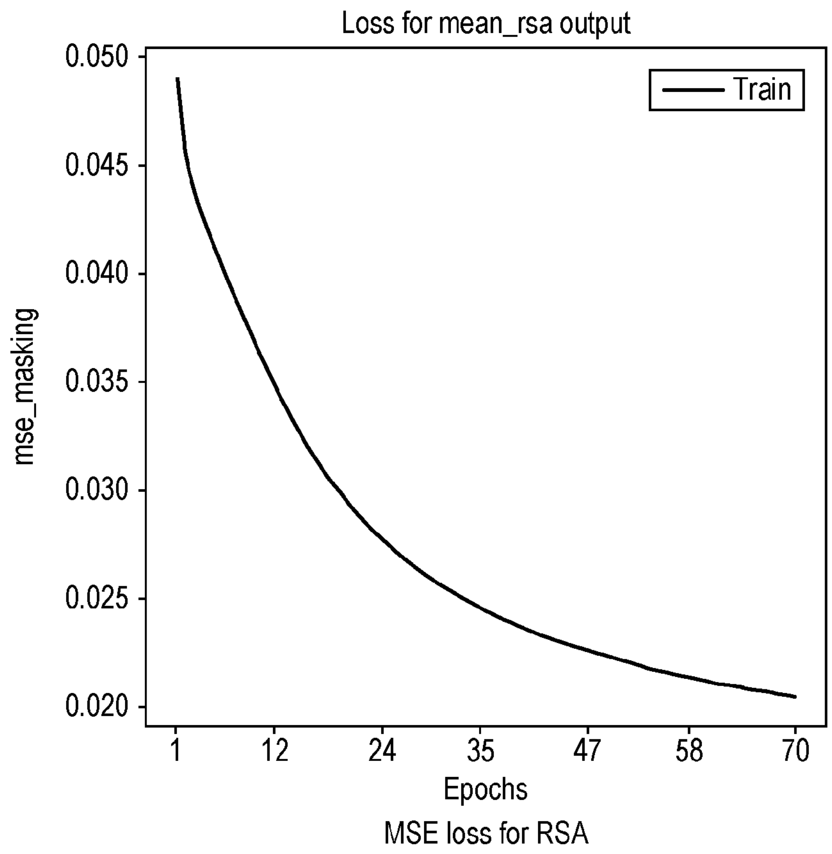


FIG. 15(d)

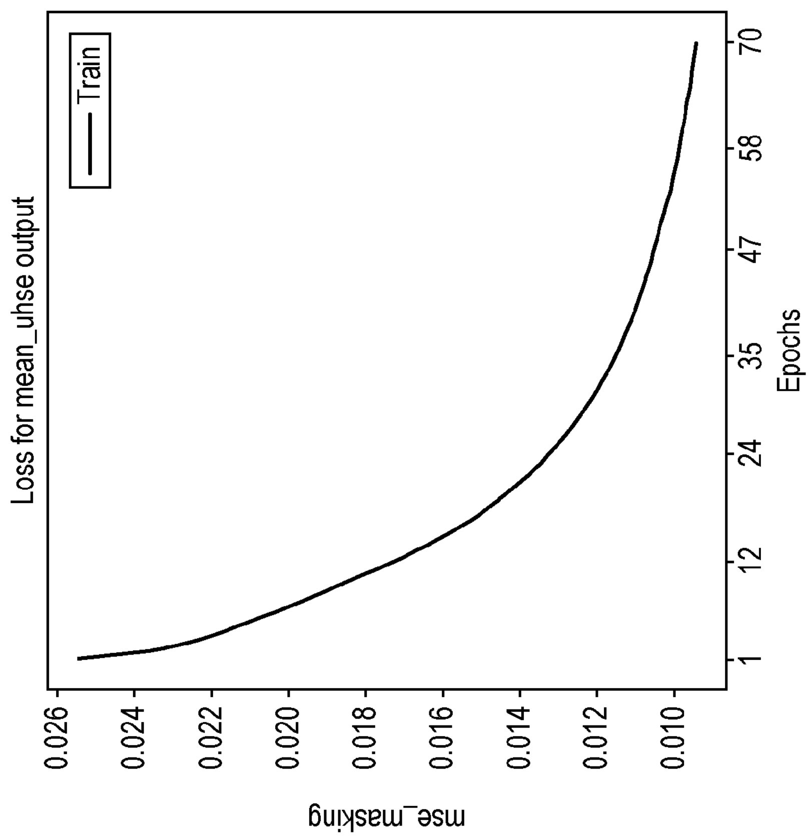

MSE loss for UHSE

FIG. 15(e)

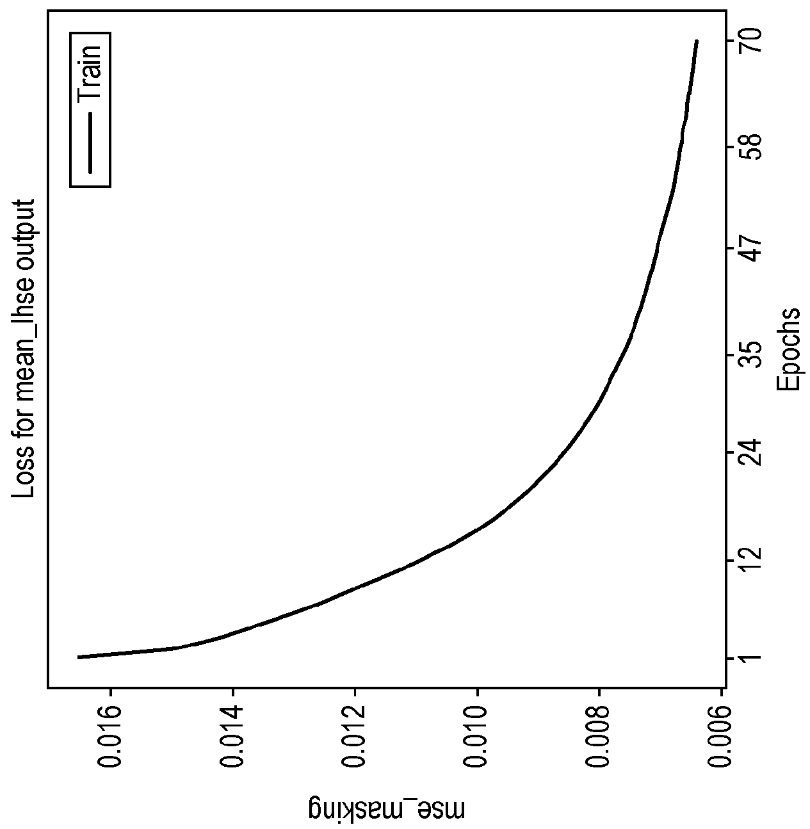

MSE loss for LHSE

FIG. 15(f)

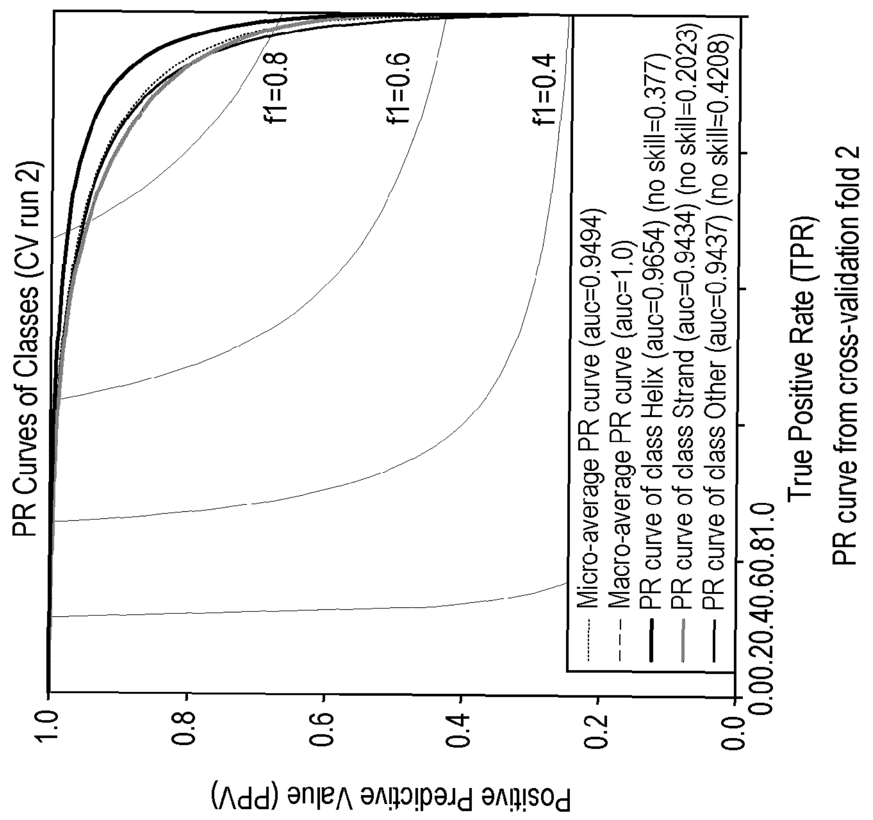


FIG. 16(b)

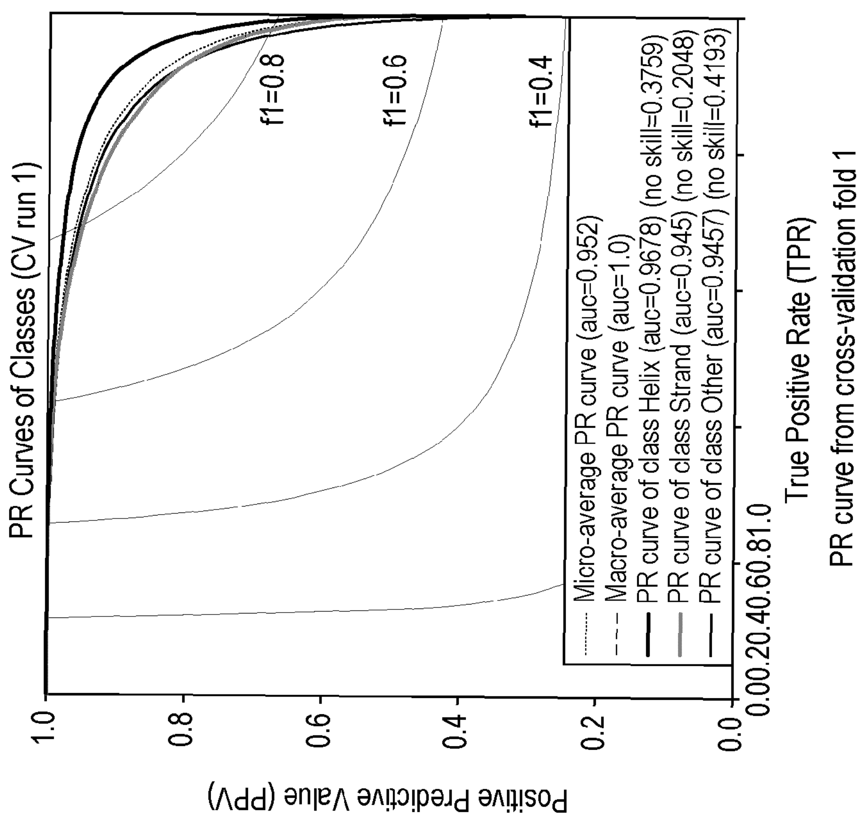


FIG. 16(a)

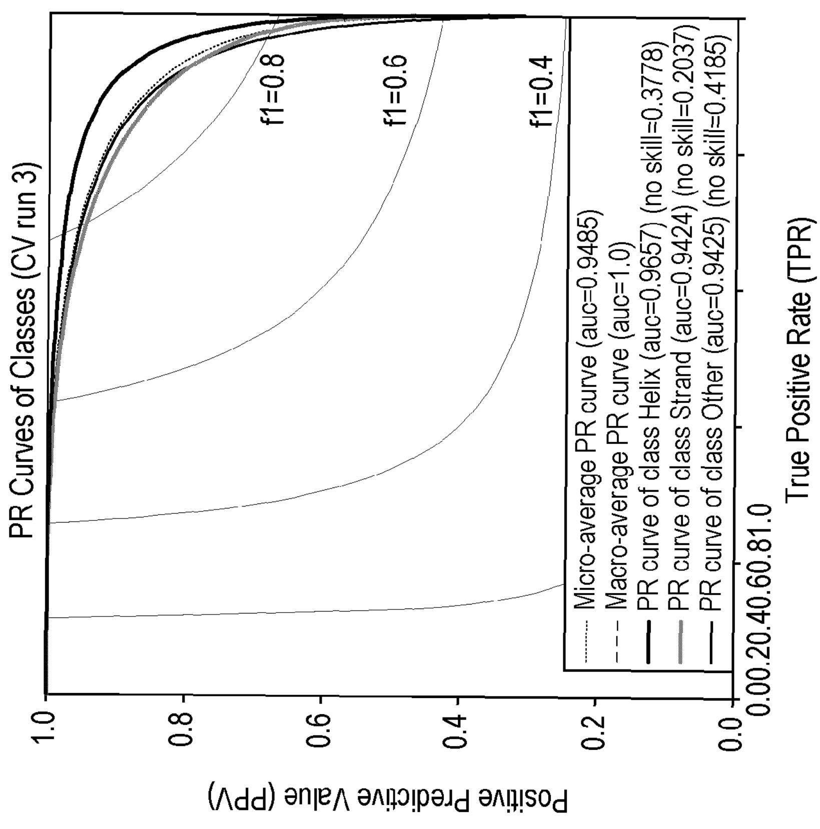

PR curve from cross-validation fold 3

FIG. 16(c)

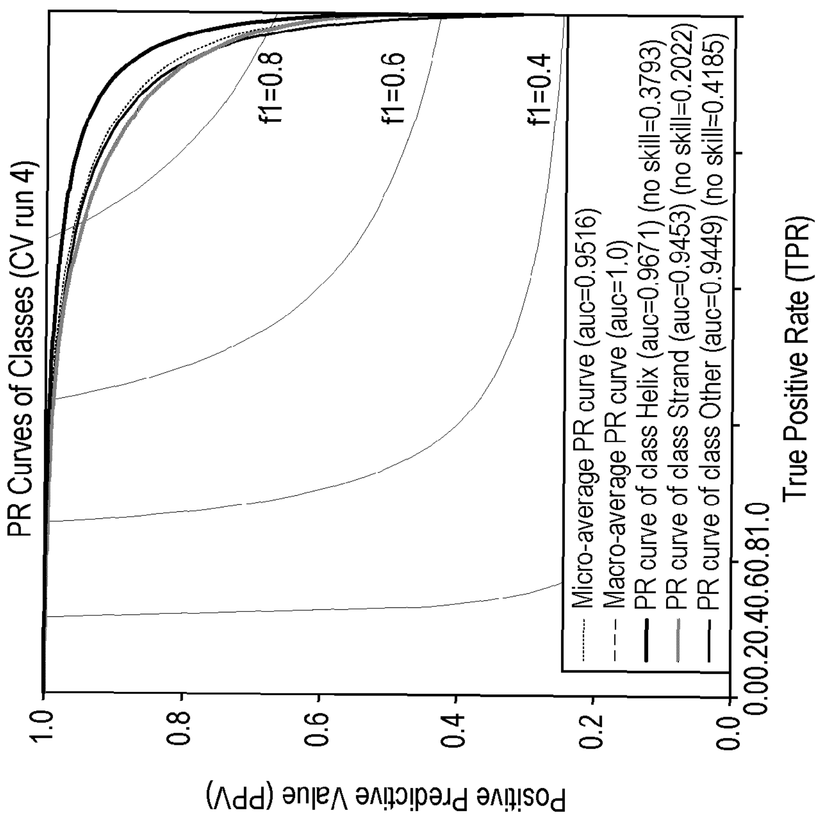

PR curve from cross-validation fold 4

FIG. 16(d)

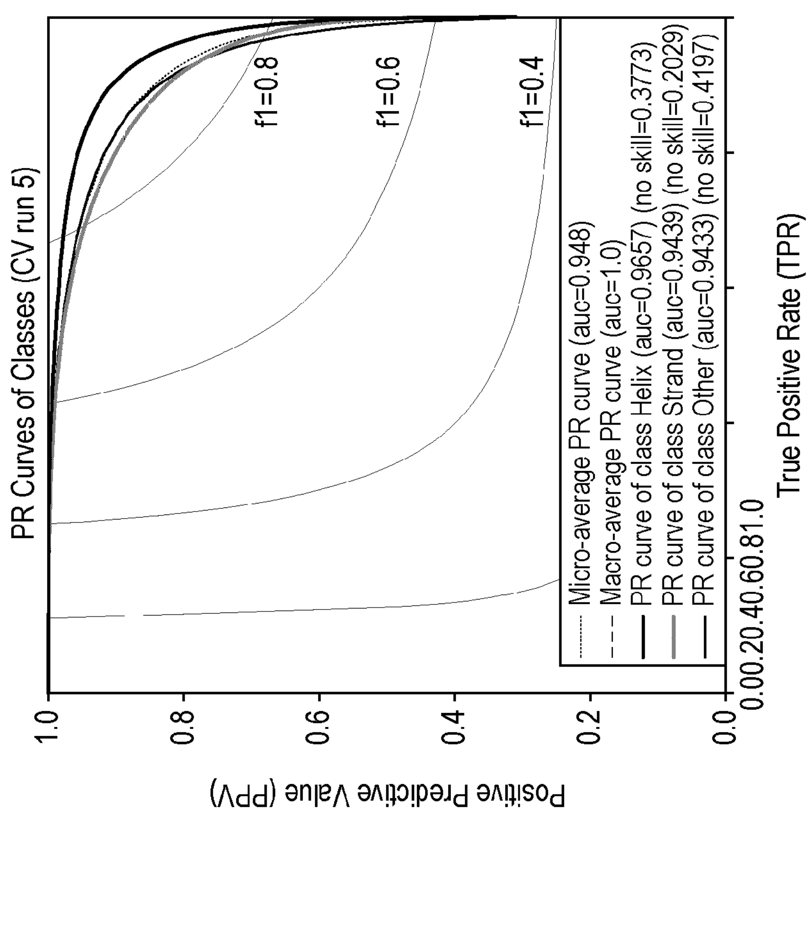


PR curve from cross-validation fold 5

FIG. 16(e)

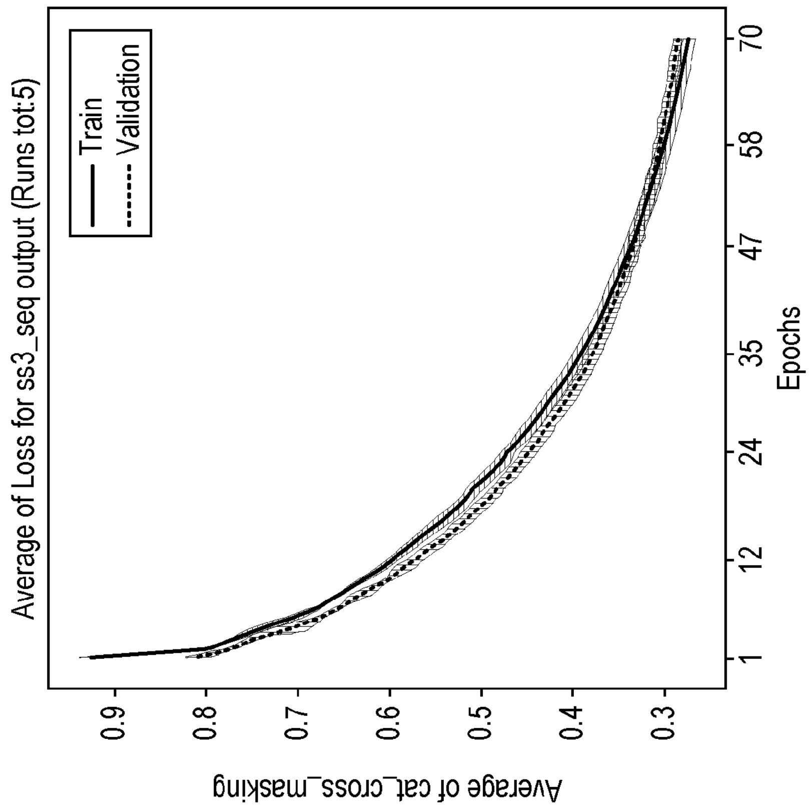


Categorical cross-entropy loss for SS

FIG. 16(f)

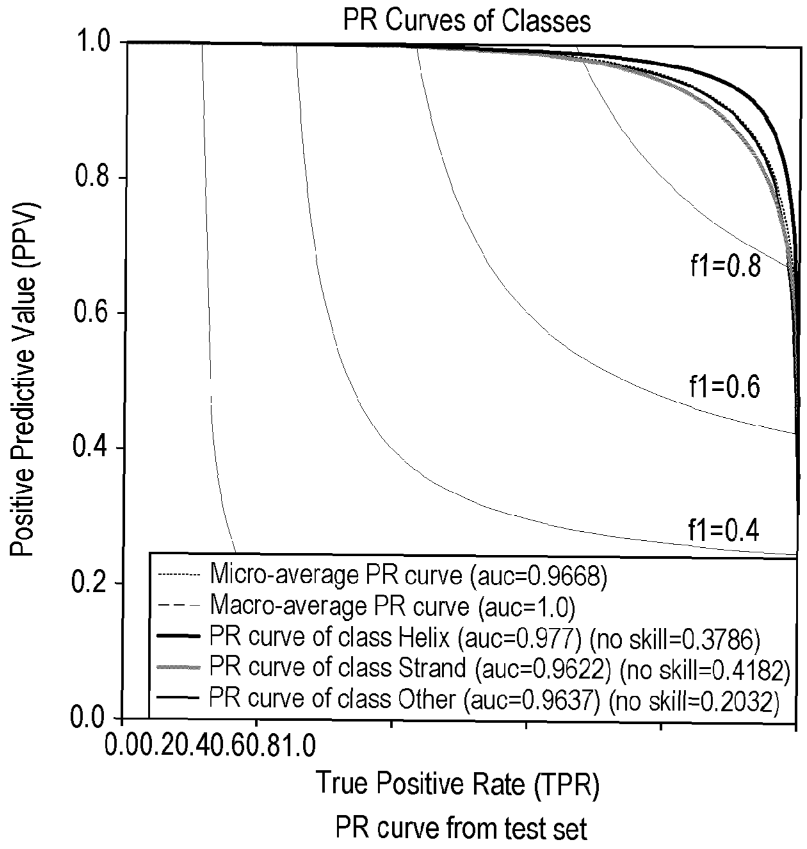


FIG. 17(a)

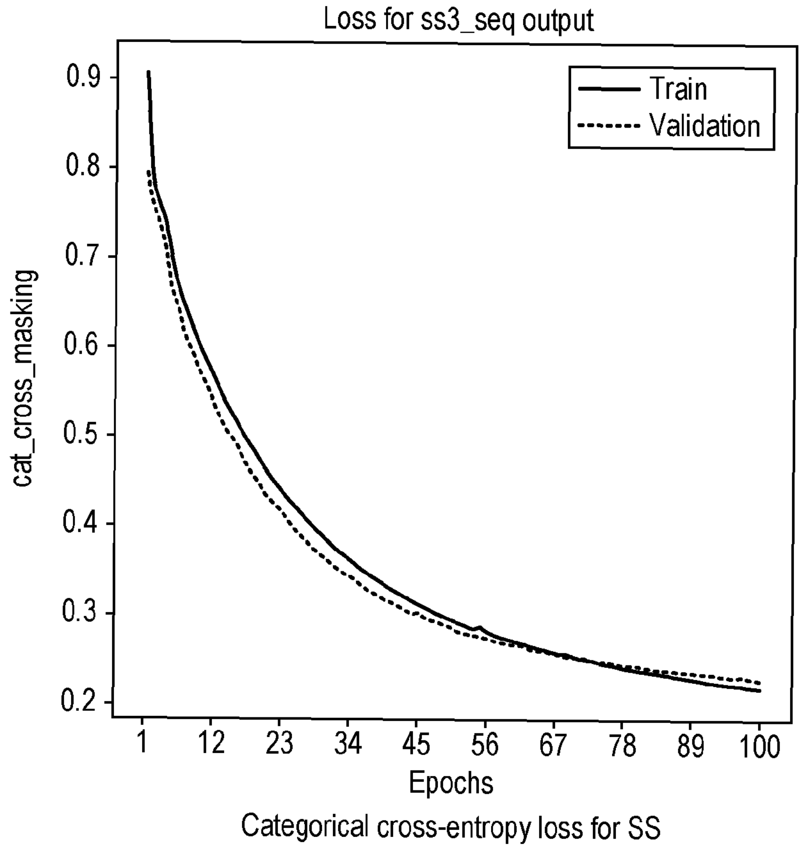


FIG. 17(b)

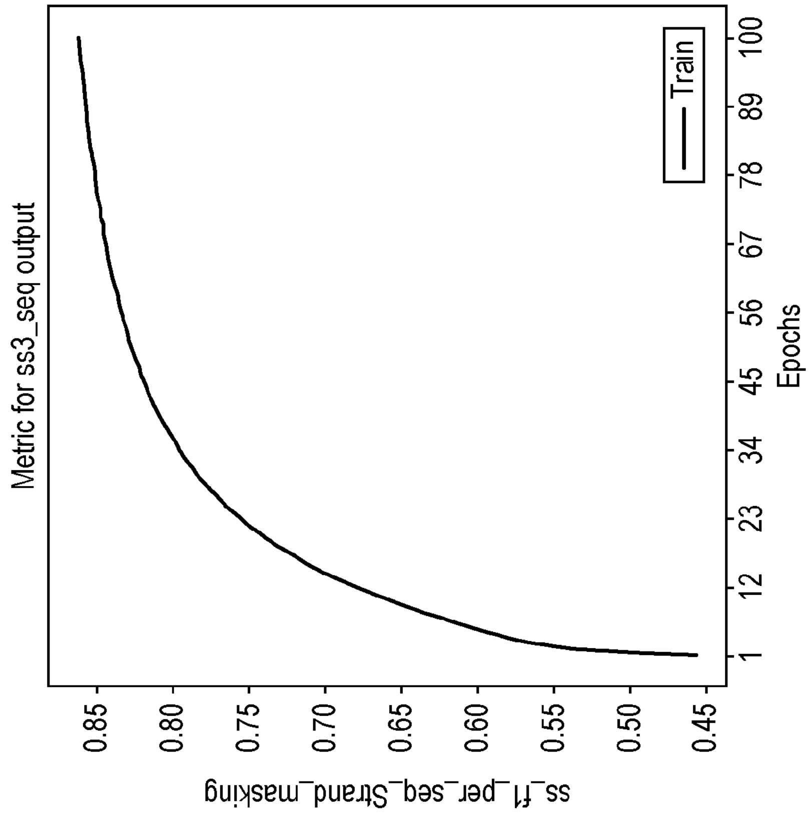


F1 curve for Strand for final estimates

FIG. 18(b)

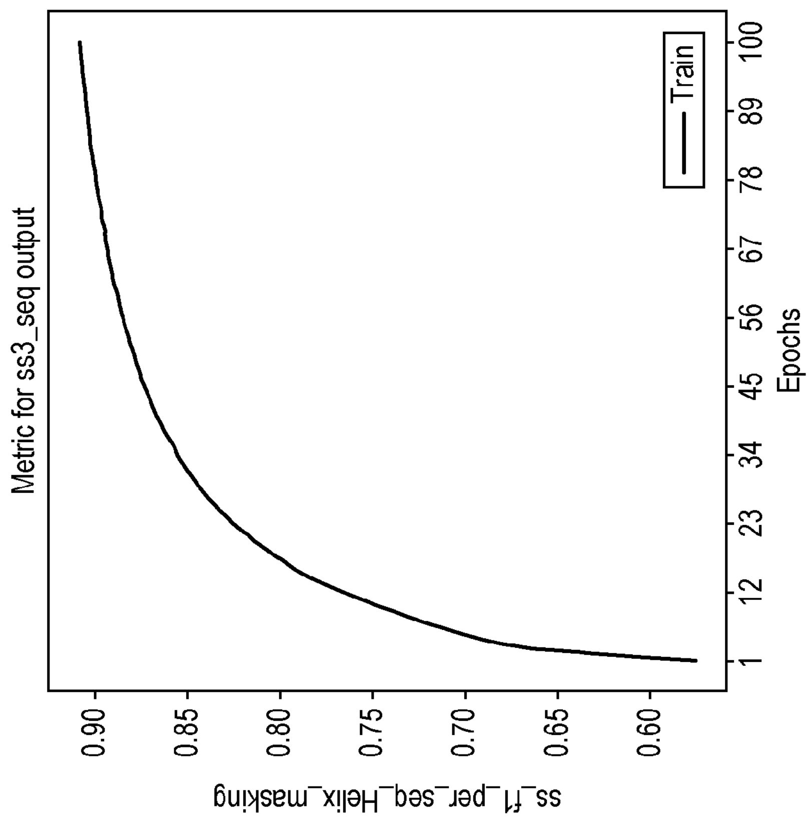


F1 curve for Helix for final estimates

FIG. 18(a)

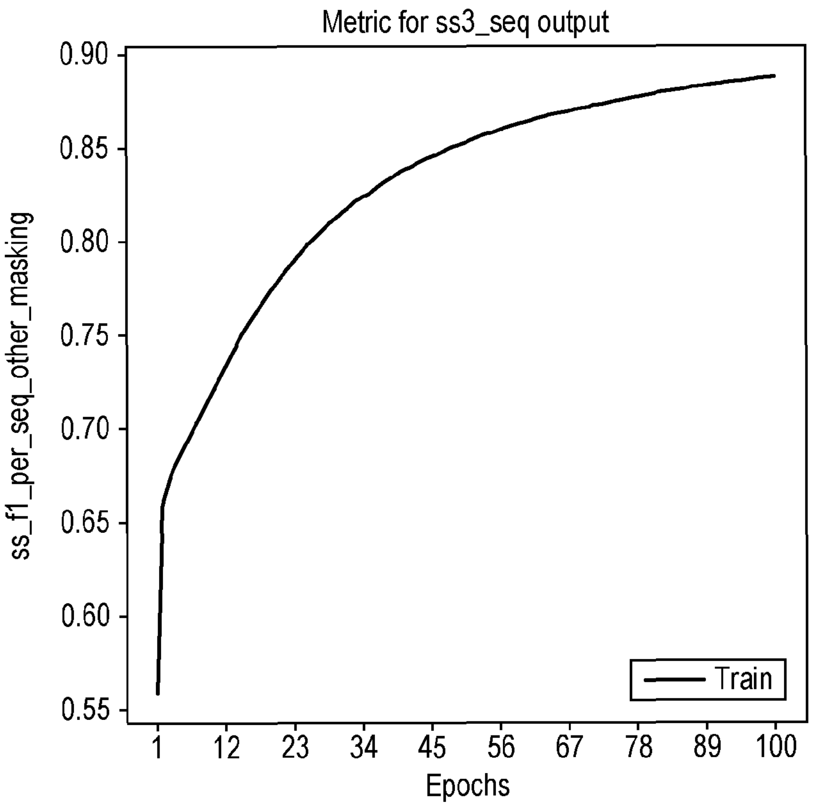


F1 curve for Coil for final estimates

*FIG. 18(c)*

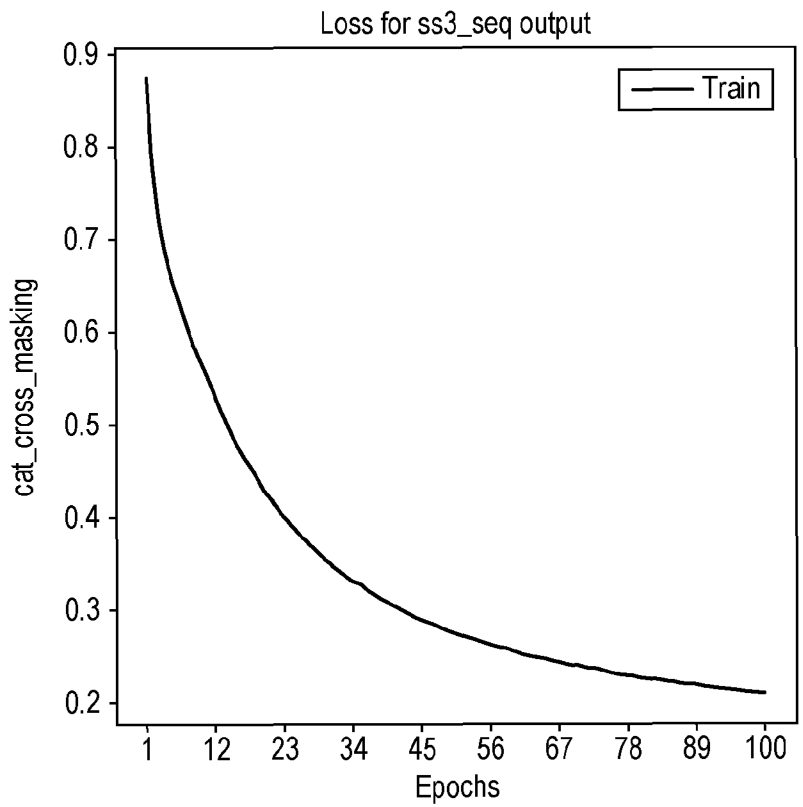


Categorical cross-entropy loss for SS

*FIG. 18(d)*

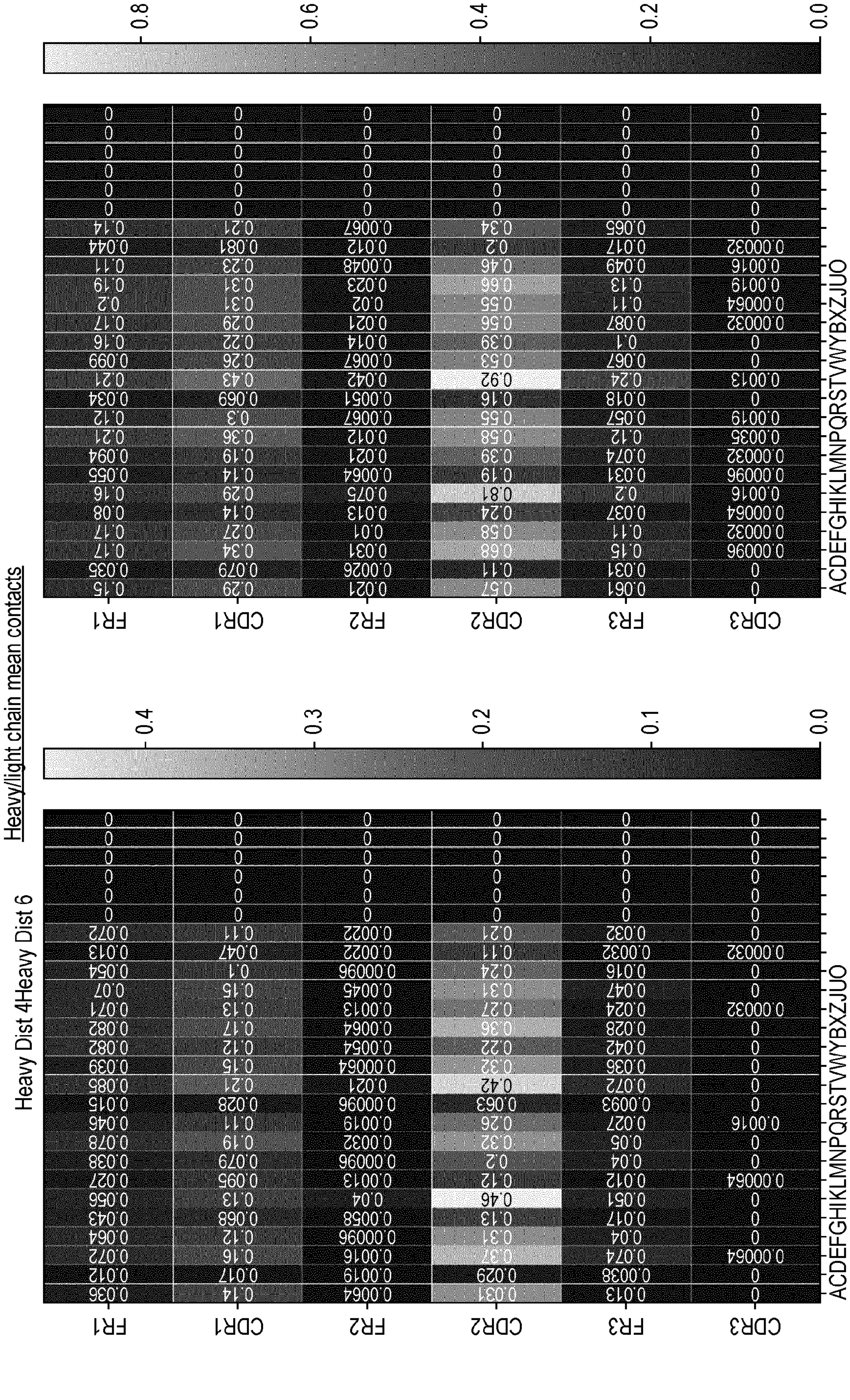
Heavy/light chain mean contacts
Heavy Dist 4Heavy Dist 6
FR1
CDR1
FR2
CDR2
FR3
CDR3
ACDEFGHIKLMNPQRSTVWYBXZJUO
0.4
0.3
0.2
0.1
0.0
0.8
0.6
0.4
0.2
0.0

FIG. 19(a)

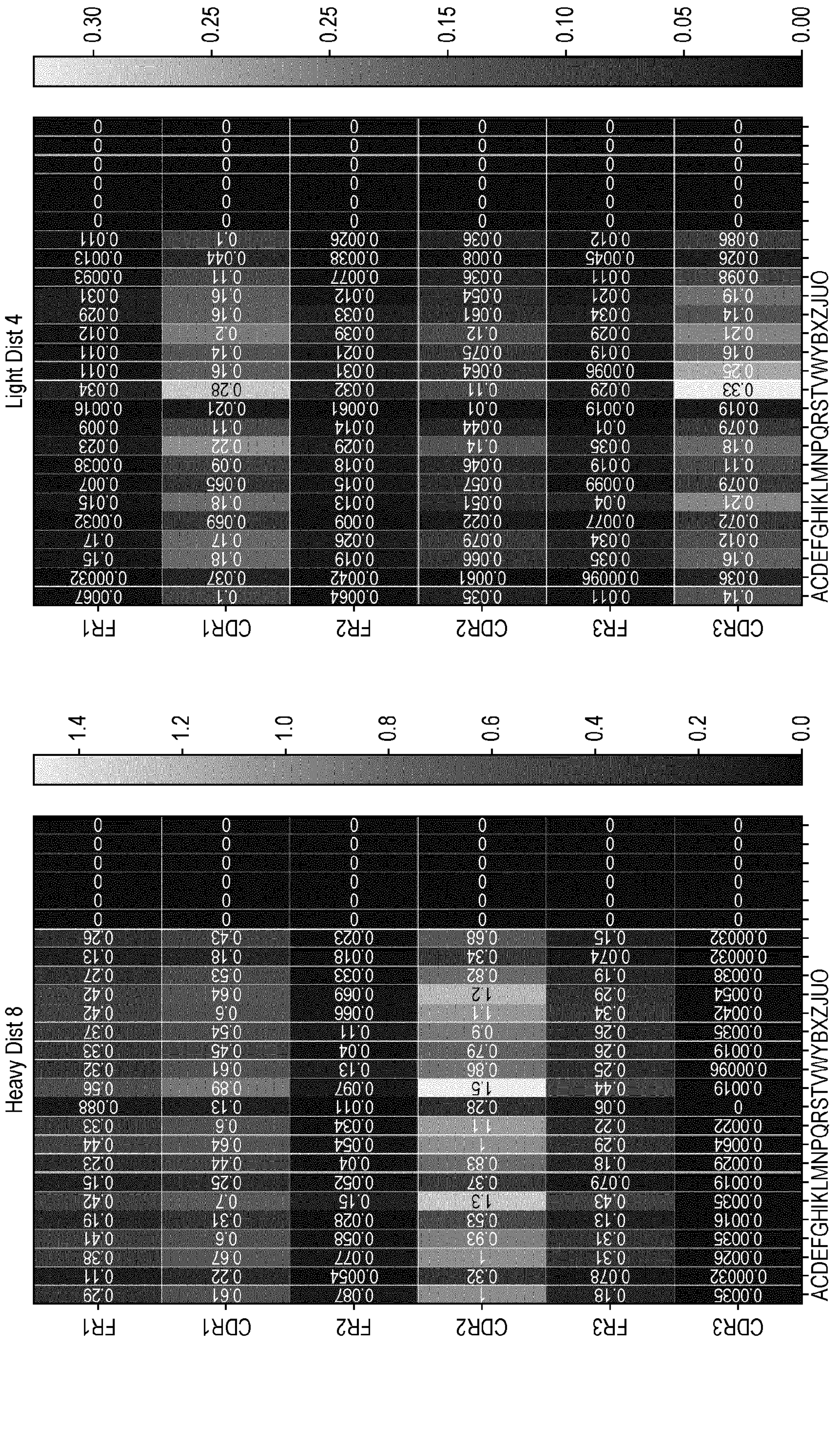


FIG. 19(a) Cont'd

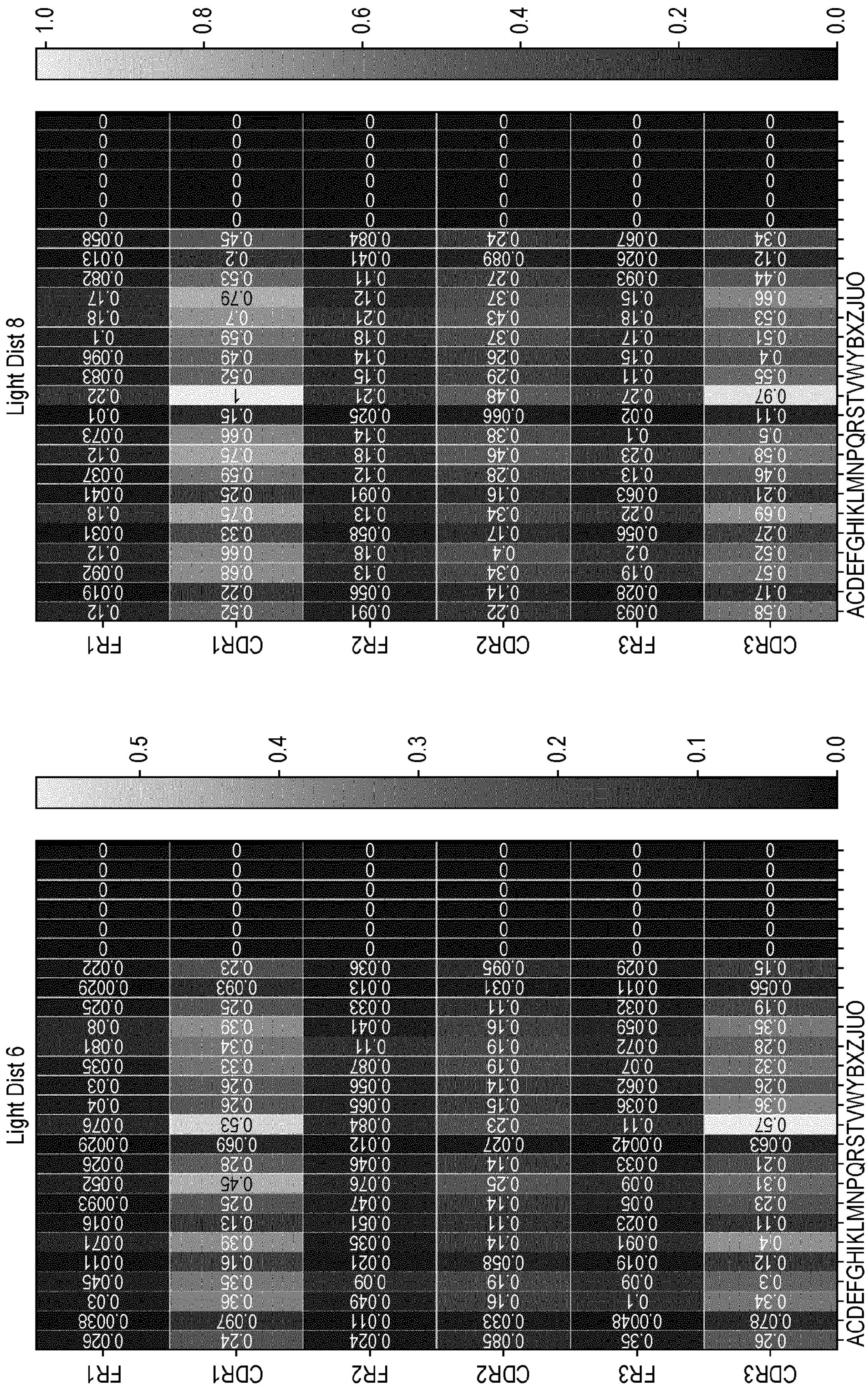


FIG. 19(a) Cont'd

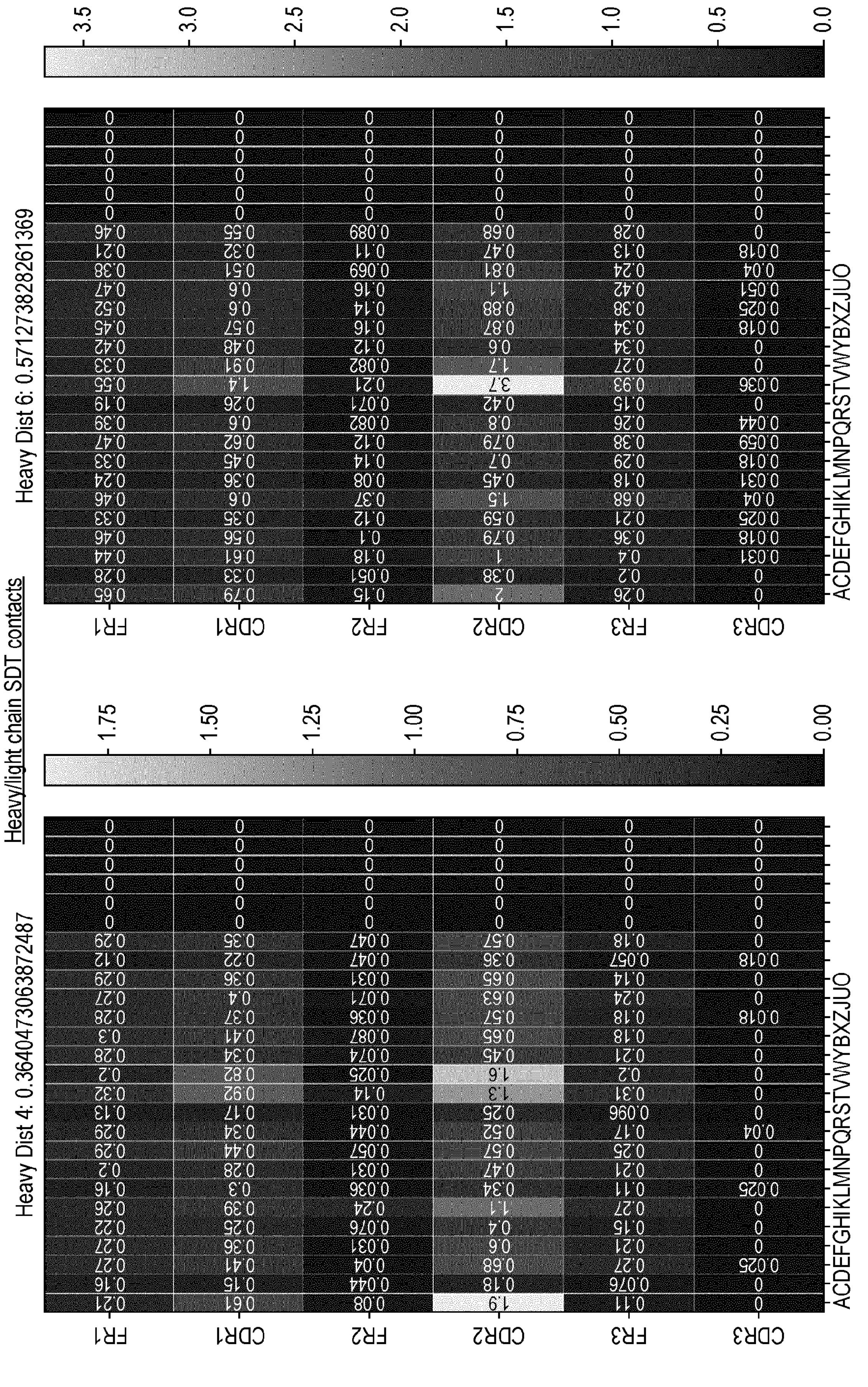
Heavy/light chain SDT contacts
Heavy Dist 4: 0.3640473063872487
Heavy Dist 6: 0.571273828261369
FR1
CDR1
FR2
CDR2
FR3
CDR3
ACDEFGHIKLMNPQRSTVWYBXZJUO
0.00 0.25 0.50 0.75 1.00 1.25 1.50 1.75
0.0 0.5 1.0 1.5 2.0 2.5 3.0 3.5

FIG. 19(b)

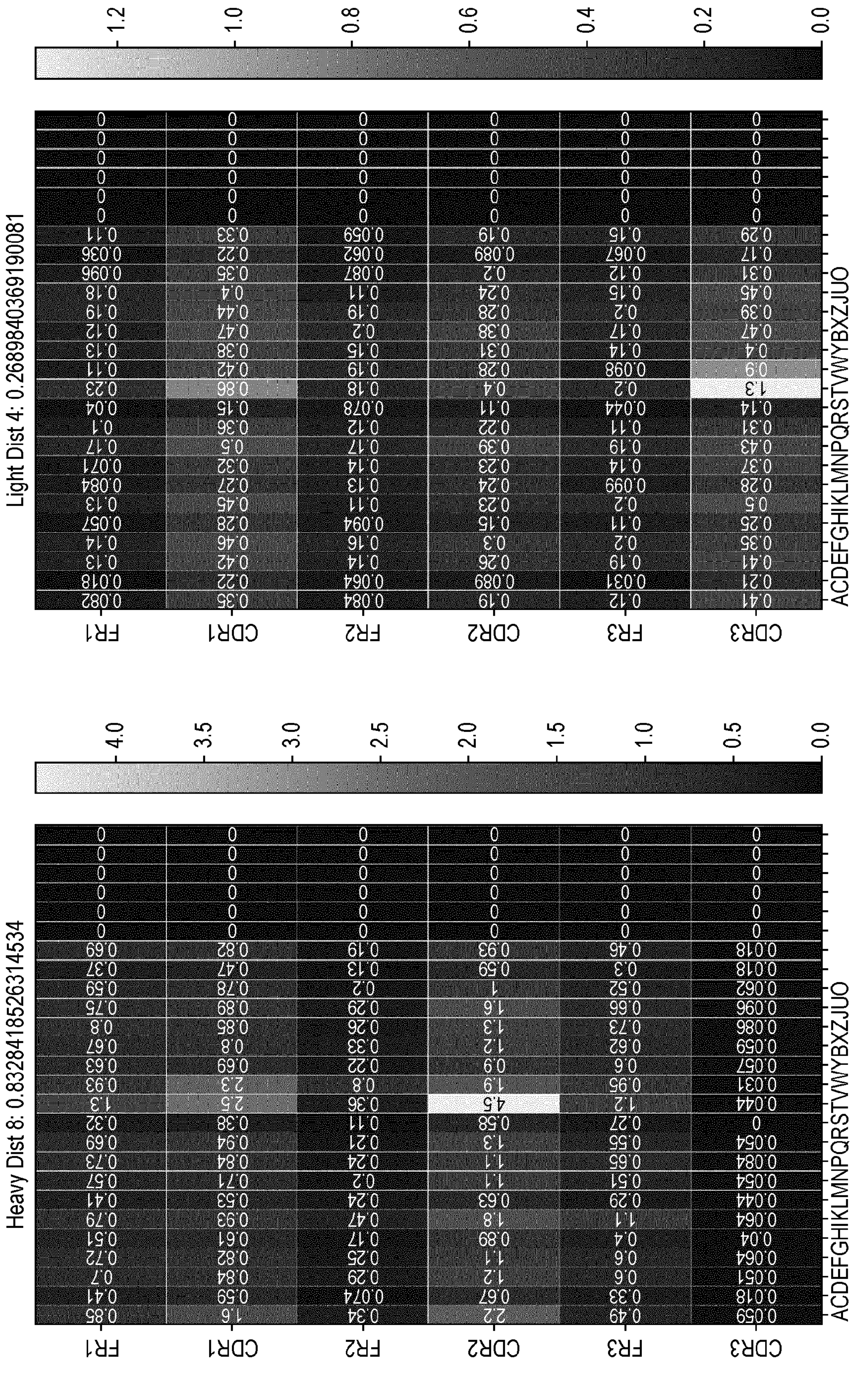


FIG. 19(b) Cont'd

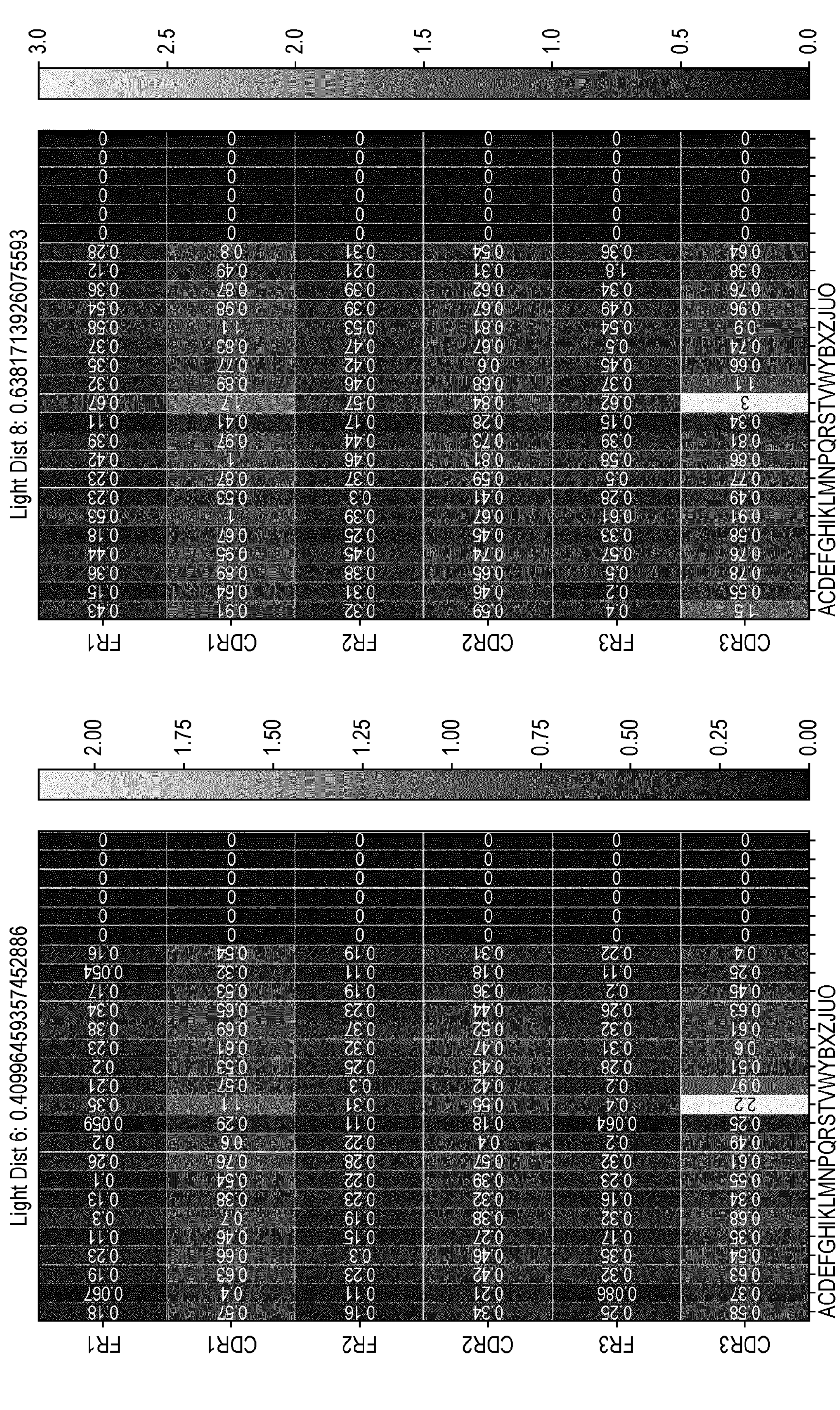


*FIG. 19(b) Cont'd*

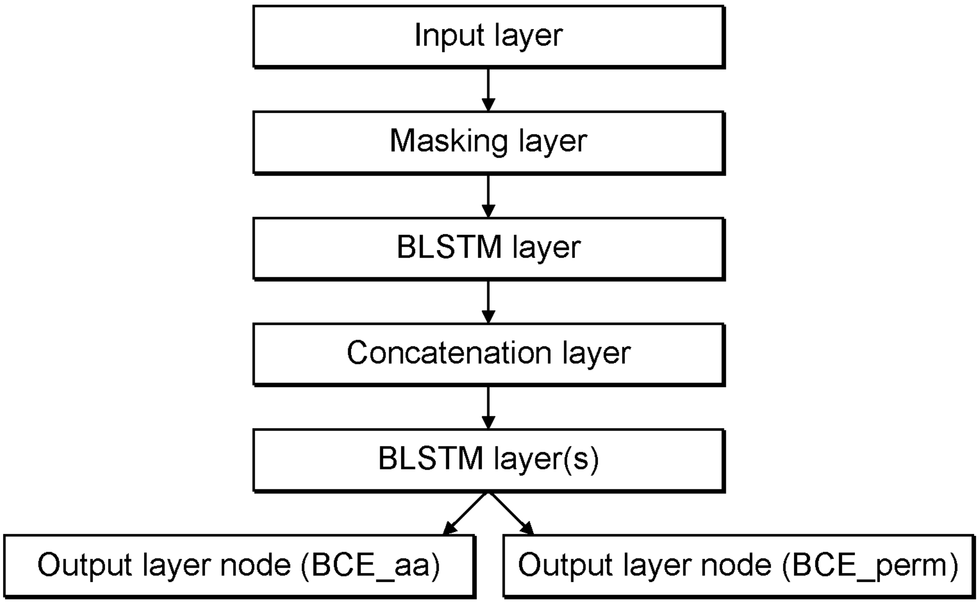


FIG. 20(a)

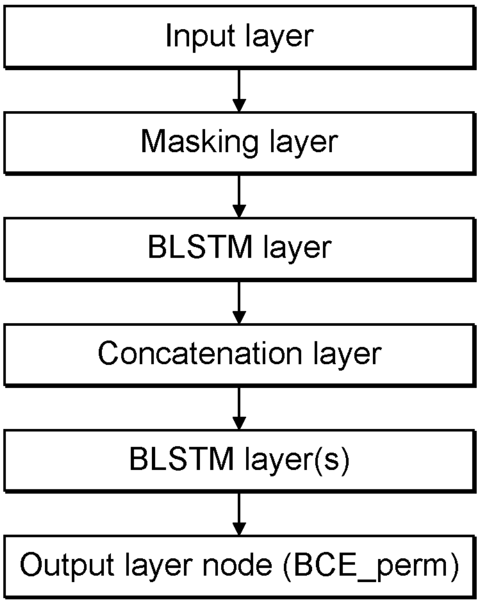


FIG. 20(b)

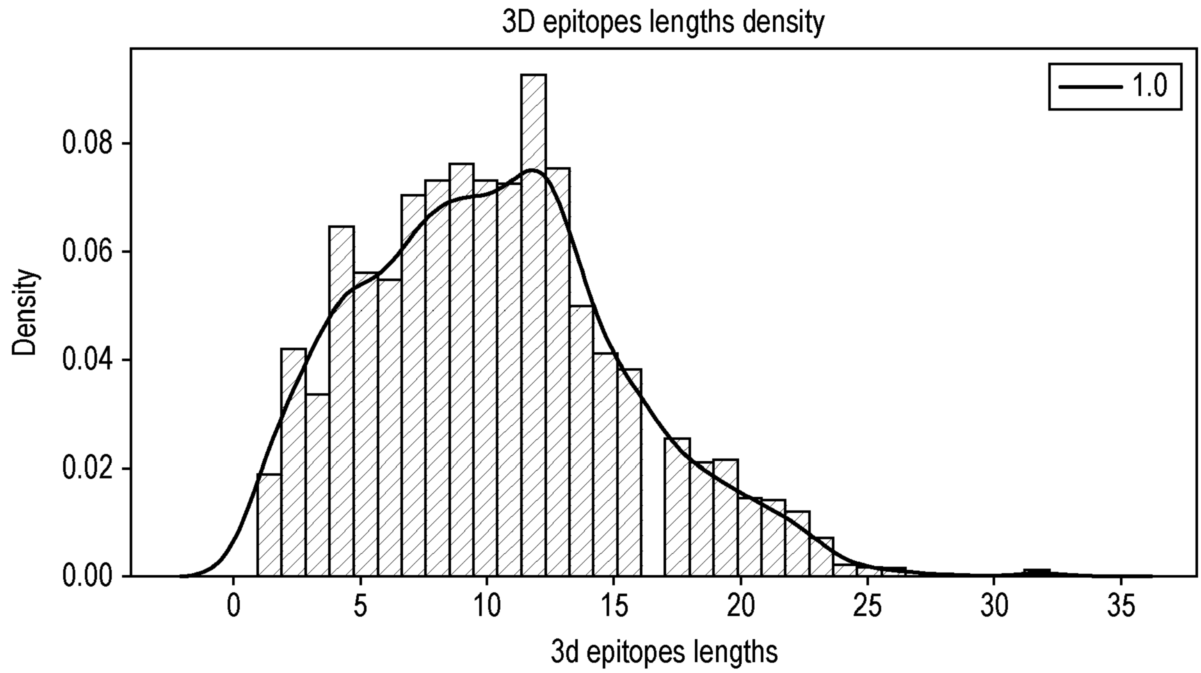


FIG. 21(a)

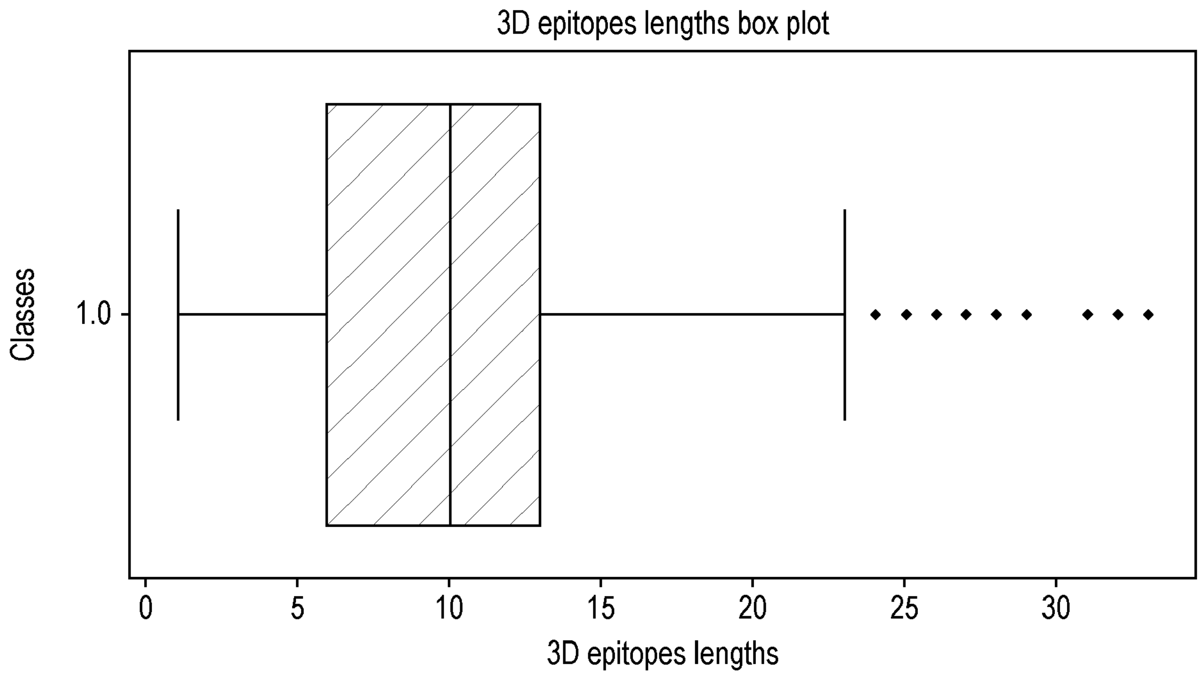


FIG. 21(b)

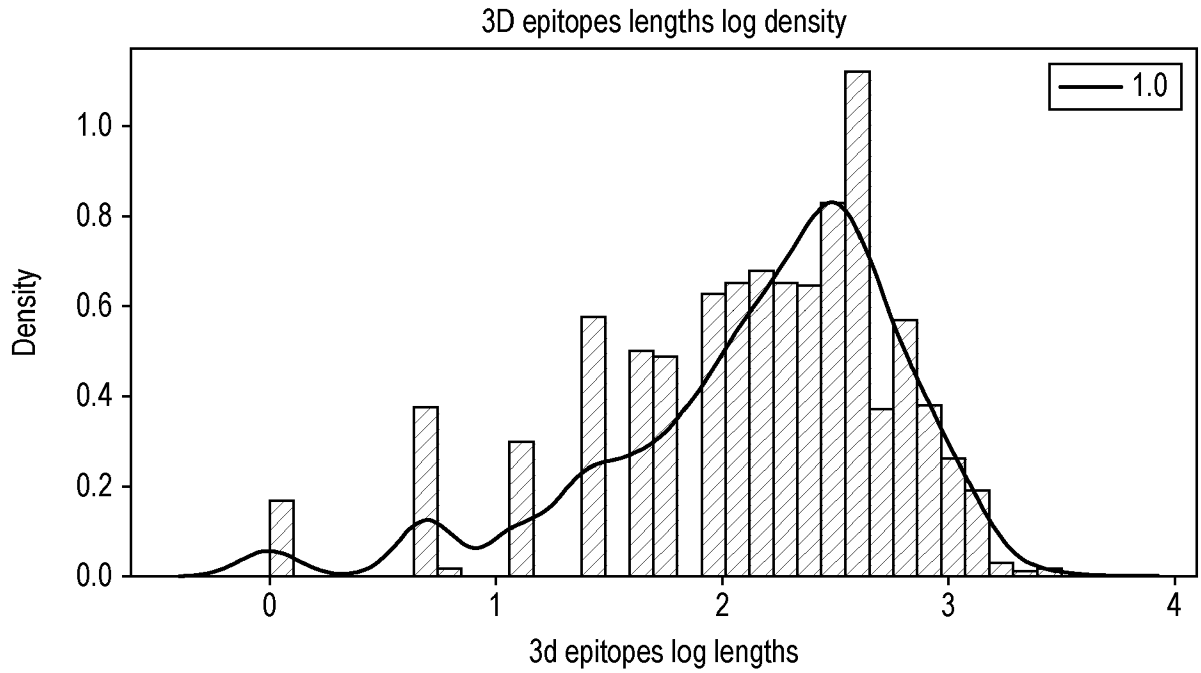


FIG. 21(c)

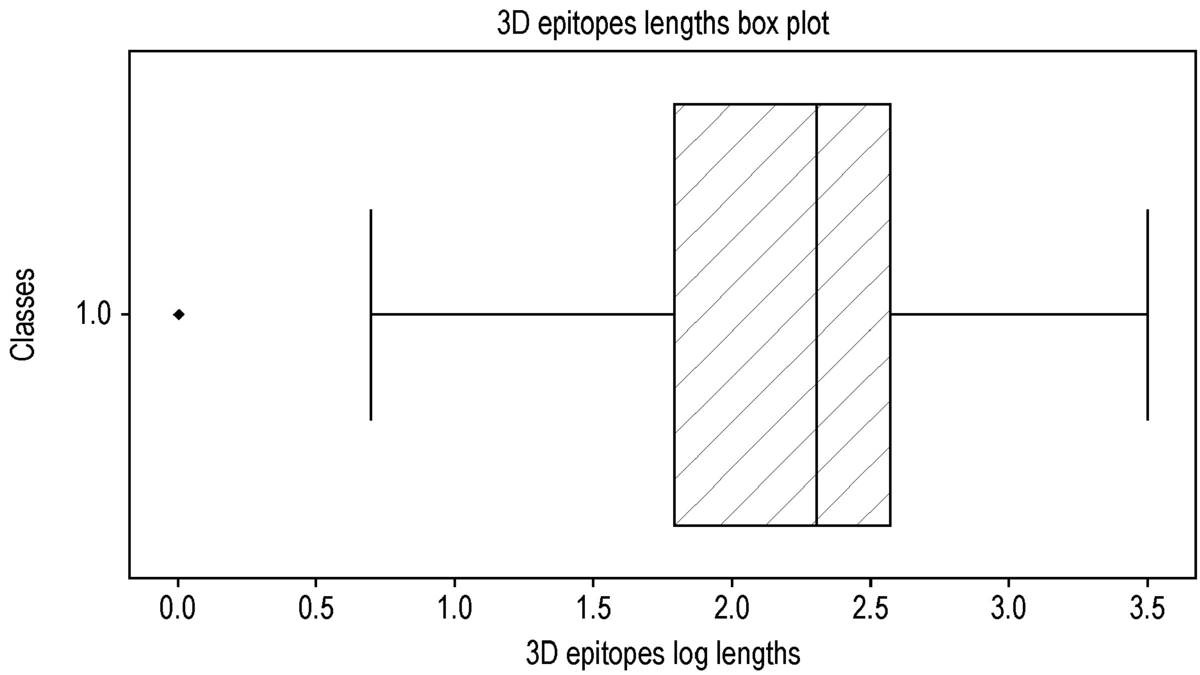


FIG. 21(d)

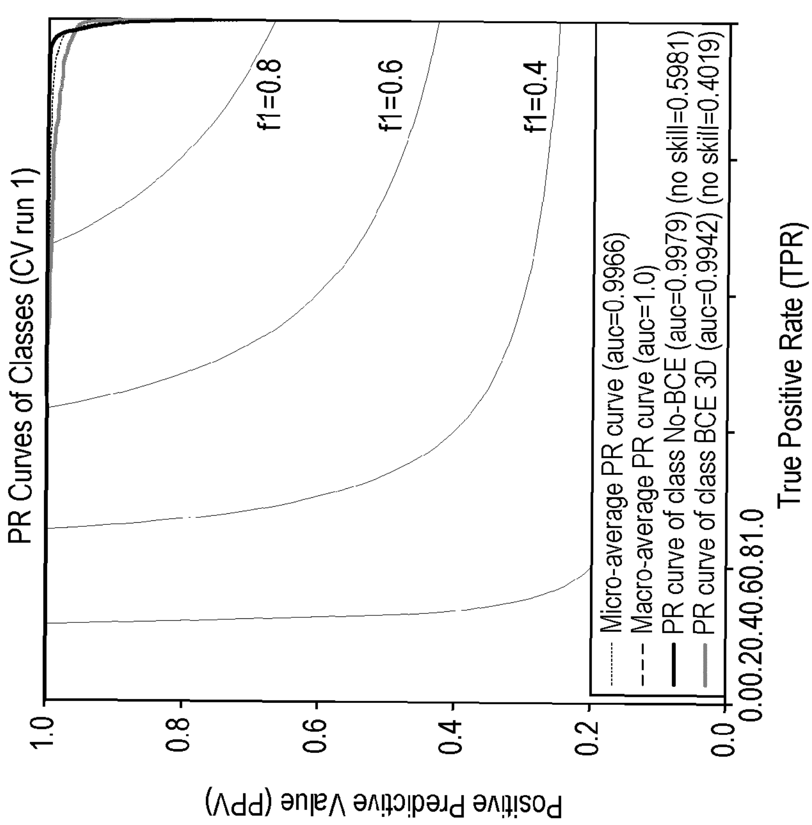


PR curve from cross-validation fold 1

*FIG. 22(a)*

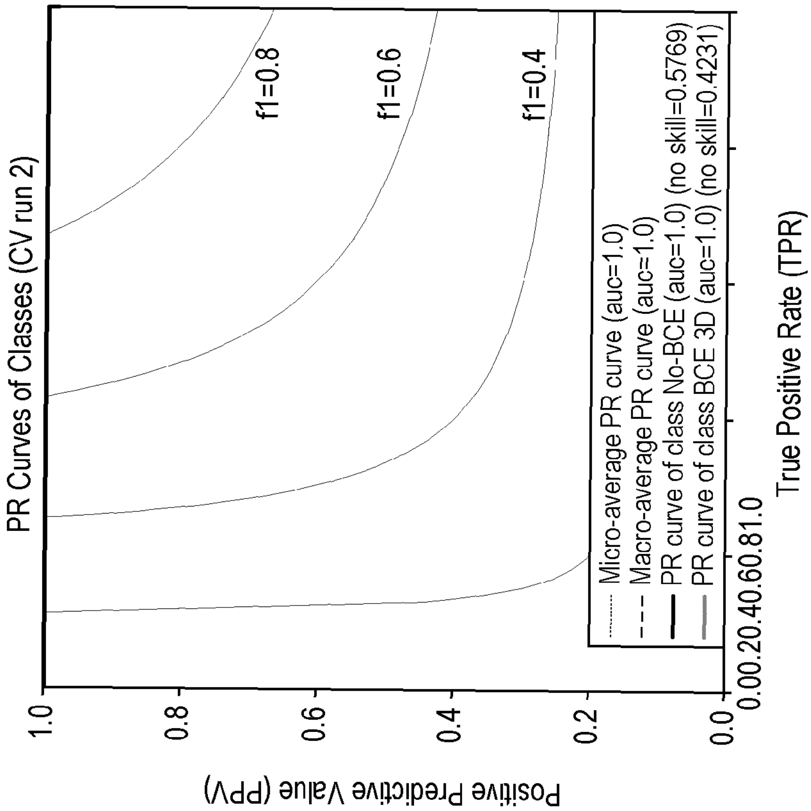


PR curve from cross-validation fold 2

*FIG. 22(b)*

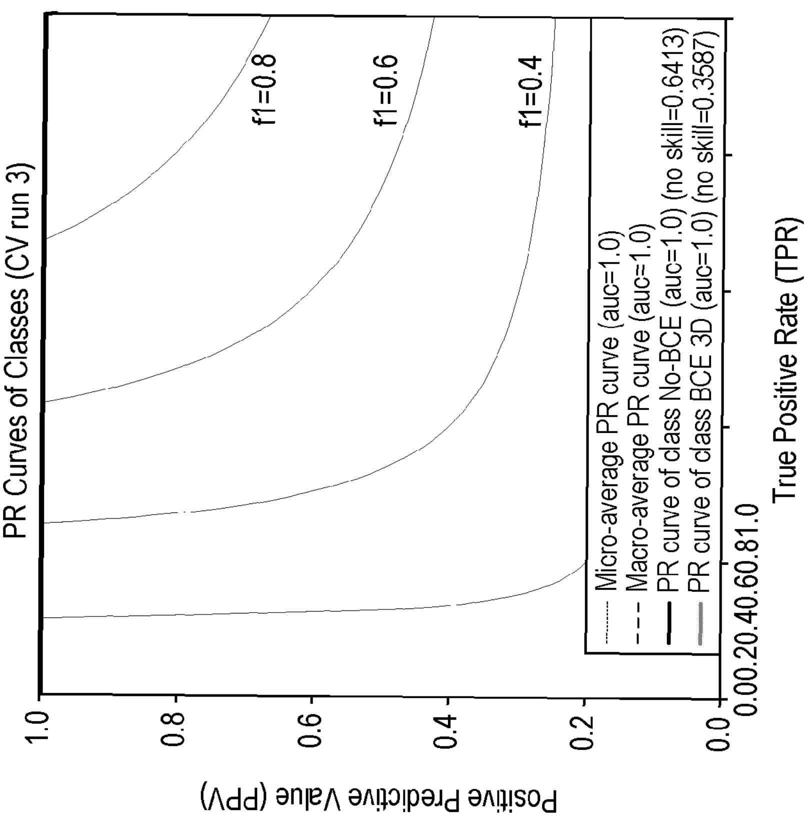


PR curve from cross-validation fold 3

*FIG. 22(c)*

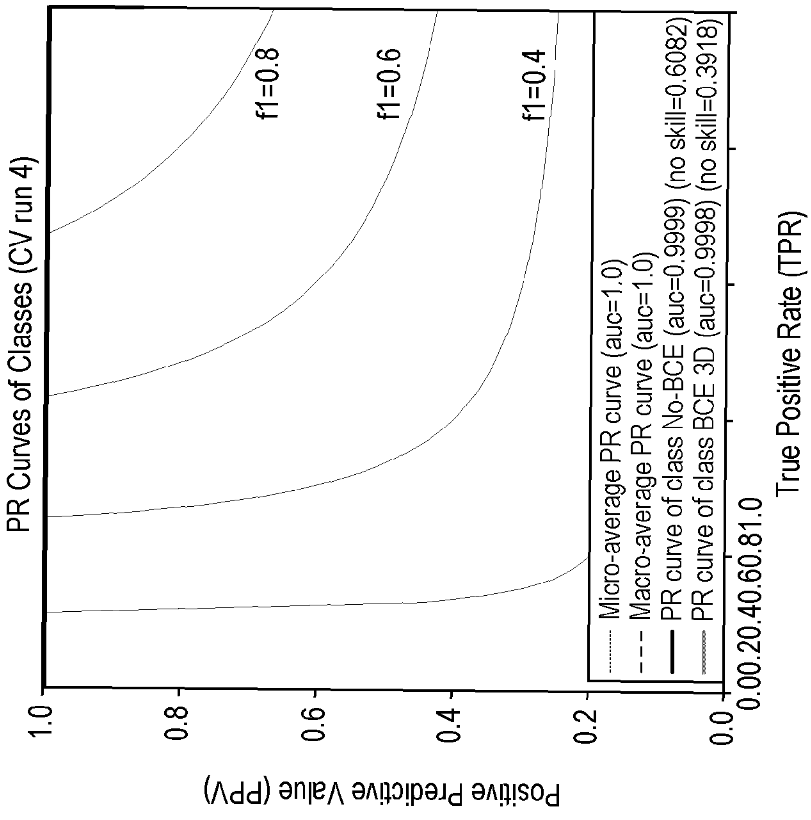


PR curve from cross-validation fold 4

*FIG. 22(d)*

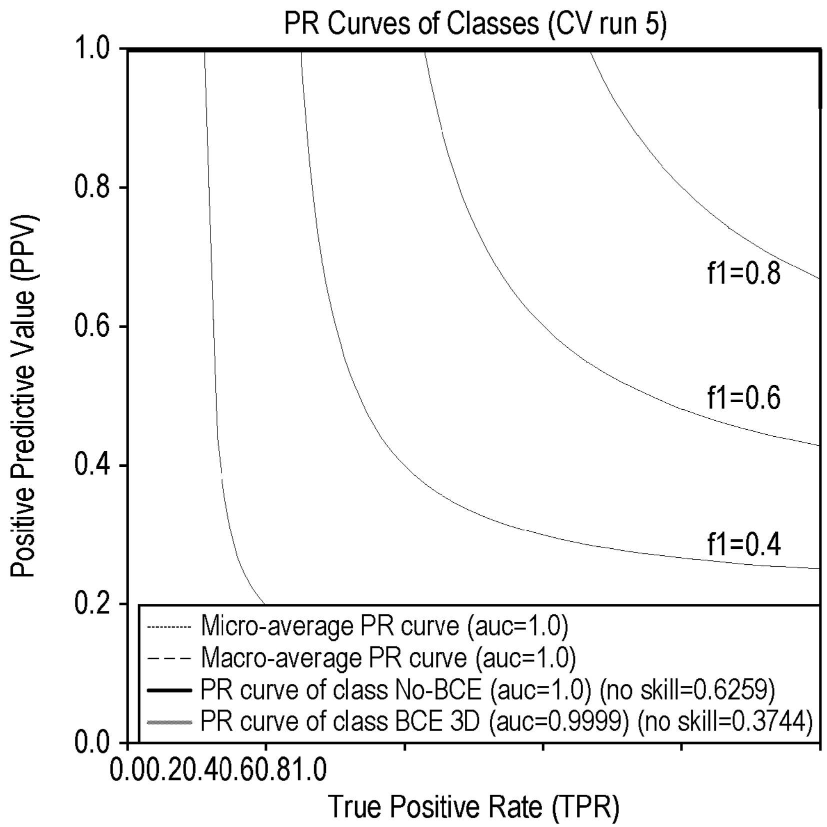

PR curve from cross-validation fold 5

FIG. 22(e)

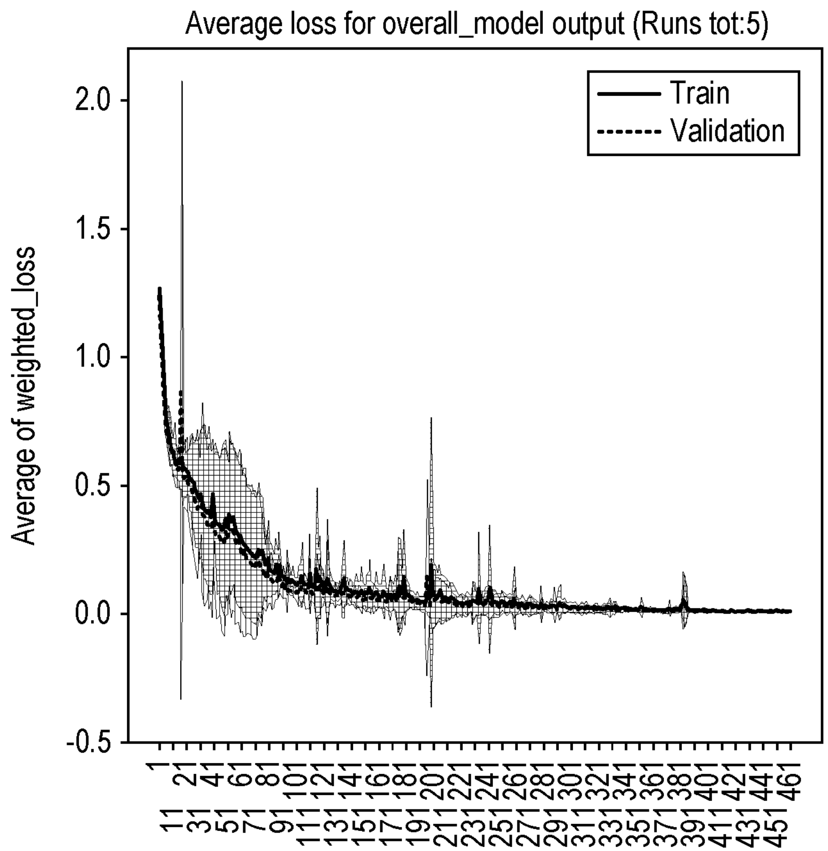

Overall model loss from all CV folds

FIG. 22(f)

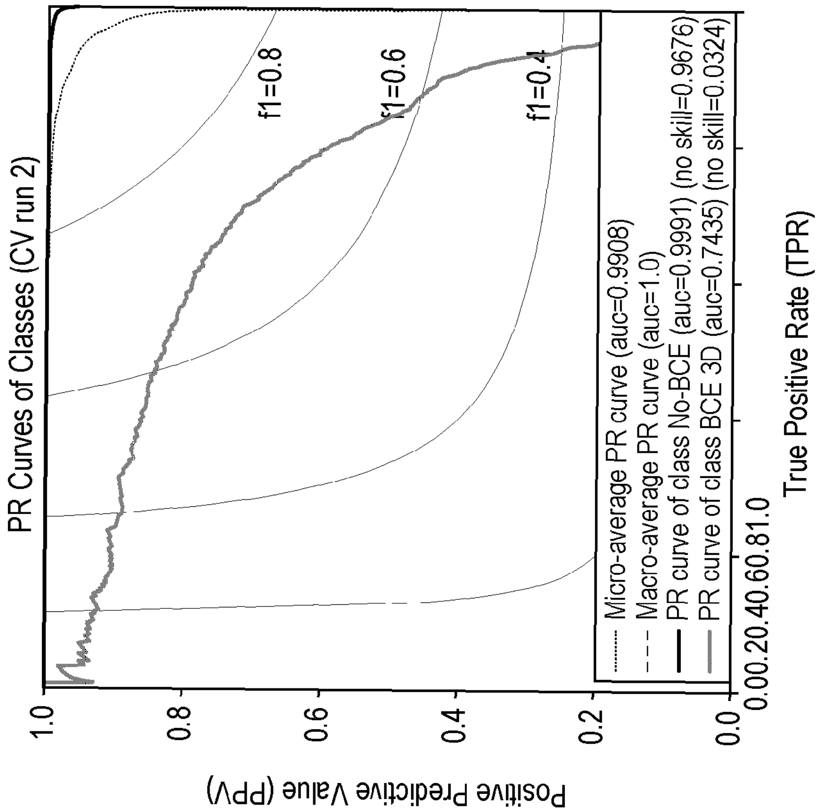


FIG. 23(b)

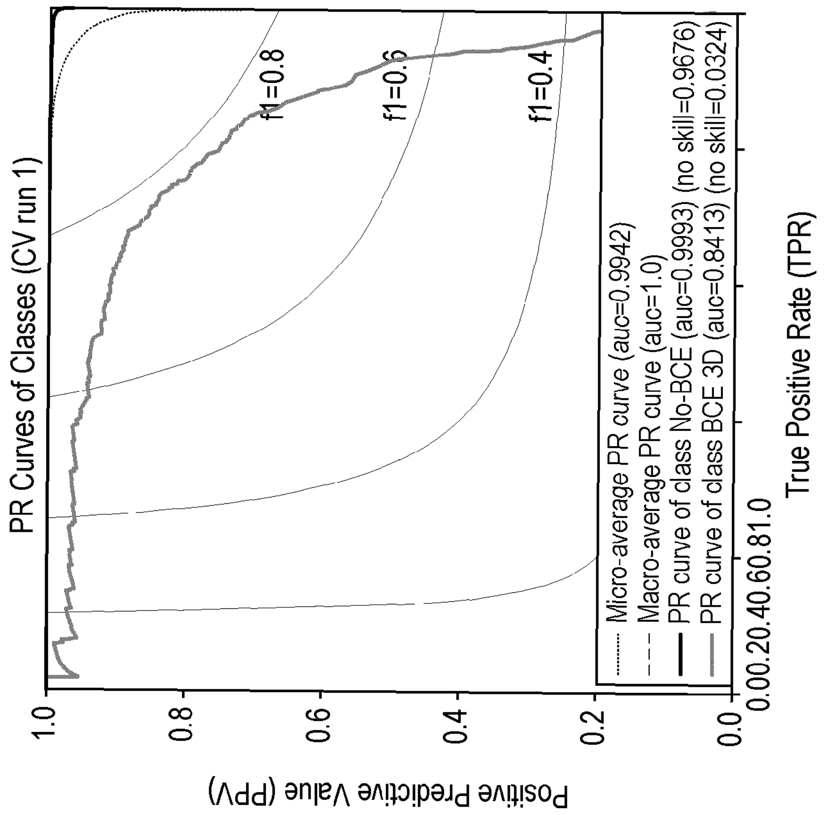


FIG. 23(a)

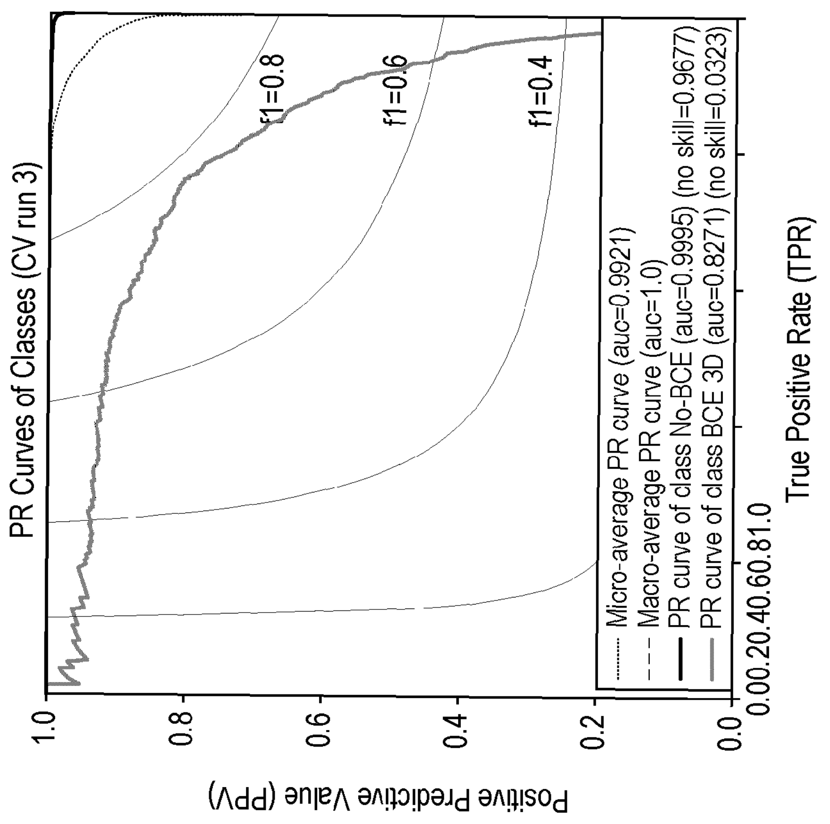


FIG. 23(c)

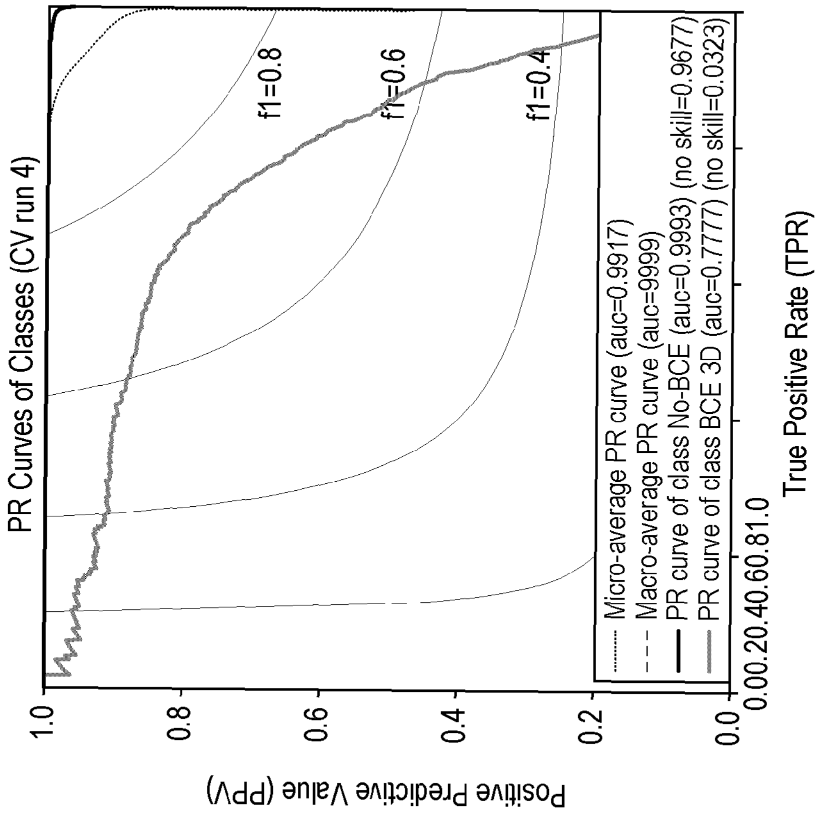


FIG. 23(d)

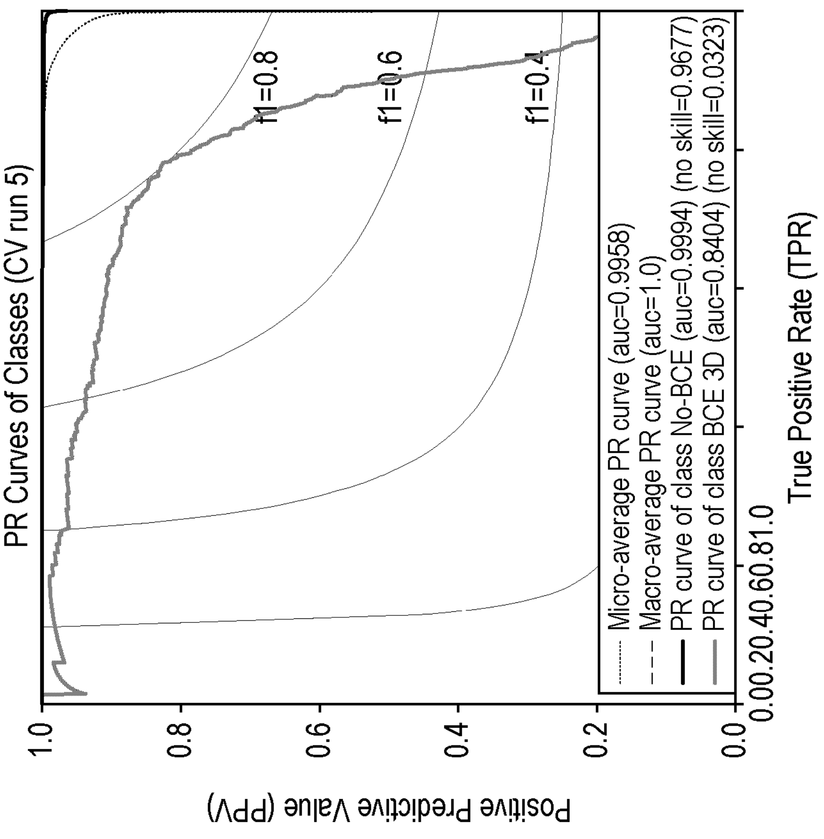


FIG. 23(e)

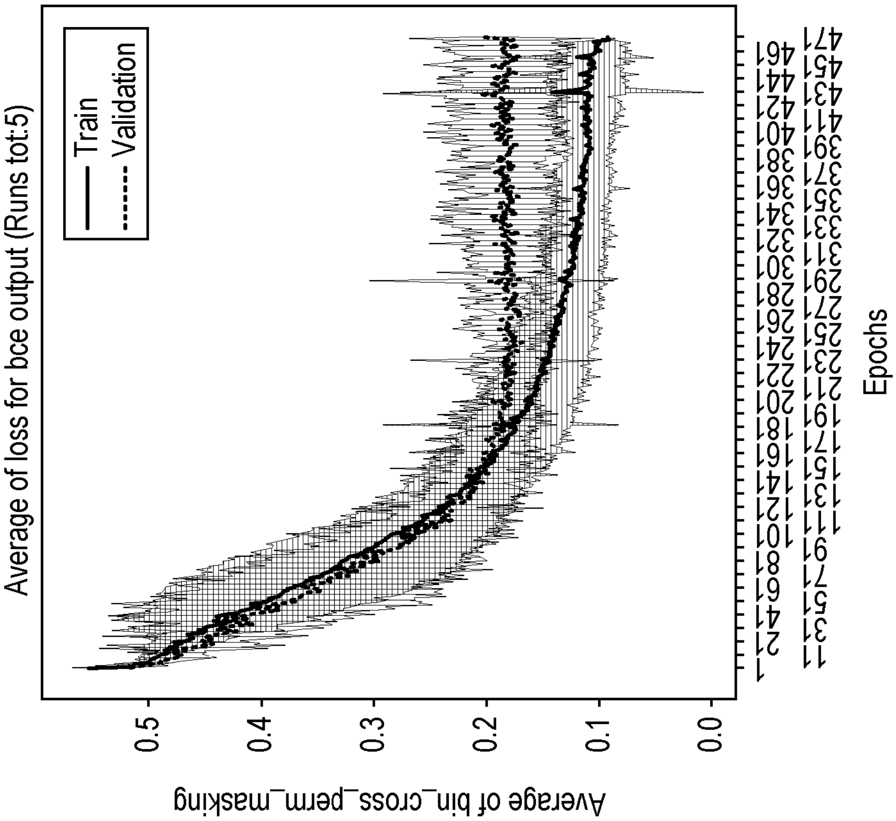


FIG. 23(f)

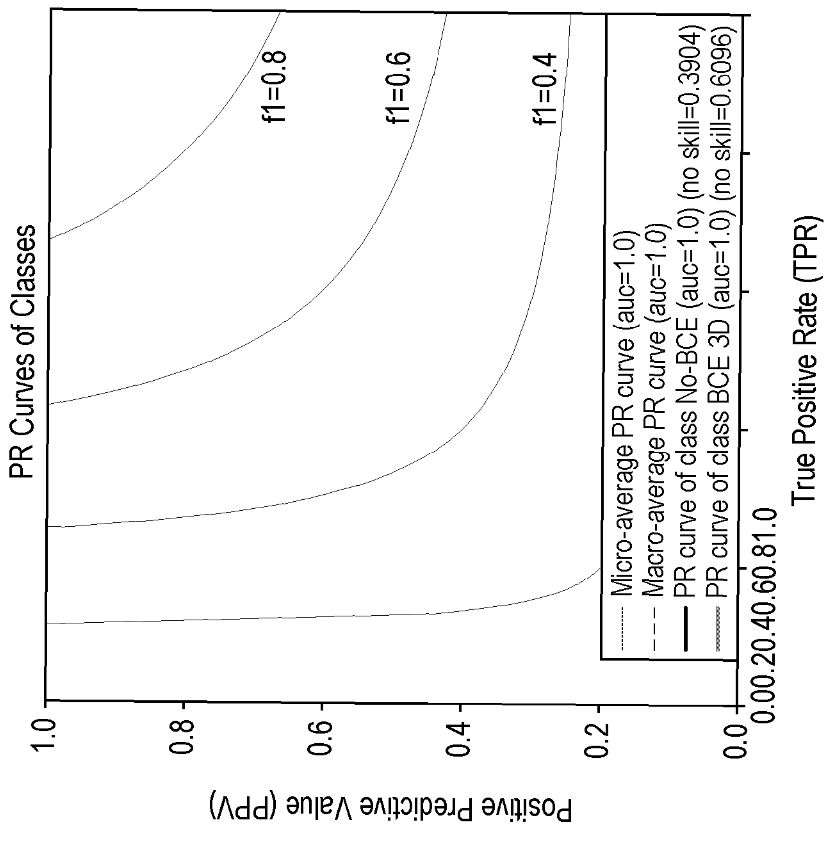


FIG. 24(a)

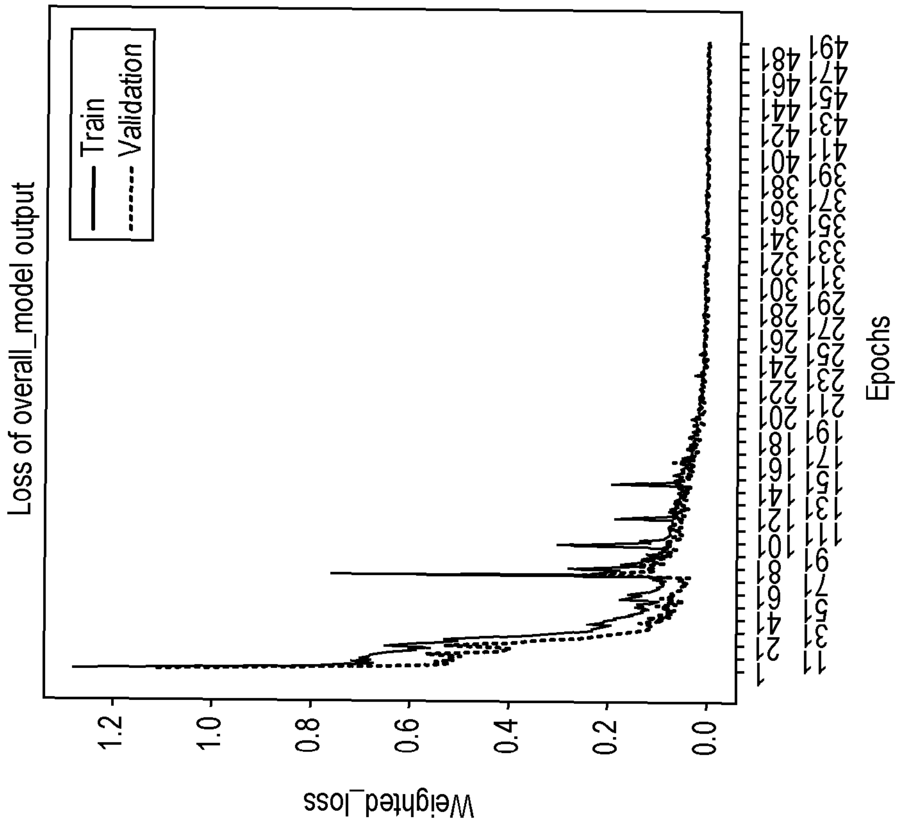


FIG. 24(b)

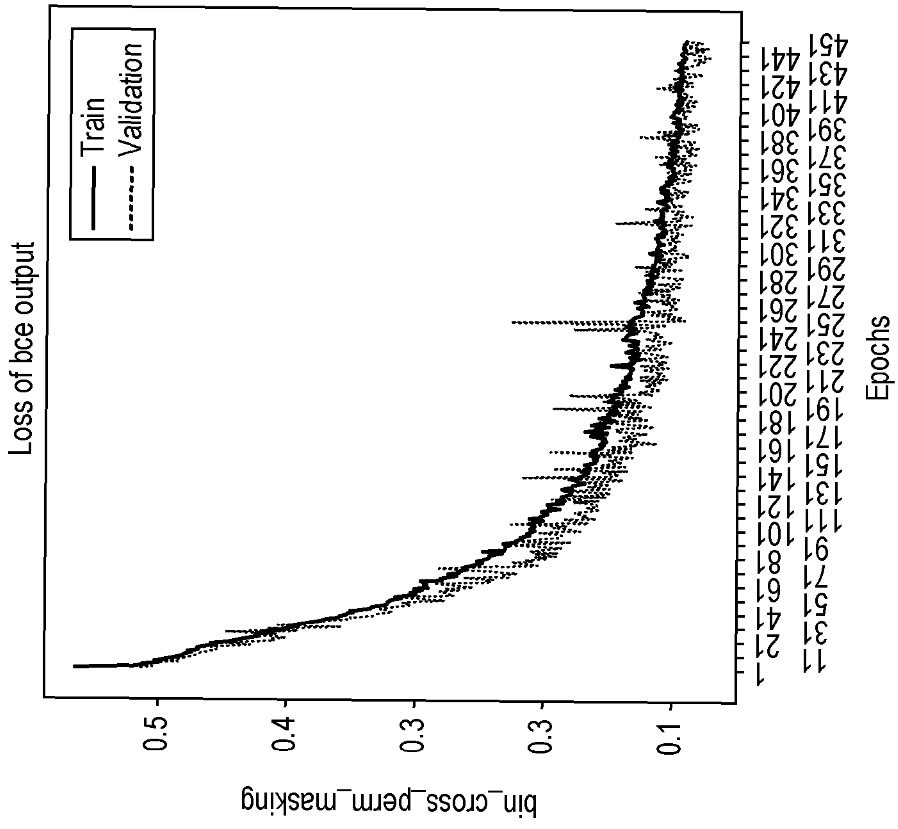


FIG. 25(b)

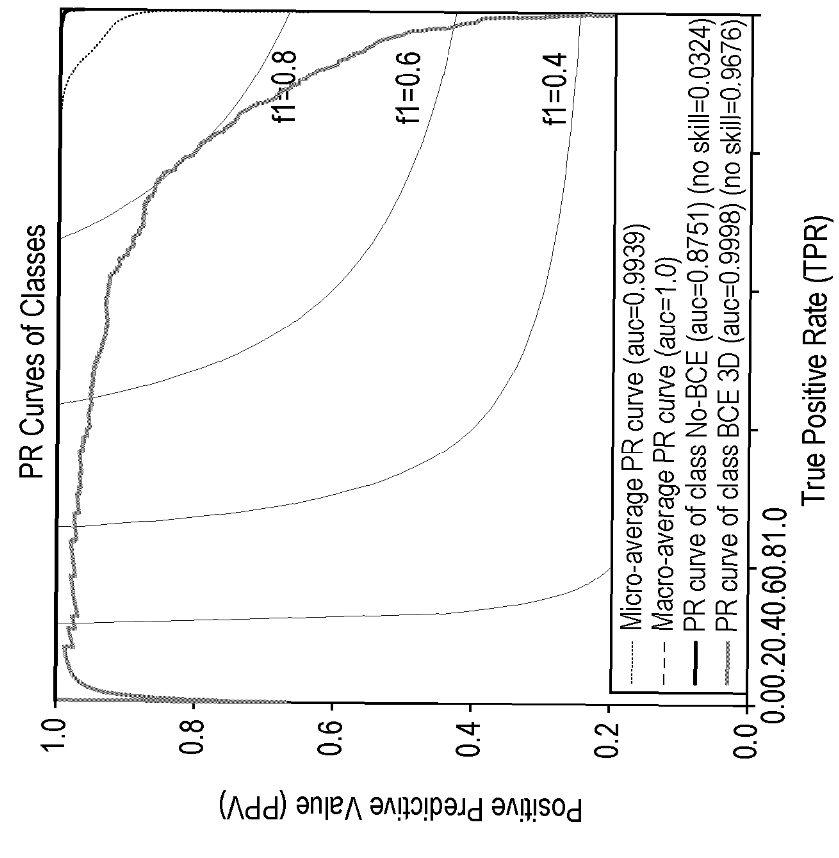


FIG. 25(a)

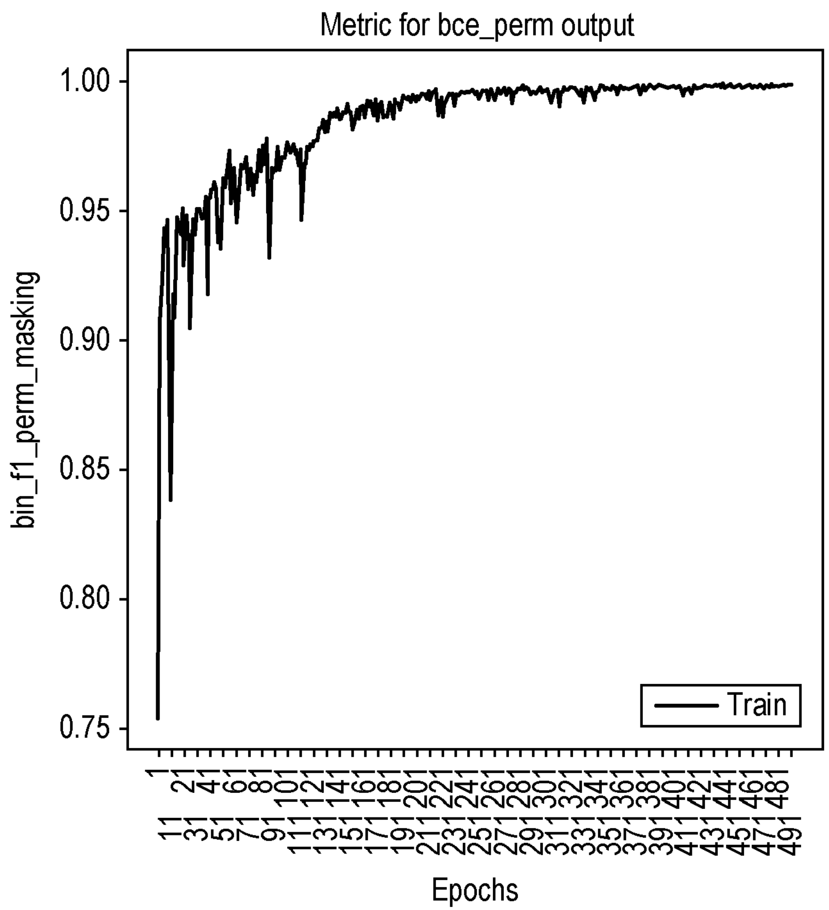


F1 curve for bce_perm for final estimates

FIG. 26(a)

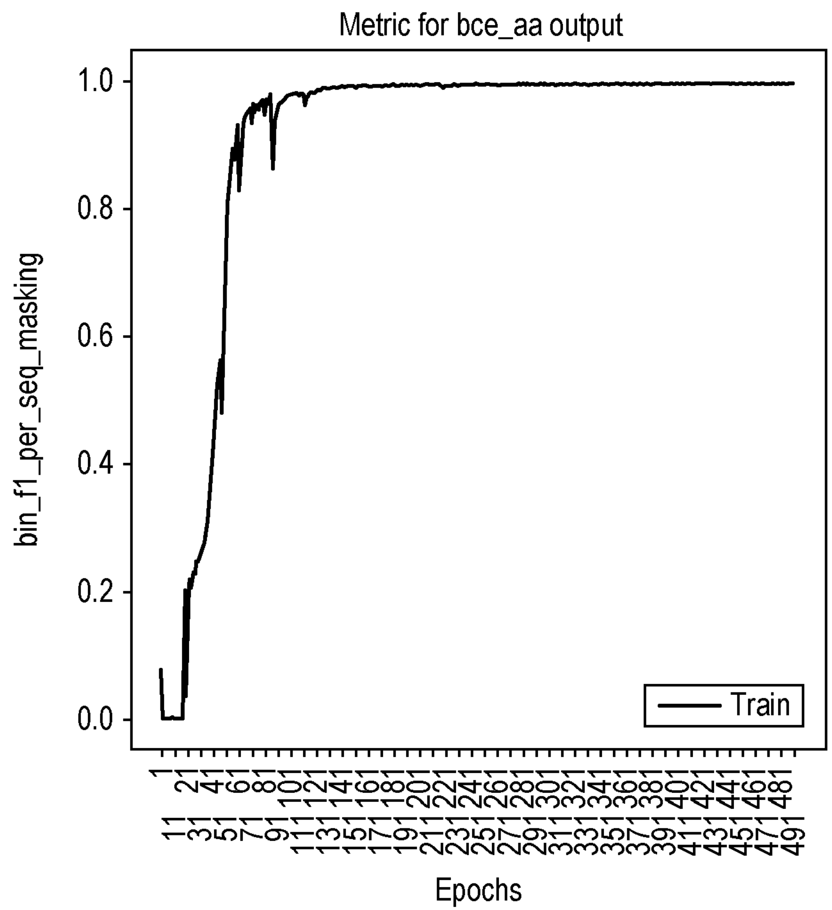


F1 curve for bce_aa for final estimates

FIG. 26(b)

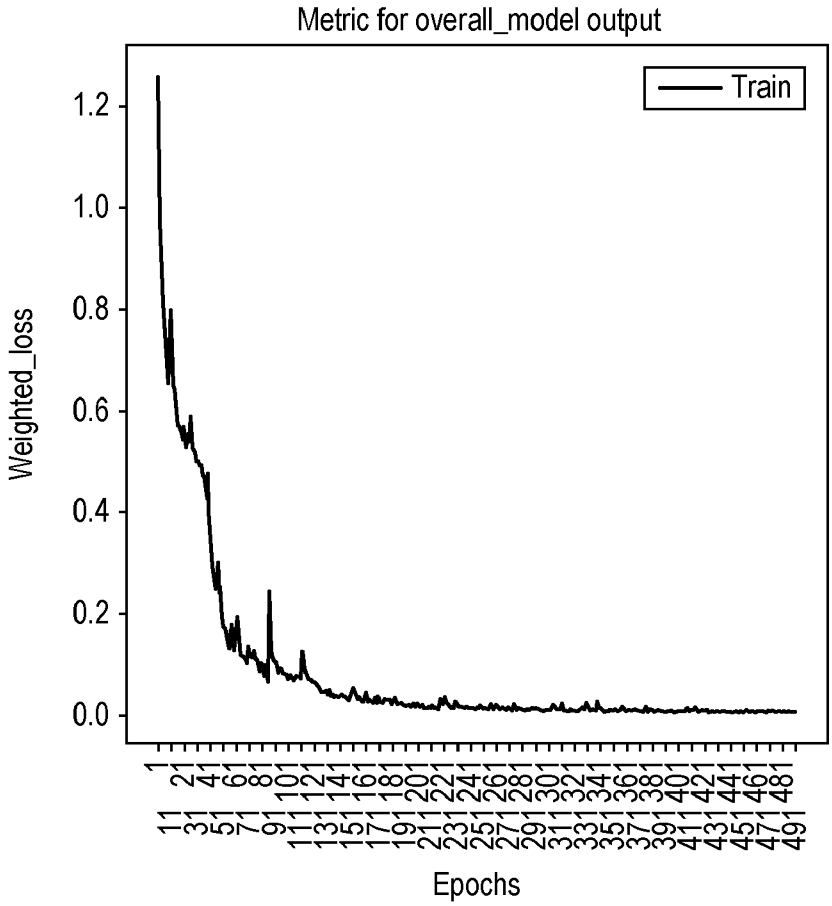


Overall model loss

FIG. 26(c)

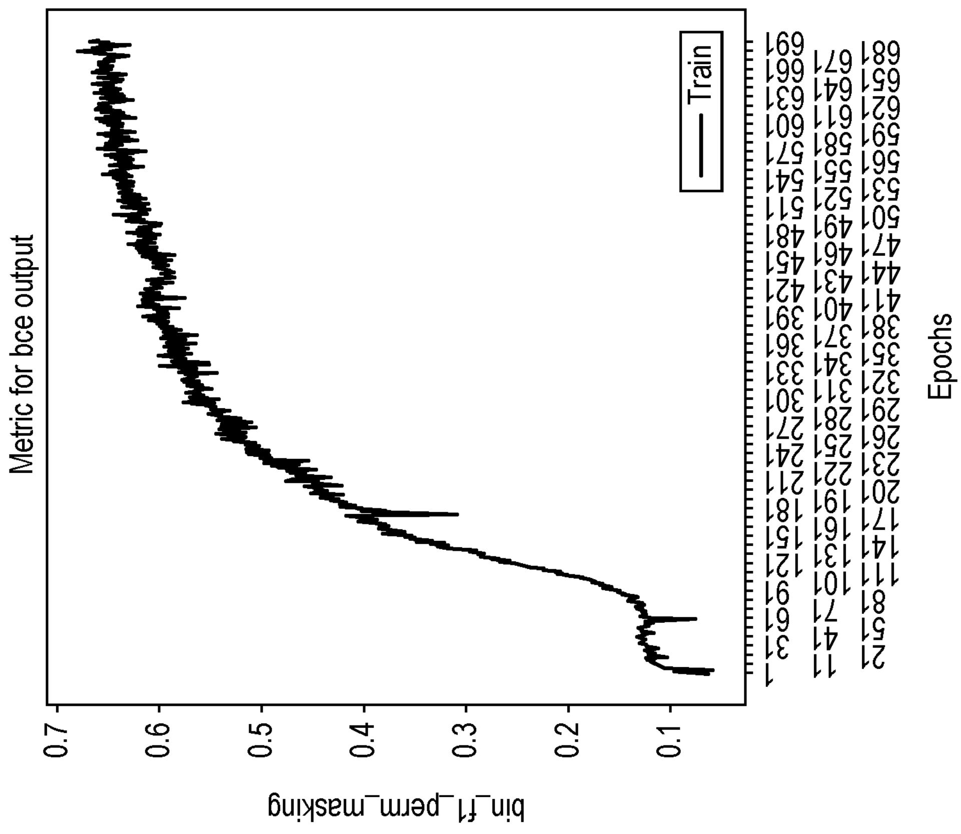


FIG. 27(a)

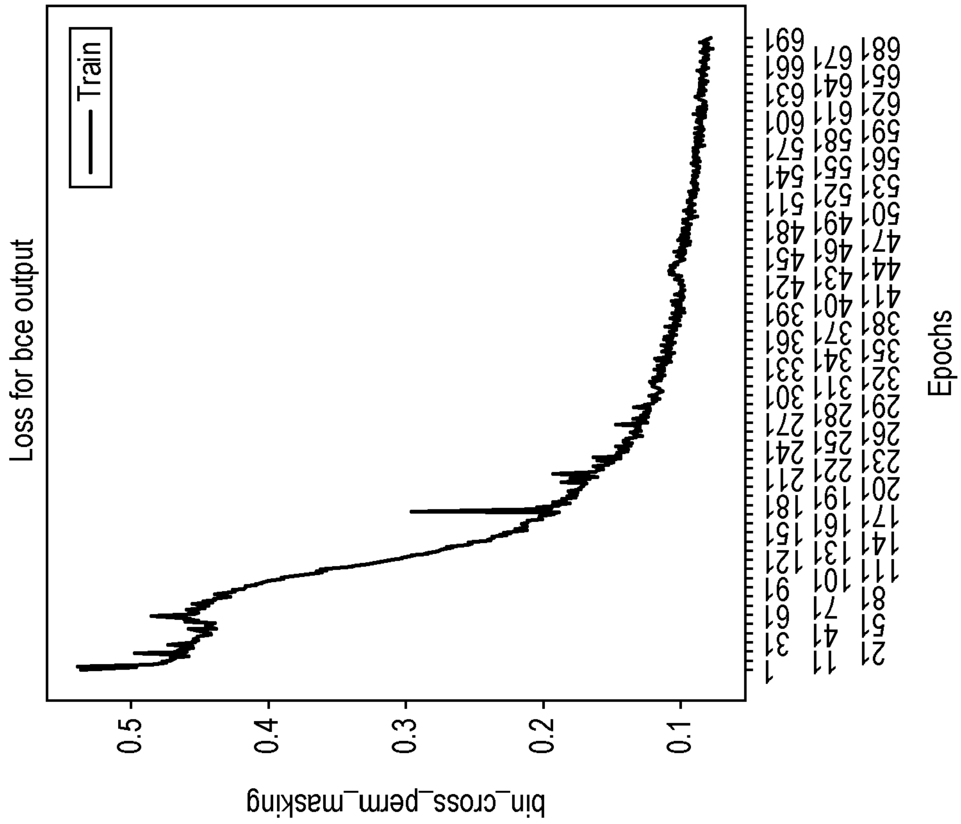


FIG. 27(b)

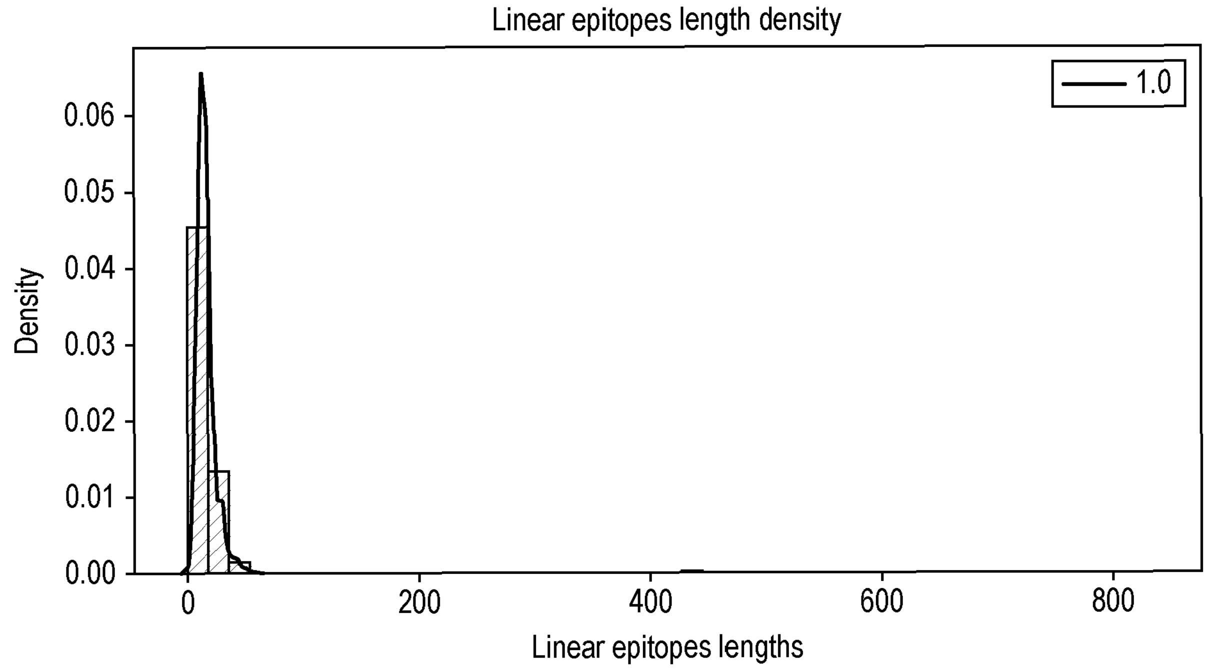


*FIG. 28(a)*

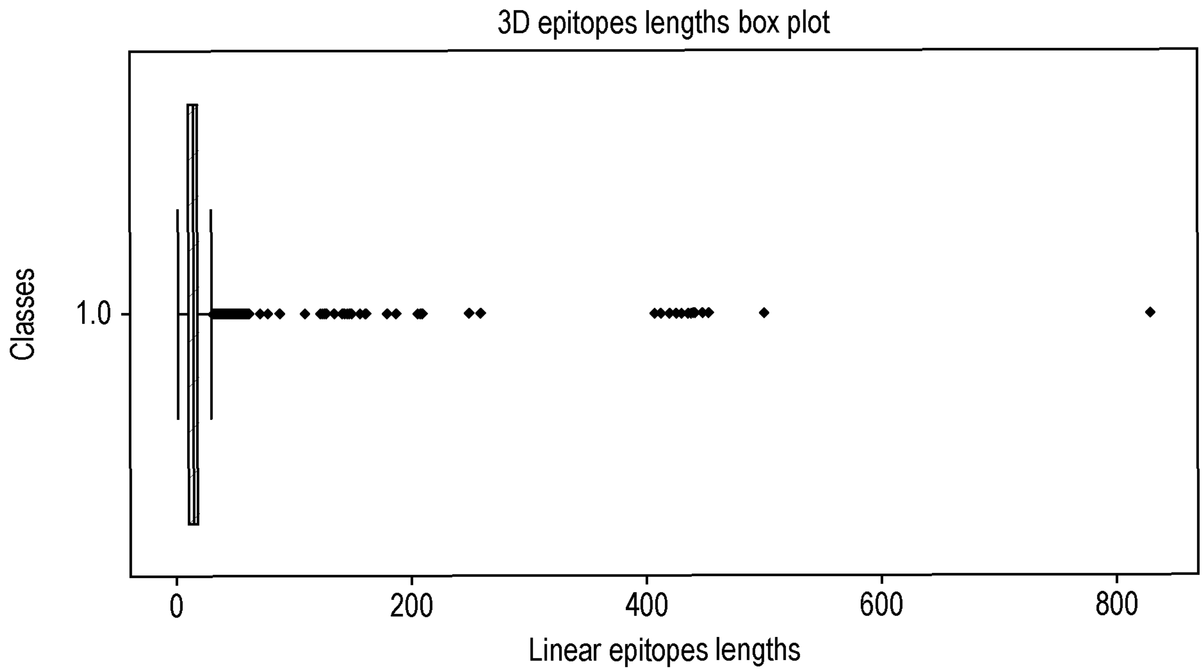


*FIG. 28(b)*

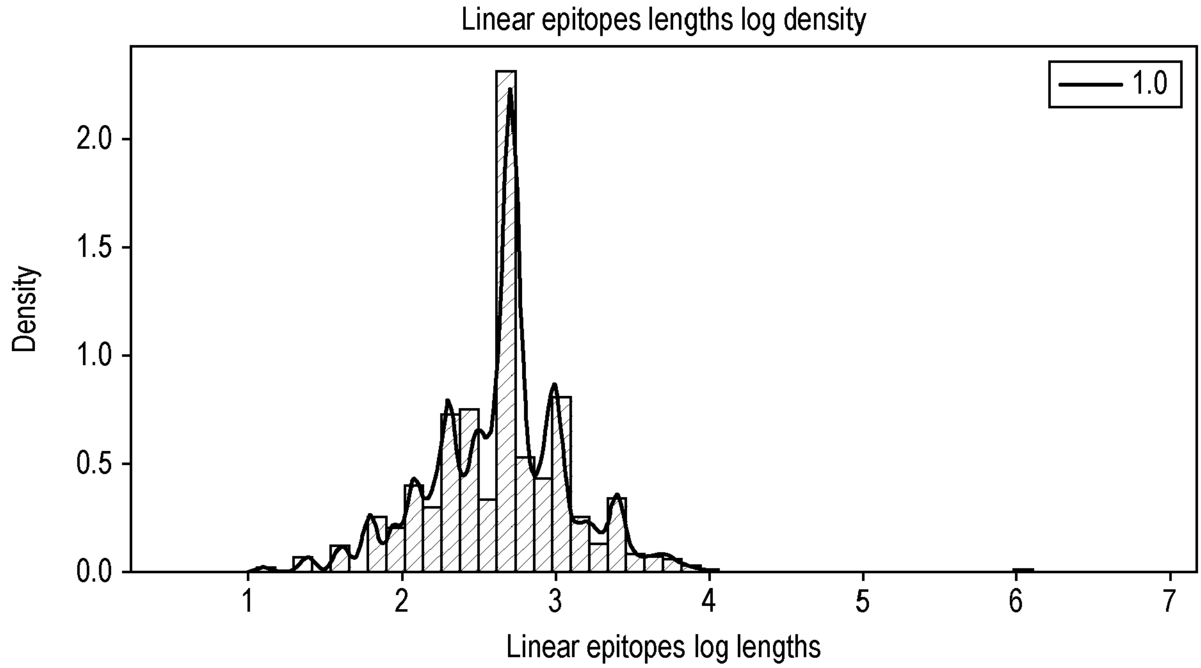


FIG. 28(c)

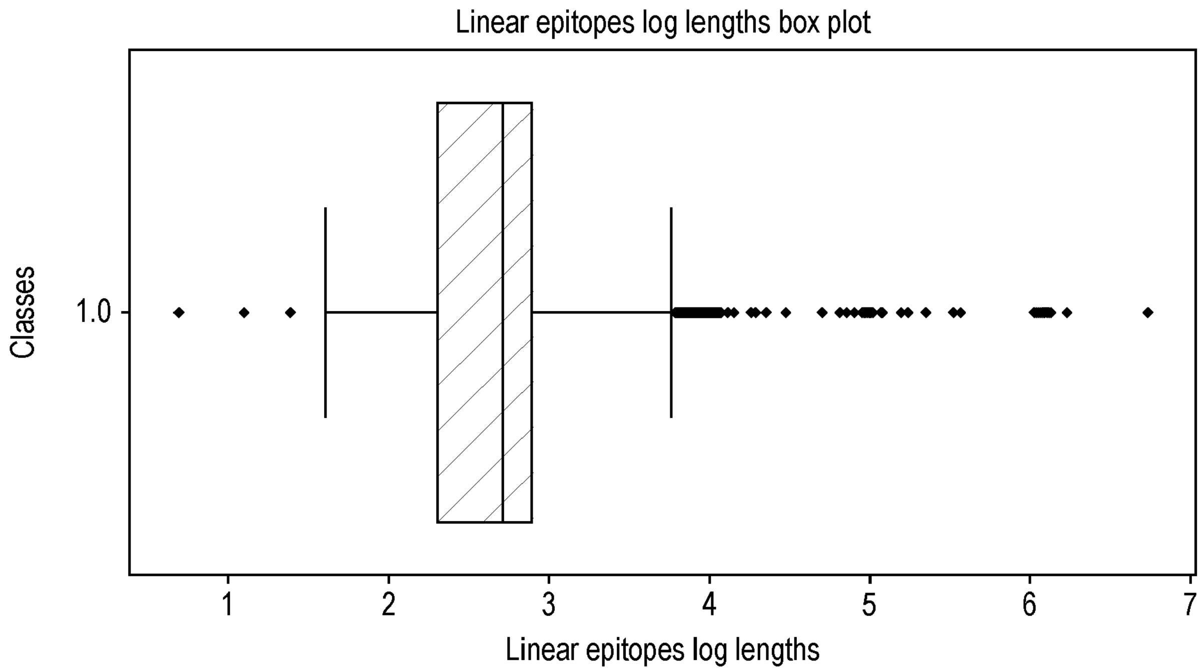


FIG. 28(d)

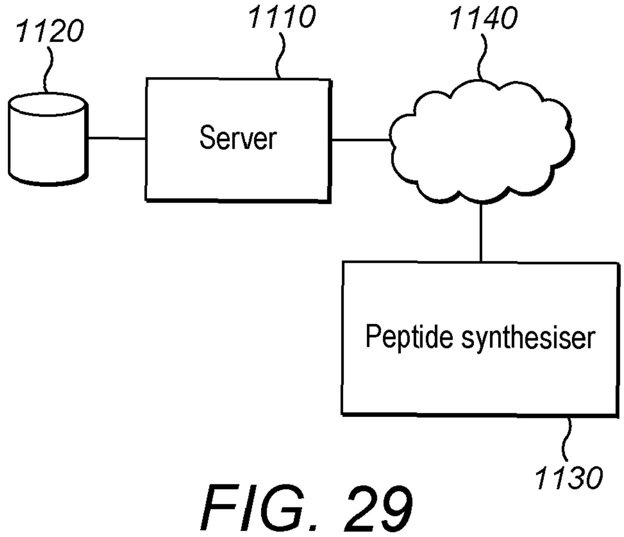
1120
1110
1140
Server
Peptide synthesiser
1130

FIG. 29

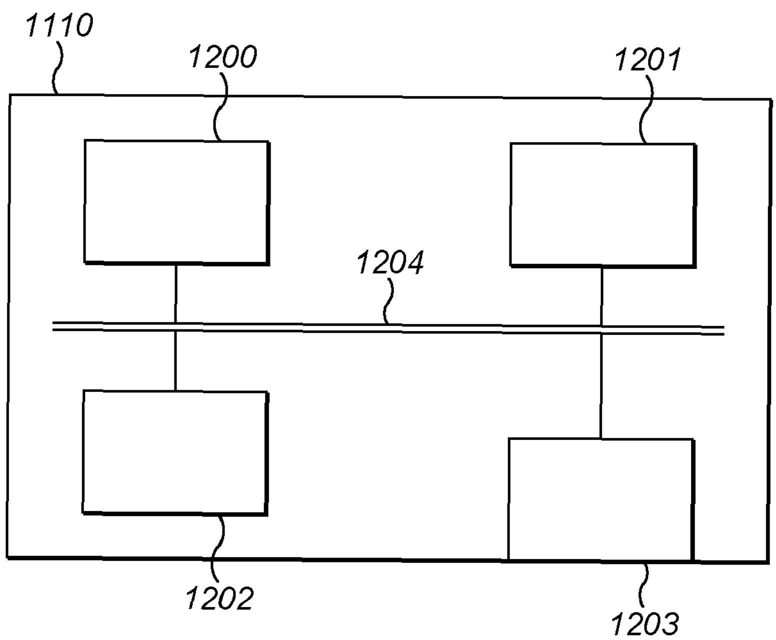
1110
1200
1201
1204
1202
1203

FIG. 30

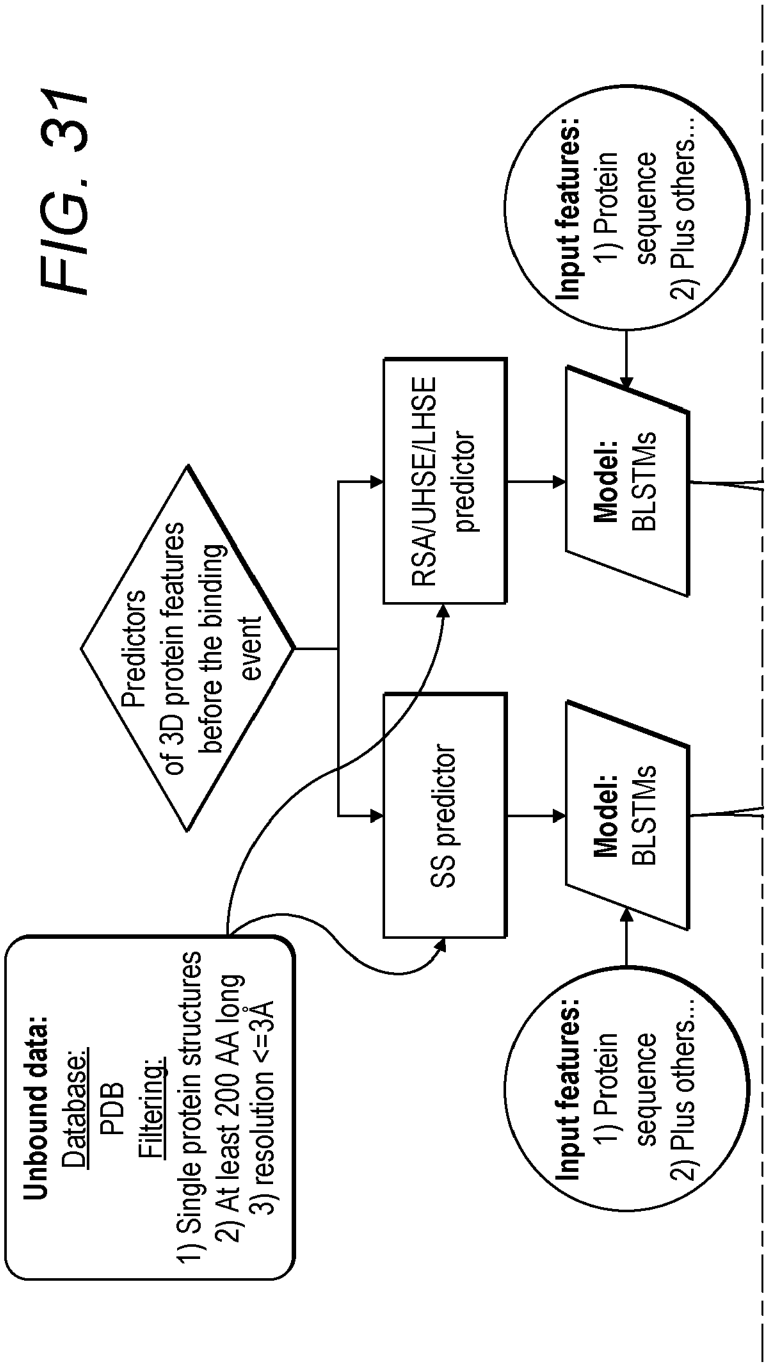
Unbound data:
Database:
PDB
Filtering:
1) Single protein structures
2) At least 200 AA long
3) resolution <=3Å
Predictors of 3D protein features before the binding event
SS predictor
RSA/UHSE/LHSE predictor
Model: BLSTMs
Model: BLSTMs
Input features:
1) Protein sequence
2) Plus others...
Input features:
1) Protein sequence
2) Plus others...

FIG. 31

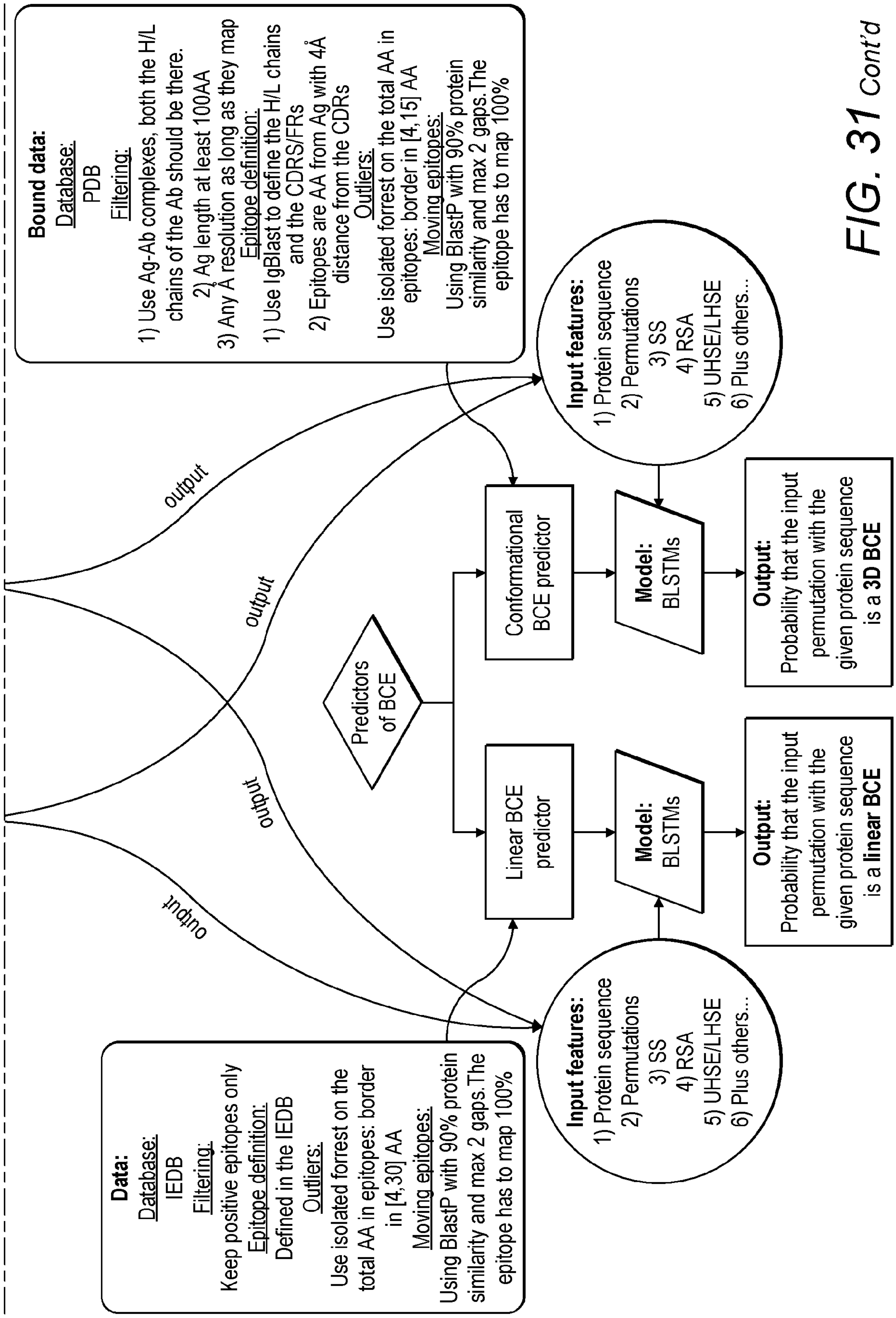
Data:
Database:
IEDB
Filtering:
Keep positive epitopes only
Epitope definition:
Defined in the IEDB
Outliers:
Use isolated forrest on the total AA in epitopes: border in [4,30] AA
Moving epitopes:
Using BlastP with 90% protein similarity and max 2 gaps.The epitope has to map 100%
Bound data:
Database:
PDB
Filtering:
1) Use Ag-Ab complexes, both the H/L chains of the Ab should be there.
2) Ag length at least 100AA
3) Any Å resolution as long as they map
Epitope definition:
1) Use IgBlast to define the H/L chains and the CDRS/FRs
2) Epitopes are AA from Ag with 4Å distance from the CDRs
Outliers:
Use isolated forrest on the total AA in epitopes: border in [4,15] AA
Moving epitopes:
Using BlastP with 90% protein similarity and max 2 gaps.The epitope has to map 100%
output
output
output
output
Predictors of BCE
Linear BCE predictor
Conformational BCE predictor
Input features:
1) Protein sequence
2) Permutations
3) SS
4) RSA
5) UHSE/LHSE
6) Plus others...
Input features:
1) Protein sequence
2) Permutations
3) SS
4) RSA
5) UHSE/LHSE
6) Plus others...
Model:
BLSTMs
Model:
BLSTMs
Output:
Probability that the input permutation with the given protein sequence is a **linear BCE**
Output:
Probability that the input permutation with the given protein sequence is a **3D BCE**

*FIG. 31 Cont'd*

Europäisches Patentamt
European Patent Office
Office européen des brevets

# EUROPEAN SEARCH REPORT

Application Number
EP 23 38 6048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/122690 A1 (LIU KAI [US] ET AL) 21 April 2022 (2022-04-21)<br>* the whole document *<br>----- | 1-15 | INV.<br>G06N3/0442<br>G06N3/08<br>G06N20/00<br>G16B15/30 |
| X | ZHANG RUOCHI ET AL: "MATCHA: Probing Multi-way Chromatin Interaction with Hypergraph Representation Learning",<br>CELL SYSTEMS,<br>vol. 10, no. 5, 1 May 2020 (2020-05-01), pages 397-407.e5, XP093014862,<br>US<br>ISSN: 2405-4712, DOI: 10.1016/j.cels.2020.04.004<br>* the whole document *<br>----- | 1-15 | |

TECHNICAL FIELDS SEARCHED (IPC)

G06F
G16B

The present search report has been drawn up for all claims

1

| Place of search | Date of completion of the search | Examiner |
|---|---|---|
| The Hague | 3 November 2023 | Hasnas, Sergiu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 38 6048

This annex lists the patent family members relating to the patent documents cited in the above-mentioned European search report. The members are as contained in the European Patent Office EDP file on
The European Patent Office is in no way liable for these particulars which are merely given for the purpose of information.

03-11-2023

| Patent document cited in search report | Publication date | Patent family member(s) | Publication date |
|---|---|---|---|
| US 2022122690 A1 | 21-04-2022 | AU 2021308081 A1 | 17-11-2022 |
| | | BR 112023000827 A2 | 07-02-2023 |
| | | CA 3180799 A1 | 20-01-2022 |
| | | CN 115997254 A | 21-04-2023 |
| | | EP 4182924 A1 | 24-05-2023 |
| | | IL 299801 A | 01-03-2023 |
| | | JP 2023534283 A | 08-08-2023 |
| | | KR 20230042048 A | 27-03-2023 |
| | | US 2022122690 A1 | 21-04-2022 |
| | | WO 2022016125 A1 | 20-01-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WOLFGANG KABSCH** ; **CHRISTIAN SANDER**. Dictionary of protein secondary structure: Pattern recognition of hydrogen-bonded and geometrical features. *Biopolymers*, December 1983, vol. 22 (12), 2577-2637
- **PETER JA COCK** ; **TIAGO ANTAO** ; **JEFFREY T CHANG** ; **BRAD A CHAPMAN** ; **CYMON J COX** ; **ANDREW DALKE** ; **IDDO FRIEDBERG** ; **THOMAS HAMELRYCK** ; **FRANK KAUFF** ; **BARTEK WILCZYNSKI et al.** Biopython: freely available python tools for computational molecular biology and bioinformatics. *Bioinformatics*, 2009, vol. 25 (11), 1422-1423
- **JIAN YE** ; **NING MA** ; **THOMAS L MADDEN** ; **JAMES M OSTELL**. Igblast: an immunoglobulin variable domain sequence analysis tool. *Nucleic Acids Res*, July 2013, vol. 41, W34-40
- **G JOHNSON** ; **T T WU**. Kabat database and its applications: 30 years after the first variability plot. *Nucleic acids research*, January 2000, vol. 28 (1), 214-218
- **F. PEDREGOSA** ; **G. VAROQUAUX** ; **A. GRAMFORT** ; **V. MICHEL** ; **B. THIRION** ; **O. GRISEL** ; **M. BLONDEL** ; **P. PRETTENHOFER** ; **R. WEISS** ; **V. DUBOURG**. Scikit-learn: Machine learning in Python. *Journal of Machine Learning Research*, 2011, vol. 12, 2825-2830
- **T P HOPP** ; **K R WOODS**. Prediction of protein antigenic determinants from amino acid sequences. *Proceedings of the National Academy of Sciences of the United States of America*, June 1981, vol. 78 (6), 3824-3828
- **R. GRANTHAM**. Amino acid difference formula to help explain protein evolution. *Science*, September 1974, vol. 185 (4154), 862
- **JOEL JANIN**. Surface and inside volumes in globular proteins. *Nature*, February 1979, vol. 277, 491
- **H R GUY**. Amino acid side-chain partition energies and distribution of residues in soluble proteins. *Biophys J*, January 1985, vol. 47 (1), 61-70
- **J. K. MOHANA RAO** ; **PATRICK ARGOS**. A conformational preference parameter to predict helices in integral membrane proteins. *Biochimica et Biophysica Acta (BBA) - Protein Structure and Molecular Enzymology*, 1986, vol. 869 (2), 197-214
- **SANZO MIYAZAWA** ; **ROBERT L. JERNIGAN**. Estimation of effective interresidue contact energies from protein crystal structures: quasi-chemical approximation. *Macromolecules*, vol. 18 (3), 534-552
- **GANG ZHAO** ; **ERWIN LONDON**. An amino acid "transmembrane tendency" scale that approaches the theoretical limit to accuracy for prediction of transmembrane helices: relationship to biological hydrophobicity. *Protein science : a publication of the Protein Society*, August 2006, vol. 15 (8), 1987-2001
- **CYRUS CHOTHIA**. The nature of the accessible and buried surfaces in proteins. *Journal of Molecular Biology*, 1976, vol. 105 (1), 1-12
- **GD ROSE** ; **AR GESELOWITZ** ; **GJ LESSER** ; **RH LEE** ; **MH ZEHFUS**. Hydrophobicity of amino acid residues in globular proteins. *Science*, August 1985, vol. 229 (4716), 834
- **P Y CHOU** ; **G D FASMAN**. Prediction of the secondary structure of proteins from their amino acid sequence. *Adv Enzymol Relat Areas Mol Biol*, 1978, vol. 47, 45-148
- **SHNEIOR LIFSON** ; **CHRISTIAN SANDER**. Antiparallel and parallel -strands differ in amino acid residue preferences. *Nature*, 1979, vol. 282 (5734), 109-111
- **M. COOPER** ; **ROBERT E. HAUSMAN**. The cell : a molecular approach. DC ASM Press, 2007, vol. 4
- **EA EMINI** ; **J V HUGHES** ; **D S PERLOW** ; **J BOGER**. Induction of hepatitis a virus-neutralizing antibody by a virus-specific synthetic peptide. *Journal of virology*, September 1985, vol. 55 (3), 836-839
- **G. DELEAGE** ; **B. ROUX**. An algorithm for protein secondary structure prediction based on class prediction. *Protein Engineering, Design and Selection*, August 1987, vol. 1 (4), 289-294
- **P. MANAVALAN** ; **P. K. PONNUSWAMY**. Hydrophobic character of amino acid residues in globular proteins. *Nature*, 1978, vol. 275 (5681), 673-674
- **MICHAEL LEVITT.** Conformational preferences of amino acids in globular proteins. *Biochemistry*, October 1978, vol. 17 (20), 4277-4285
- **SERAFIN FRAGA**. Theoretical prediction of protein antigenic determinants from amino acid sequences. *Canadian Journal of Chemistry*, 01 March 2019, vol. 60 (20), 2606-2610

- **GJALT W. WELLING** ; **WICHER J. WEIJER** ; **RUURD VAN DER ZEE** ; **SYTSKE WELLING-WESTER**. Prediction of sequential antigenic regions in proteins. *FEBS Letters*, 28 February 2019, vol. 188 (2), 215-218
- **HENRY B. BULL** ; **KEITH BREESE**. Surface tension of amino acid solutions: A hydrophobicity scale of the amino acid residues. *Archives of Biochemistry and Biophysics*, 1974, vol. 161 (2), 665-670
- **C. A. BROWNE** ; **H. P. J. BENNETT** ; **S. SOLOMON**. The isolation of peptides by high-performance liquid chromatography using predicted elution positions. *Analytical Biochemistry*, 1982, vol. 124 (1), 201-208
- **R. WOLFENDEN** ; **L. ANDERSSON** ; **P. M. CULLIS** ; **C. C. B. SOUTHGATE**. Affinities of amino acid side chains for solvent water. *Biochemistry*, February 1981, vol. 20 (4), 849-855
- **J L MEEK**. Prediction of peptide retention times in high-pressure liquid chromatography on the basis of amino acid composition. *Proceedings of the National Academy of Sciences of the United States of America*, March 1980, vol. 77 (3), 1632-1636
- **R. BHASKARAN** ; **P. K. PONNUSWAMY**. Positional flexibilities of amino acid residues in globular proteins. *International Journal of Peptide and Protein Research*, 28 February 2019, vol. 32 (4), 241-255
- A model of evolutionary change in proteins. **SCHWARTZ R.M.** ; **DAYHOFF, M.O.** ; **B.C. ORCUTT.** Atlas of Protein Sequence and Structure. Natl. Biomed. Res. Found., 1978, vol. 5
- Lehninger principles of biochemistry. **DAVID L. (DAVID LEE) NELSON** ; **MICHAEL M. COX.** Principles of biochemistry. W.H.Freeman, 2017
- **K JWILSON** ; **A HONEGGER** ; **R P STOTZEL** ; **G J HUGHES**. The behaviour of peptides on reverse-phase supports during high-pressure liquid chromatography. *The Biochemical journal*, October 1981, vol. 199 (1), 31-41
- **A. S. KOLASKAR** ; **PRASAD C. TONGAONKAR**. A semi-empirical method for prediction of antigenic determinants on protein antigens. *FEBS Letters*, 10 December 1990, vol. 276 (1-2), 172-174
- **PETER MCCALDON** ; **PATRICK ARGOS.** Oligopeptide biases in protein sequences and their use in predicting protein coding regions in nucleotide sequences. *Proteins: Structure, Function, and Bioinformatics*, 28 February 2019, vol. 4 (2), 99-122
- **J.M. ZIMMERMAN** ; **NAOMI ELIEZER** ; **R. SIMHA.** The characterization of amino acid sequences in proteins by statistical methods. *Journal of Theoretical Biology*, 1968, vol. 21 (2), 170-201
- **DANIEL D. JONES**. *Amino acid properties and side-chain orientation in proteins: A cross correlation approach*, April 1975, vol. 50
- **D. EISENBERG** ; **E. SCHWARZ** ; **M. KOMAROMY** ; **R.WALL.** Analysis of membrane and surface protein sequences with the hydrophobic moment plot. *Journal of Molecular Biology*, 1984, vol. 179 (1), 125-142
- **J. M. R. PARKER** ; **D. GUO** ; **R. S. HODGES**. New hydrophilicity scale derived from high-performance liquid chromatography peptide retention data: correlation of predicted surface residues with antigenicity and x-ray-derived accessible sites. *Biochemistry*, September 1986, vol. 25 (19), 5425-5432
- **SHAUN D. BLACK** ; **DIANE R.** Mould. Development of hydrophobicity parameters to analyze proteins which bear post- or cotranslational modifications. *Analytical Biochemistry*, 1991, vol. 193 (1), 72-82
- **J L. FAUCHERE** ; **V PLISKA**. *Hydrophobic parameters II of amino acid side-chains from the partitioning of N-acetyl-amino acid amides*, January 1983, vol. 18
- **ROBERT M. SWEET** ; **DAVID EISENBERG**. Correlation of sequence hydrophobicities measures similarity in three-dimensional protein structure. *Journal of Molecular Biology*, 1983, vol. 171 (4), 479-488
- **JACK KYTE** ; **RUSSELL F. DOOLITTLE**. A simple method for displaying the hydropathic character of a protein. *Journal of Molecular Biology*, 1982, vol. 157 (1), 105-132
- **CHARLES. TANFORD**. Contribution of hydrophobic interactions to the stability of the globular conformation of proteins. *Journal of the American Chemical Society*, November 1962, vol. 84 (22), 4240-4247
- **DONALD J. ABRAHAM** ; **ALBERT J. LEO.** Extension of the fragment method to calculate amino acid zwitterion and side chain partition coefficients. *Proteins: Structure, Function, and Bioinformatics*, 28 February 2019, vol. 2 (2), 130-152
- **RICHARD COWAN** ; **R GWHITTAKER**. *Hydrophobicity indices for amino acid residues as determined by HPLC*, January 1990, vol. 3
- **MARK A. ROSEMAN**. Hydrophilicity of polar amino acid side-chains is markedly reduced by flanking peptide bonds. *Journal of Molecular Biology*, 1988, vol. 200 (3), 513-522
- **A. A. ABODERIN**. An empirical hydrophobicity scale for alpha-amino-acids and some of its applications. *International Journal of Biochemistry*, 1971, vol. 2, 537-544